# EUROPÄISCHE PATENTSCHRIFT

(11) **EP 3 116 868 B1**
(45) Veröffentlichungstag und Bekanntmachung des Hinweises auf die Patenterteilung: **06.06.2018**
(21) Anmeldenummer: 15707980.7
(22) Anmeldetag: 06.03.2015
(51) Int. Cl.: C07D 403/04, A01N 43/713, C07D 213/77, C07D 401/04

(54) **HETEROCYLISCHE VERBINDUNGEN ALS SCHÄDLINGSBEKÄMPFUNGSMITTEL**
HETEROCYCLIC COMPOUNDS AS PEST CONTROLLERS
COMPOSÉS HÉTÉROCYCLIQUES EN TANT QUE MOYEN DE LUTTE CONTRE LES PARASITES

(30) Priorität: 10.03.2014 EP 14158461
(43) Veröffentlichungstag der Anmeldung: 18.01.2017
(73) Patentinhaber: Bayer CropScience Aktiengesellschaft, 40789 Monheim am Rhein (DE)
(72) Erfinder: BRETSCHNEIDER, Thomas, deceased (DE); CEREZO-GALVEZ, Silvia, 40764 Langenfeld (DE); GRONDAL, Christoph, 50937 Köln (DE); FISCHER, Reiner, 40789 Monheim (DE); FÜSSLEIN, Martin, 40225 Düsseldorf (DE); REINISCH, Peter, 40764 Langenfeld (DE); GÜCLÜ, Mehmet, 51063 Köln (DE); ILG, Kerstin, 50670 Köln (DE); LÖSEL, Peter, 51371 Leverkusen (DE); MALSAM, Olga, 51503 Rösrath (DE)
(74) Vertreter: BIP Patents
(86) Internationale Anmeldenummer: PCT/EP2015/054696
(87) Internationale Veröffentlichungsnummer: WO 2015/135843

(56) Entgegenhaltungen:
- WO-A1-2010/100189
- JP-A- H11 171 702
- LUDWIG BAUER ET AL: "Pyrazolo- N -hydroxyuracils from the modified lossen rearrangement of vicinal pyrazoledicarbohydroxamates", JOURNAL OF HETEROCYCLIC CHEMISTRY, Bd. 4, Nr. 3, 1. September 1967 (1967-09-01), Seiten 325-334, XP055112417, ISSN: 0022-152X, DOI: 10.1002/jhet.5570040303
- WANG ET AL: "Rapid hit to lead evaluation of pyrazolo[3,4-d]pyrimidin-4-one as selective and orally bioavailable mGluR1 antagonists", BIOORGANIC & MEDICINAL CHEMISTRY LETTERS, PERGAMON, AMSTERDAM, NL, Bd. 17, Nr. 15, 1. August 2007 (2007-08-01), Seiten 4303-4307, XP022144693, ISSN: 0960-894X, DOI: 10.1016/J.BMCL.2007.05.028

## Beschreibung

Die vorliegende Anmeldung betrifft neue heterocyclische Verbindungen, Verfahren und Zwischenprodukte zu Ihrer Herstellung und ihre Verwendung zur Bekämpfung von tierischen Schädlingen.

In WO 2012/102387 A1 sind heterocyclische Verbindungen beschrieben, die insbesondere als Insektizide und Akarizide verwendet werden können.

Heterocyclische Verbindungen für pharmazeutische Anwendungen werden in WO 2004/009597 A2, WO 2008/107418 A1, WO 2009/068617 A1 und US 2004/242596 A1 offenbart.

In JP H11 171702 A ist die Verwendung phenylsubstituierter Pyrazole in der Landwirtschaft beschrieben.

Die Herstellung von Pyrazolouracilen und hierbei verwendete Zwischenprodukte sind Gegenstand der Publikation in Journal of Heterocyclic Chemistry, Bd. 4, Nr. 3, 1. September 1967, Seiten 325-334.

In Bioorganic & Medicinal Chemistry Letters (2007), 17(15), 4303-4307 wird über Synthese und pharmakologische Eigenschaften bestimmter Pyrazolo[3,4-d]pyrimidin-4-one berichtet.

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Umwelteigenschaften, der Aufwandmengen, der Kombinierbarkeit mit anderen Wirkstoffen oder Formulierhilfsmitteln eine Rolle sowie die Frage des Aufwands, der für die Synthese eines Wirkstoffs betrieben werden muss, ferner können Resistenzen auftreten, um nur einige Paramenter zu nennen. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten ergänzt wird.

Gelöst wird die Aufgabe, sowie weitere nicht explizit genannte Aufgaben, die aus den hierin diskutierten Zusammenhängen ableitbar oder erschließbar sind, durch die Bereitstellung von Verbindungen der Formel (I) 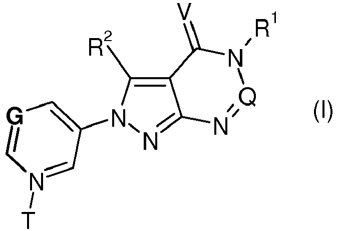 in welcher
- G: für N oder C-A¹ steht,
- A¹: für einen Rest aus der Reihe Wasserstoff, Halogen, Cyano, Nitro, Alkyl, Halogenalkyl, Alkoxy, Halogenalkoxy oder jeweils gegebenenfalls substituiertes Cycloalkyl und Cycloalkenyl steht,
- T: für ein Elektronenpaar oder für Sauerstoff steht,
- R¹: für den Rest der Formel 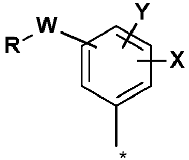 steht, worin die Bindung zum Stickstoffatom in der C(=V)-N-Q-Gruppe in der Formel (I) mit dem Stern (*) markiert ist,
- R: für NR⁷R⁸ oder für einen jeweils gegebenenfalls substituierten Rest aus der Reihe Alkyl, Alkenyl, Alkinyl, Alkoxyalkyl, Alkyl-S(O)ₘ-alkyl, R⁷-CO-alkyl, NR⁷R⁸-CO-alkyl, Cycloalkyl, Cycloalkenyl, Cycloalkylalkyl, Cycloalkenylalkyl, Heterocyclyl, Heterocyclylalkyl, Phenyl, Phenylalkyl, Hetaryl und Hetarylalkyl steht,
- W: für einen Rest aus der Reihe O, S, SO und SO₂ steht,
- X: für einen Rest aus der Reihe Wasserstoff, Halogen, Cyano, Alkyl, Halogenalkyl, Alkoxy, Halogenalkoxy und Cycloalkyl steht,
- Y: für einen Rest aus der Reihe Wasserstoff, Halogen, Cyano, Alkyl, Halogenalkyl, Alkoxy, Halogenalkoxy, Cycloalkyl und NR⁵R⁶ steht,
- R²: für Wasserstoff oder Alkyl steht,

- Q: für Stickstoff oder C-R³ steht, worin
- R³: für einen Rest aus der Reihe Wasserstoff, Halogen, Cyano, Nitro, Hydroxy, Alkyl, Halogenalkyl, Cycloalkyl, Alkoxy, Halogenalkoxy, Cycloalkylalkyl, Alkoxyalkyl, Halogenalkoxyalkyl, SH, Alkylsulfanyl, Alkylsulfinyl, Alkylsulfonyl, Halogenalkylsulfanyl, Halogenalkylsulfinyl, Halogenalkylsulfonyl, NH₂, Alkylamino und Dialkylamino steht,
- V: für einen Rest aus der Reihe Sauerstoff, Schwefel und NR⁴ steht und
- R⁴: für einen Rest aus der Reihe Wasserstoff, Cyano, Alkyl, Halogenalkyl, Cycloalkyl, Nitro, Carbonylalkyl, Carbonylhalogenalkyl und Carbonylalkoxy steht,
- R⁵: für einen Rest aus der Reihe Wasserstoff, Alkyl und Halogenalkyl steht,
- R⁶: für einen Rest aus der Reihe Wasserstoff, Alkyl und Halogenalkyl steht,
oder
- R⁵ und R⁶: gemeinsam mit dem Stickstoff, an dem sie gebunden sind, für einen gegebenenfalls substituierten und gegebenenfalls weitere Heteroatome enthaltenden gesättigten oder ungesättigten 3- bis 6-gliedrigen Ring stehen.
- R⁷: für Wasserstoff, Hydroxy oder einen jeweils gegebenenfalls substituierten Rest aus der Reihe Alkyl, Alkoxy, Alkoxyalkyl, Alkyl-S(O)ₘ-alkyl, Alkylcarbonyl, Alkoxycarbonyl, Cycloalkyl, Cycloalkenyl, Cycloalkylalkyl, Cycloalkenylalkyl, Heterocyclyl, Heterocyclylalkyl, Phenyl, Phenylalkyl, Hetaryl und Hetarylalkyl steht,
- R⁸: für Wasserstoff, ein Metallion, ein gegebenenfalls substituiertes Ammoniumion oder einen jeweils gegebenenfalls substituierten Rest aus der Reihe Alkyl, Alkoxy, Alkoxyalkyl, Alkyl-S(O)ₘ-alkyl steht und
- m: für eine Zahl aus der Reihe 0, 1 und 2 steht.

Bevorzugte Substituenten bzw. Bereiche der in den Verbindungen der Formel (I) aufgeführten Reste werden im Folgenden erläutert. Ihre Kombination bildet den Vorzugsbereich (1).
- G: steht für N oder C-A¹.
- A¹: steht für einen Rest aus der Reihe Wasserstoff, Halogen, Cyano, C₁-C₆-Alkyl, C₁-C₆-Halogenalkyl und C₃-C₆-Cycloalkyl.
- T: steht für ein Elektronenpaar oder für Sauerstoff.
- R¹: steht für den Rest der Formel 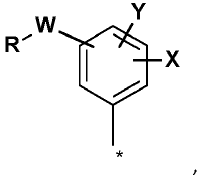 worin die Bindung zum Stickstoffatom in der C(=V)-N-Q-Gruppe in der Formel (I) mit dem Stern (*) markiert ist.
- R: steht für NR⁷R⁸ oder für jeweils gegebenenfalls durch Halogen oder Cyano substituiertes C₁-C₆-Alkyl, C₃-C₆-Alkenyl, C₃-C₆-Alkinyl, C₁-C₆-Alkoxy-C₁-C₄-alkyl, C₁-C₆-Alkyl-S(O)ₘ-C₁-C₄-alkyl, für R⁷-CO-C₁-C₄-alkyl, für NR⁷R⁸-CO-C₁-C₄-alkyl, für jeweils gegebenenfalls einfach oder zweifach durch Sauerstoff (führt zu C=O), Halogen, Cyano, C₁-C₄-Alkyl, C₁-C₄-Alkoxy und C₁-C₄-Halogenalkyl substituiertes C₃-C₈-Cycloalkyl, für jeweils gegebenenfalls einfach oder zweifach durch Sauerstoff (führt zu C=O), C₁-C₄-Alkyl, C₁-C₄-Alkoxy und C₁-C₄-Halogenalkyl substituiertes C₃-C₈-Cycloalkenyl, für gegebenenfalls einfach oder zweifach durch Sauerstoff (führt zu C=O), Halogen, Cyano, C₁-C₄-Alkyl, C₁-C₄-Alkoxy und C₁-C₄-Halogenalkyl substituiertes C₃-C₆-Cycloalkyl-C₁-C₄-alkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes C₃-C₆-Cycloalkenyl-C₁-C₄-alkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes Heterocyclyl, für gegebenenfalls einfach oder zweifach durch Sauerstoff (führt zu C=O), C₁-C₄-Alkyl, C₁-C₄-Alkoxy und C₁-C₄-Halogenalkyl substituiertes Heterocyclyl-C₁-C₄-alkyl oder für jeweils gegebenenfalls einfach bis dreifach durch Halogen, Cyano, C₁-C₄-Alkyl, C₁-C₄-Halogenalkyl, C₁-C₄-Alkoxy oder C₁-C₄-Halogenalkoxy substituiertes Phenyl, Phenyl-C₁-C₄-alkyl, Hetaryl und Hetaryl-C₁-C₄-alkyl.
- m: steht für eine Zahl aus der Reihe 0, 1 und 2.
- W: steht für einen Rest aus der Reihe O, S, SO und SO₂.
- X: steht für einen Rest aus der Reihe Wasserstoff, Halogen, Cyano, C₁-C₆-Alkyl, C₁-C₆-Halogenalkyl, C₁-C₆-Alkoxy, C₁-C₆-Halogenalkoxy und C₃-C₆-Cycloalkyl.
- Y: steht für einen Rest aus der Reihe Wasserstoff, Halogen, Cyano, C₁-C₆-Alkyl, C₁-C₆-Halogenalkyl, C₁-C₆-Alkoxy, C₁-C₆-Halogenalkoxy, C₃-C₆-Cycloalkyl und NR⁵R⁶.
- R²: steht für Wasserstoff oder C₁-C₆-Alkyl.

- Q: steht für Stickstoff oder C-R³.
- R³: steht für einen Rest aus der Reihe Wasserstoff, Hydroxy, C₁-C₆-Alkyl, C₁-C₆-Halogenalkyl, C₃-C₆-Cycloalkyl, C₁-C₆-Alkoxy, C₁-C₆-Halogenalkoxy, C₃-C₆-Cycloalkyl-C₁-C₄-alkyl, C₁-C₆-Alkoxy-C₁-C₄-alkyl, C₁-C₆-Halogenalkoxy-C₁-C₄-alkyl, SH, C₁-C₆-Alkylsulfanyl, C₁-C₆-Alkylsulfinyl, C₁-C₆-Alkylsulfonyl, NH₂, C₁-C₆-Alkylamino und Di-(C₁-C₆-alkyl)-amino.
- V: steht für einen Rest aus der Reihe Sauerstoff, Schwefel und NR⁴.
- R⁴: steht für einen Rest aus der Reihe Wasserstoff, Cyano, C₁-C₆-Alkyl, C₂-C₆-Halogenalkyl und C₃-C₆-Cycloalkyl.
- R⁵: steht für einen Rest aus der Reihe Wasserstoff, C₁-C₆-Alkyl und C₂-C₆-Halogenalkyl.
- R⁶: steht für einen Rest aus der Reihe Wasserstoff, C₁-C₆-Alkyl und C₂-C₆-Halogenalkyl.
- R⁵ und R⁶: können auch gemeinsam mit dem Stickstoffatom, an das sie gebunden sind, für einen gegebenenfalls durch Halogen, Cyano, C₁-C₄-Alkyl, C₁-C₄-Halogenalkyl, C₁-C₄-Alkoxy, C₁-C₄-Halogenalkoxy substituierten gesättigten bis dreifach ungesättigten 3- bis 6-gliedrigen Ring stehen, insbesondere für Aziridinyl, Azirenyl, Diaziridinyl, Diazirenyl, Azetidinyl, Dihydroazetyl, Diazetidinyl, Dihydrodiazetyl, Oxazetidinyl, Oxazetyl, Thiazetidinyl, Thiazetyl, Pyrrolidinyl, Dihydropyrrolyl, Pyrazolidinyl, Dihydropyrazolyl, Imidazolidinyl, Dihydroimidazolyl, Oxazolidinyl, Dihydrooxazolyl, Thiazolidinyl, Dihydrothyazolyl, Piperidinyl, Piperazinyl, Hexahydropyridazinyl, Hexahydropyrimidinyl, Morpholin, Dioxazinanyl, Thiomorpholin, Dithiazinan, Pyrrolyl, Pyrazolyl, Imidazolyl, Triazolyl und Tetrazolyl.
- R⁷: steht für einen Rest aus der Reihe Wasserstoff, Hydroxy, jeweils gegebenenfalls einfach oder mehrfach durch Halogen oder einfach oder zweifach durch Cyano substituiertes C₁-C₆-Alkyl, C₁-C₆-Alkoxy, C₁-C₆-Alkoxy-C₁-C₄-alkyl, C₁-C₆-Alkyl-S(O)ₘ-C₁-C₄-alkyl, C₁-C₆-Alkylcarbonyl, C₁-C₆-Alkoxy-carbonyl, C₃-C₆-Cycloalkyl, C₃-C₆-Cycloalkenyl, C₃-C₆-Cycloalkyl-C₁-C₃-alkyl, C₃-C₆-Cycloalkenyl-C₁-C₃-alkyl, Heterocyclyl, Heterocyclyl-C₁-C₃-alkyl und jeweils gegebenenfalls einfach bis vierfach durch C₁-C₄-Alkyl, C₁-C₄-Halogenalkyl, C₁-C₄-Alkoxy, C₁-C₄-Halogenalkoxy, C₃-C₆-Cycloalkyl, Halogen oder Cyano substituiertes Phenyl, Phenyl-C₁-C₃-alkyl, Hetaryl und Hetaryl-C₁-C₃-alkyl.
- R⁸: steht für Wasserstoff, ein Metallion oder für ein gegebenenfalls einfach bis vierfach durch C₁-C₄-Alkyl substituiertes Ammoniumion oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₄-alkyl und C₁-C₄-Alkyl-S(O)ₘ-alkyl.
Besonders bevorzugte Substituenten bzw. Bereiche der in den Verbindungen der Formel (I) aufgeführten Reste werden im Folgenden erläutert. Ihre Kombination bildet den Vorzugsbereich (2).
- G: steht für N oder C-A¹.
- A¹: steht für einen Rest aus der Reihe Wasserstoff, Fluor, Chlor, Brom, C₁-C₄-Alkyl und C₁-C₄-Halogenalkyl.
- T: steht für ein Elektronenpaar oder für Sauerstoff.
- R¹: steht für den Rest der Formel 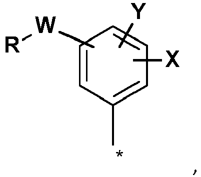 worin die Bindung zum Stickstoffatom in der C(=V)-N-Q-Gruppe in der Formel (I) mit dem Stern (*) markiert ist.
- R: steht für NR⁷R⁸ oder für jeweils gegebenenfalls einfach bis siebenfach durch Halogen, oder einfach oder zweifach durch Cyano substituiertes C₁-C₆-Alkyl, C₃-C₆-Alkenyl, C₃-C₆-Alkinyl, C₁-C₄-Alkoxy-C₁-C₃-alkyl, C₁-C₄-Alkyl-S(O)ₘ-C₁-C₃-alkyl, für R⁷-CO-C₁-C₂-alkyl, für NR⁷R⁸-CO-C₁-C₂-alkyl, für gegebenenfalls einfach oder zweifach durch Sauerstoff (führt zu C=O), Halogen, Cyano, C₁-C₄-Alkyl, C₁-C₄-Alkoxy und C₁-C₄-Halogenalkyl substituiertes C₃-C₈-Cycloalkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes C₃-C₈-Cycloalkenyl, für gegebenenfalls einfach oder zweifach durch Sauerstoff (führt zu C=O), Halogen, Cyano, C₁-C₄-Alkyl, C₁-C₄-Alkoxy und C₁-C₄-Halogenalkyl substituiertes C₃-C₆-Cycloalkyl-C₁-C₄-alkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes, gesättigtes oder ungesättigtes C₃-C₆-Cycloalkenyl-C₁-C₄-alkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes Heterocyclyl, für gegebenenfalls einfach oder zweifach durch Sauerstoff (führt zu C=O), C₁-C₄-Alkyl, C₁-C₄-Alkoxy und C₁-C₄-Halogenalkyl substituiertes Heterocyclyl-C₁-C₄-alkyl oder für jeweils gegebenenfalls einfach bis dreifach durch Halogen, Cyano, C₁-C₄-Alkyl, C₁-C₄-Halogenalkyl, C₁-C₄-Alkoxy oder C₁-C₄-Halogenalkoxy substituiertes Phenyl, Phenyl-C₁-C₃-alkyl, Hetaryl, und Hetaryl-C₁-C₃-alkyl.
- W: steht für einen Rest aus der Reihe S, SO und SO₂.

- X: steht für einen Rest aus der Reihe Wasserstoff, Fluor, Chlor, Brom, Jod, Cyano, C₁-C₄-Alkyl, C₁-C₄-Halogenalkyl, C₁-C₄-Alkoxy und C₁-C₄-Halogenalkoxy.
- Y: steht für einen Rest aus der Reihe Wasserstoff, Fluor, Chlor, Brom, Jod, Cyano, C₁-C₄-Alkyl, C₁-C₄-Halogenalkyl, C₁-C₄-Alkoxy und C₁-C₄-Halogenalkoxy.
- R²: steht für Wasserstoff oder C₁-C₄-Alkyl.
- Q: steht für Stickstoff oder C-R³.
- R³: steht für einen Rest aus der Reihe Wasserstoff, Hydroxy, C₁-C₄-Alkyl, C₁-C₄-Halogenalkyl, C₃-C₆-Cycloalkyl, C₁-C₄-Alkoxy, C₁-C₄-Halogenalkoxy, C₃-C₆-Cycloalkyl-C₁-C₃-alkyl, C₁-C₄-Alkoxy-C₁-C₃-alkyl, C₁-C₄-Halogenalkoxy-C₁-C₃-alkyl, SH, C₁-C₄-Alkylsulfanyl, C₁-C₄-Alkylsulfinyl, C₁-C₄-Alkylsulfonyl, NH₂, C₁-C₄-Alkylamino und Di-(C₁-C₄-alkyl)-amino.
- V: steht für Sauerstoff.
- R⁷: steht für Wasserstoff, Hydroxy oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₄-alkyl, C₁-C₄-Alkyl-S(O)ₘ-C₁-C₃-alkyl, C₁-C₄-Alkyl-carbonyl, C₁-C₄-Alkoxy-carbonyl, C₃-C₆-Cycloalkyl, C₃-C₆-Cycloalkyl-C₁-C₃-alkyl, Heterocyclyl, Heterocyclyl-C₁-C₃-alkyl und jeweils gegebenenfalls einfach bis dreifach durch C₁-C₄-Alkyl, C₁-C₃-Halogenalkyl, C₁-C₃-Alkoxy, C₁-C₃-Halogenalkoxy, Cyclopropyl, Fluor Chlor, Brom oder Cyano substituiertes Phenyl, Benzyl, Pyridyl, Pyrimidyl, Thiazolyl, Oxazolyl, Pyrazolyl, Thienyl, Furanyl, Pyridinyl-methyl und Thiazolylmethyl.
- R⁸: steht für Wasserstoff, für ein Metallion oder für ein gegebenenfalls einfach bis vierfach durch C₁-C₄-Alkyl substituiertes Ammoniumion oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₂-alkyl und C₁-C₄-Alkyl-S(O)ₘ-C₁-C₂-alkyl.
- m: steht für eine Zahl aus der Reihe 0, 1 und 2.

Ganz besonders bevorzugte Substituenten bzw. Bereiche der in den Verbindungen der Formel (I) aufgeführten Reste werden im Folgenden erläutert. Ihre Kombination bildet den Vorzugsbereich (3).
- G: steht für N oder C-A¹.
- A¹: steht für einen Rest aus der Reihe Wasserstoff, Fluor, Chlor, Brom, Methyl, Ethyl, Isopropyl, *tert-*Butyl, Trifluormethyl, Difluormethyl, 2,2,2-Trifluorethyl und 2,2-Difluorethyl.
- T: steht für ein Elektronenpaar oder für Sauerstoff.

- R¹: steht für den Rest der Formel 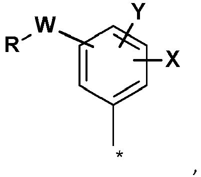 worin die Bindung zum Stickstoffatom in der C(=V)-N-Q-Gruppe in der Formel (I) mit dem Stern (*) markiert ist.
- R: steht für NR⁷R⁸ oder für einen jeweils gegebenenfalls einfach, zweifach, dreifach, vierfach oder fünffach durch Fluor, Chlor oder zweifach durch Cyano substituierten Rest aus der Reihe C₁-C₄-Alkyl, C₃-C₄-Alkenyl, C₃-C₄-Alkinyl, C₁-C₂-Alkoxy-C₁-C₂-alkyl und C₁-C₂-Alkyl-S(O)ₘ-C₁-C₂-alkyl, für R⁷-CO-C₁-C₂-alkyl, für NR⁷R⁸-CO-C₁-C₂-alkyl, für gegebenenfalls einfach oder zweifach durch Halogen, Cyano, C₁-C₂-Alkyl, C₁-C₂-Alkoxy oder C₁-C₂-Halogenalkyl oder durch ein Sauerstoffatom (führt zu C=O) substituiertes C₃-C₆-Cycloalkyl, für gegebenenfalls einfach oder zweifach durch C₁-C₂-Alkyl, C₁-C₂-Alkoxy oder C₁-C₂-Halogenalkyl oder durch ein Sauerstoffatom (führt zu C=O) substituiertes C₃-C₆-Cycloalkenyl, für gegebenenfalls einfach bis zweifach durch Halogen, Cyano, C₁-C₂-Alkyl, C₁-C₂-Alkoxy oder C₁-C₂-Halogenalkyl substituiertes C₃-C₆-Cycloalkyl-C₁-C₂-alkyl, für gegebenenfalls einfach oder zweifach durch C₁-C₂-Alkyl, C₁-C₂-Alkoxy oder C₁-C₂-Halogenalkyl substituiertes C₃-C₆-Cycloalkenyl-C₁-C₂-alkyl, für gegebenenfalls einfach oder zweifach durch C₁-C₂-Alkyl, C₁-C₂-Alkoxy oder C₁-C₂-Halogenalkyl substituiertes Heterocyclyl, für gegebenenfalls einfach oder zweifach durch C₁-C₂-Alkyl, C₁-C₂-Alkoxy oder C₁-C₂-Halogenalkyl substituiertes Heterocyclyl-C₁-C₂-alkyl und für jeweils gegebenenfalls einfach oder zweifach durch Fluor, Chlor, Brom, Cyano, C₁-C₄-Alkyl, C₁-C₄-Halogenalkyl, C₁-C₄-Alkoxy oder C₁-C₄-Halogenalkoxy substituiertes Phenyl, Benzyl, Pyridyl, Pyrimidyl, Thiazolyl, Oxazolyl, Pyrazolyl, Thienyl, Furanyl, Pyridinylmethyl und Thiazolylmethyl.
- W: steht für einen Rest aus der Reihe S, SO und SO₂.
- X: steht für einen Rest aus der Reihe Wasserstoff, Fluor, Chlor, Brom, Cyano, Methyl, Ethyl, Trifluomethyl, Methoxy, Ethoxy, Difluormethoxy und Trifluormethoxy.
- Y: steht für einen Rest aus der Reihe Wasserstoff, Fluor, Chlor, Brom, Cyano, Methyl, Ethyl, Trifluomethyl, Methoxy, Ethoxy, Difluormethoxy und Trifluormethoxy.
- R²: steht für Wasserstoff oder Methyl.

- Q: steht für Stickstoff oder C-R³.
- R³: steht für einen Rest aus der Reihe Wasserstoff, Methyl, Trifluormethyl und Cyclopropyl.
- V: steht für Sauerstoff.
- R⁷: steht für einen Rest aus der Reihe Wasserstoff, Hydroxy, gegebenenfalls einfach, zweifach, dreifach, vierfach oder fünffach durch Fluor, Chlor oder einfach oder zweifach durch Cyano substituiertes C₁-C₄-Alkyl, C₁-C₄-Alkoxy, C₁-C₄-Alkoxy-C₁-C₄-alkyl, C₁-C₄-Alkyl-S(O)ₘ-C₁-C₂-alkyl, C₁-C₄-Alkylcarbonyl, C₁-C₄-Alkoxycarbonyl, C₃-C₆-Cycloalkyl, C₃-C₆-Cycloalkyl-C₁-C₃-alkyl, Heterocyclyl und Heterocyclyl-C₁-C₃-alkyl und jeweils gegebenenfalls einfach bis dreifach durch C₁-C₄-Alkyl, C₁-C₃-Halogenalkyl, C₁-C₃-Alkoxy, C₁-C₃-Halogenalkoxy, Cyclopropyl, Fluor, Chlor, Brom oder Cyano substituiertes Phenyl, Benzyl, Pyridyl, Pyrimidyl, Thiazolyl, Oxazolyl, Pyrazolyl, Thienyl, Furanyl, Pyridinylmethyl und Thiazolylmethyl.
- R⁸: steht für Wasserstoff, ein Alkali- oder Erdalkaliion, für ein gegebenenfalls einfach bis vierfach durch C₁-C₄-Alkyl substituiertes Ammoniumion oder für einen jeweils gegebenenfalls einfach oder mehrfach durch Fluor, Chlor oder einfach oder zweifach durch Cyano substituierten Rest aus der Reihe C₁-C₄-Alkyl, C₁-C₄-Alkoxy, C₁-C₄-Alkoxy-C₁-C₂-alkyl und C₁-C₄-Alkyl-S(O)ₘ-C₁-C₂-alkyl.
- m: steht für eine Zahl aus der Reihe 0, 1 und 2.

Insbesonders bevorzugte Substituenten bzw. Bereiche der in den Verbindungen der Formel (I) aufgeführten Reste werden im Folgenden erläutert. Ihre Kombination bildet den Vorzugsbereich (4).
- G: steht für N oder C-A¹.
- A¹: steht für einen Rest aus der Reihe Wasserstoff und Fluor.
- T: steht für ein Elektronenpaar oder für Sauerstoff.
- R¹: steht für einen der folgenden Reste 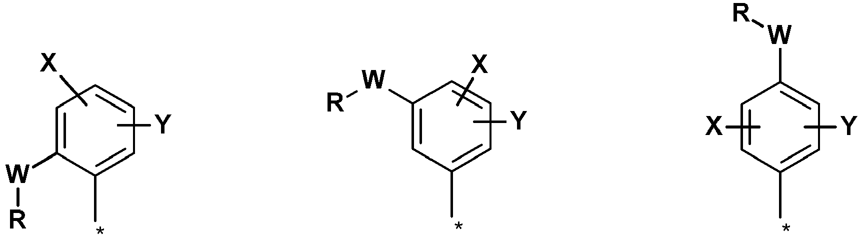 worin die Bindung zum Stickstoffatom in der C(=V)-N-Q-Gruppe in der Formel (I) mit dem Stern (*) markiert ist.
- R: steht für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Fluor, Chlor, Cyano, Methyl, Ethyl, Methoxy, Ethoxy oder Trifluormethyl substituiertes C₃-C₆-Cycloalkyl, für gegebenenfalls einfach durch Fluor, Chlor, Cyano, Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethlyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl.
- W: steht für einen Rest aus der Reihe S, SO und SO₂.
- X: steht für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy.
- Y: steht für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy.
- R²: steht für Wasserstoff oder Methyl.
- Q: steht für Stickstoff oder C-R³.
- R³: steht für einen Rest aus der Reihe Wasserstoff, Methyl, Trifluormethyl und Cyclopropyl.
- V: steht für Sauerstoff.

Hervorgehoben bevorzugte Substituenten bzw. Bereiche der in den Verbindungen der Formel (I) aufgeführten Reste werden im Folgenden erläutert. Ihre Kombination bildet den Vorzugsbereich (5).
- G: steht für N oder C-A¹.
- A¹: steht für einen Rest aus der Reihe Wasserstoff und Fluor.
- T: steht für ein Elektronenpaar.
- R¹: steht für den Rest der Formel 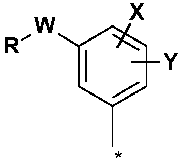 worin die Bindung zum Stickstoffatom in der C(=V)-N-Q-Gruppe in der Formel (I) mit dem Stern (*) markiert ist.
- R: steht für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Fluor, Chlor, Cyano, Methyl, Ethyl, Methoxy, Ethoxy oder Trifluormethyl substituiertes C₃-C₆-Cycloalkyl, für gegebenenfalls einfach durch Fluor, Chlor, Cyano, Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethlyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl.
- W: steht für einen Rest aus der Reihe S, SO und SO₂.
- X: steht für einen Rest aus der Reihe Wasserstoff, Fluor, Chlor, Brom, Cyano, Methyl, Ethyl und Trifluormethyl.
- Y: steht für einen Rest aus der Reihe Wasserstoff, Fluor, Chlor, Brom, Cyano, Methyl, Ethyl und Trifluormethyl.
- R²: steht für Wasserstoff oder Methyl.
- Q: steht für Stickstoff oder C-R³.
- R³: steht für einen Rest aus der Reihe Wasserstoff und Methyl.
- V: steht für Sauerstoff.

Weiter hervorgehoben bevorzugte Substituenten bzw. Bereiche der in den Verbindungen der Formel (I) aufgeführten Reste werden im Folgenden erläutert. Ihre Kombination bildet den Vorzugsbereich (6).
- G: steht für N oder C-A¹.
- A¹: steht für einen Rest aus der Reihe Wasserstoff oder Fluor,
- T: steht für ein Elektronenpaar
- R¹: steht für den Rest der Formel 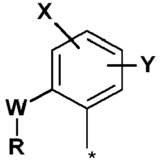 worin die Bindung zum Stickstoffatom in der C(=V)-N-Q-Gruppe in der Formel (I) mit dem Stern (*) markiert ist.
- R: steht für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Fluor, Chlor, Cyano, Methyl, Ethyl, Methoxy, Ethoxy oder Trifluormethyl substituiertes C₃-C₆-Cycloalkyl, für gegebenenfalls einfach durch Fluor, Chlor, Cyano, Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethlyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl.
- W: steht für einen Rest aus der Reihe S, SO und SO₂.
- X: steht für einen Rest aus der Reihe Wasserstoff, Fluor, Chlor, Brom, Cyano, Methyl, Ethyl und Trifluormethyl.
- Y: steht für einen Rest aus der Reihe Wasserstoff, Fluor, Chlor, Brom, Cyano, Methyl, Ethyl und Trifluormethyl.
- R²: steht für Wasserstoff oder Methyl.
- Q: steht für Stickstoff oder C-R³.
- R³: steht für einen Rest aus der Reihe Wasserstoff und Methyl.
- V: steht für Sauerstoff.

Weiter hervorgehoben bevorzugte Substituenten bzw. Bereiche der in den Verbindungen der Formel (I) aufgeführten Reste werden im Folgenden erläutert. Ihre Kombination bildet den Vorzugsbereich (7).
- G: steht für N oder C-A¹.
- A¹: steht für einen Rest aus der Reihe Wasserstoff oder Fluor.
- T: steht für ein Elektronenpaar.
- R¹: steht für den Rest der Formel 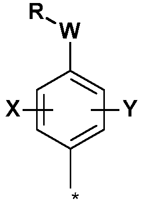 worin die Bindung zum Stickstoffatom in der C(=V)-N-Q-Gruppe in der Formel (I) mit dem Stern (*) markiert ist.
- R: steht für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Fluor, Chlor, Cyano, Methyl, Ethyl, Methoxy, Ethoxy oder Trifluormethyl substituiertes C₃-C₆-Cycloalkyl, für gegebenenfalls einfach durch Fluor, Chlor, Cyano, Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethlyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl.
- W: steht für einen Rest aus der Reihe S, SO und SO₂.
- X: steht für einen Rest aus der Reihe Wasserstoff, Fluor, Chlor, Brom, Cyano, Methyl, Ethyl und Trifluormethyl
- Y: steht für einen Rest aus der Reihe Wasserstoff, Fluor, Chlor, Brom, Cyano, Methyl, Ethyl und Trifluormethyl
- R²: steht für Wasserstoff oder Methyl.
- Q: steht für Stickstoff oder C-R³.
- R³: steht für einen Rest aus der Reihe Wasserstoff und Methyl.
- V: steht für Sauerstoff.

Eine weitere besondere Ausführungsform (Vorzugsbereich (8)) der Erfindung betrifft Verbindungen der Formel (I), in welchen
- G: für C-A¹ steht,
- A¹: für Wasserstoff steht,
- T: für ein Elektronenpaar oder Sauerstoff steht,
- R¹: für den Rest der Formel 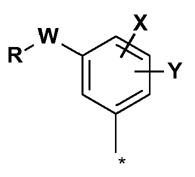 steht, worin die Bindung zum Stickstoffatom in der C(=V)-N-Q-Gruppe in der Formel (I) mit dem Stern (*) markiert ist,
- R: für einen Rest aus der Reihe Methyl, Trifluorethyl und Cyclopropylmethyl steht,
- W: für einen Rest aus der Reihe S und SO steht,
- X: für einen Rest aus der Reihe Wasserstoff, Fluor, Chlor und Methyl steht,
- Y: für einen Rest aus der Reihe Wasserstoff, Chlor und Methyl steht,
- R²: für Wasserstoff steht,
- Q: für Stickstoff oder C-R³ steht,
- R³: für Wasserstoff oder Methyl steht und
- V: für Sauerstoff steht.

Im Vorzugsbereich (1) ist, sofern nichts anderes angegeben ist,

Halogen ausgewählt aus der Reihe Fluor, Chlor, Brom und Iod, bevorzugt wiederum aus der Reihe Fluor, Chlor und Brom,

Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,

Heterocyclyl für einen gesättigten 3-, 4-, 5- oder 6-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für Aziridinyl, Azetidinyl, Azolidinyl, Azinanyl, Oxiranyl, Oxetanyl, Oxolanyl, Oxanyl, Dioxanyl, Thiiranyl, Thietanyl, Thiolanyl, Thianyl, Tetrahydrofuryl.

Im Vorzugsbereich (2) steht, sofern nichts anderes angegeben ist,

Halogen für Fluor, Chlor, Brom und Iod, bevorzugt für Fluor, Chlor und Brom,

Hetaryl (auch als Teil einer größeren Einheit, wie Hetarylalkyl) für Pyridyl, Pyrimidyl, Thiazolyl, Oxazolyl, Pyrazolyl, Thienyl, Furanyl, Benzyl, Pyridinylmethyl und Thiazolylmethyl sowie Heterocyclyl (auch als Teil einer größeren Einheit, wie Heterocyclylalkyl) für einen gesättigten oder ungesättigten 3-, 4- oder 5-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für 1- oder 2-Aziridinyl, 2-Oxiranyl, 2-Thiiranyl, 1- oder 2-Azetidinyl, 2- oder 3-Oxetanyl, 2- oder 3-Thietanyl, 1,3-Dioxetan-2-yl, 1-, 2- oder 3-Pyrrolidinyl.

Im Vorzugsbereich (3) steht, sofern nichts anderes angegeben ist,

Halogen für Fluor, Chlor, Brom und Iod, bevorzugt für Fluor, Chlor und Brom und

Heterocyclyl (auch als Teil einer größeren Einheit, wie Heterocyclylalkyl) für einen gesättigten oder ungesättigten 3- oder 4-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für 1- oder 2-Aziridinyl, 2-Oxiranyl, 2-Thiiranyl, 1- oder 2-Azetidinyl, 2- oder 3-Oxetanyl, 2- oder 3-Thietanyl oder 1,3-Dioxetan-2-yl.

Durch Halogen substituierte Reste, z.B. Halogenalkyl (= Haloalkyl), sind, sofern nichts anderes gesagt ist, einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Der Begriff Metallion umfasst Alkaliionen und Erdalkaliionen, ist aber nicht darauf beschränkt.

Der Begriff Alkaliion steht in dieser Anmeldung für ein Ion aus der Reihe Lithium, Natrium, Kalium, Rubidium und Cäsium, bevorzugt aus der Reihe Lithium, Natrium und Kalium.

Der Begriff Erdalkaliion steht in dieser Anmeldung für ein Ion aus der Reihe Beryllium, Magnesium, Calcium, Strontium und Barium, bevorzugt aus der Reihe Magnesium und Calcium.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Wenn T in den Verbindungen der Formel (I) für Sauerstoff steht, liegen diese Verbindungen als N-Oxide vor.

Wenn T in den Verbindungen der Formel (I) für ein Elektronenpaar steht, liegen diese Verbindungen als Pyridine oder für G = N als Pyrimidine vor.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt sind und werden verwendet Verbindungen der Formel (I), in welchen eine Kombination der vorstehend als bevorzugt aufgeführten Bedeutungen vorliegt (Vorzugsbereich (1)).

Erfindungsgemäß besonders bevorzugt sind und werden verwendet Verbindungen der Formel (I), in welchen eine Kombination der vorstehend als besonders bevorzugt aufgeführten Bedeutungen vorliegt (Vorzugsbereich (2)).

Erfindungsgemäß ganz besonders bevorzugt sind und werden verwendet Verbindungen der Formel (I), in welchen eine Kombination der vorstehend als ganz besonders bevorzugt aufgeführten Bedeutungen vorliegt (Vorzugsbereich (3)).

Eine weitere bevorzugte Ausführungsform der Erfindung ist durch den Vorzugsbereich (4) definiert.

Eine weitere bevorzugte Ausführungsform der Erfindung ist durch den Vorzugsbereich (5) definiert.

Eine weitere bevorzugte Ausführungsform der Erfindung ist durch den Vorzugsbereich (6) definiert.

Eine weitere bevorzugte Ausführungsform der Erfindung ist durch den Vorzugsbereich (7) definiert.

Eine weitere bevorzugte Ausführungsform der Erfindung ist durch den Vorzugsbereich (8) definiert.

Eine weitere bevorzugte Ausführungsform der Erfindung betrifft Verbindungen der Formel (I), in welchen G für CH steht.

Eine weitere bevorzugte Ausführungsform der Erfindung betrifft Verbindungen der Formel (I), in welchen V für O steht.

Eine weitere bevorzugte Ausführungsform der Erfindung betrifft Verbindungen der Formel (I), in welchen R¹ für den Rest der Formel 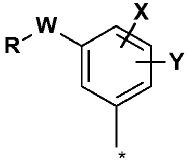 steht.

Eine weitere bevorzugte Ausführungsform der Erfindung betrifft Verbindungen der Formel (I), in welchen R² für H steht.

Die Verbindungen der Formel (I) können gegebenenfalls auch in Abhängigkeit von der Art der Substituenten als Stereoisomere, d.h. als geometrische und/oder als optische Isomere oder Isomerengemische vorliegen in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Vorzugsweise werden jedoch die optisch aktiven, stereoisomeren und tautomeren Formen der Verbindungen der Formel (I) und deren Salze erfindungsgemäß verwendet.

Als geeignete Salze der Verbindungen der Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzyl¬ethylen-diammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Tautomere Formen treten beispielsweise für den Fall auf, dass R³ für Hydroxy steht:

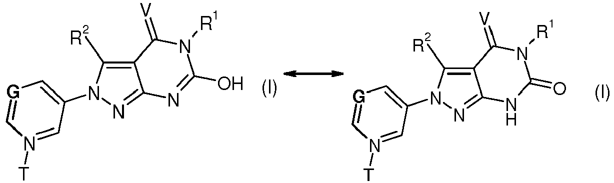

Die Erfindung betrifft daher sowohl die reinen Enantiomeren und Diastereomeren, als auch deren Gemische zur Bekämpfung von tierischen Schädlingen, zu denen Arthropoden und insbesondere Insekten zählen.

Weiter wurde gefunden, dass sich die neuen Verbindungen der Formel (I) nach den im Folgenden beschriebenen Verfahren herstellen lassen.

Die Verbindungen der Formel (I) können beispielsweise nach Verfahren A-1 und A-2 in zwei Schritten hergestellt werden, wie im folgenden Schema dargestellt. Het steht in diesem und den folgenden Schemata, wenn sich aus dem Zusammenhang nichts anderes ergibt, für den Rest 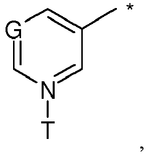 der über die mit dem Stern gekennzeichnete Bindung mit dem Stickstoff des Pyrazolrings verbunden ist.

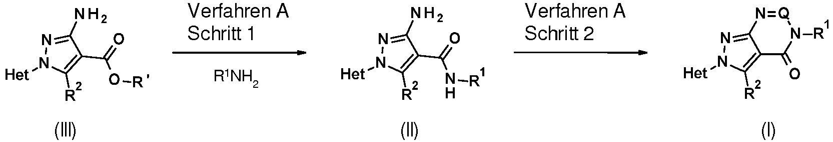

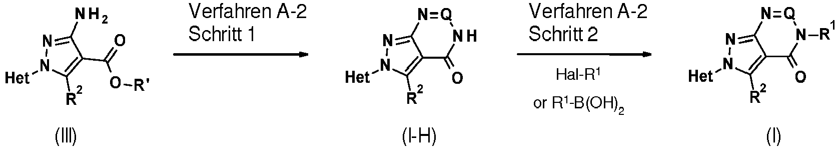

Die für die Verfahren A-1 und A-2 benötigten Aminopyrazole der Formel (III) können beispielweise nach den Verfahren B und C hergestellt werden. 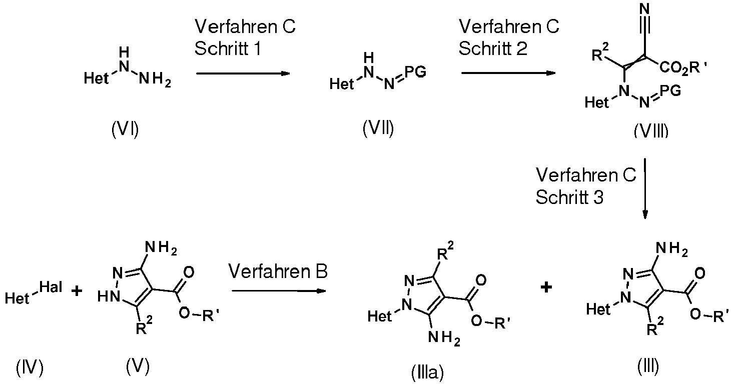 wobei Het, Q, R¹ und R² die oben angegebenen Bedeutungen haben, R'für Wasserstoff oder Alkyl (insbesondere Methyl und Ethyl) steht, Hal für Halogen (bevorzugt Chlor, Brom und Iod) und PG für eine geeignete Schutzgruppe steht.

### Verfahren A-1

Die Verbindungen der Formel (I) können mit Hilfe von literaturbekannten Methoden in zwei Schritten synthetisiert werden.

Im ersten Syntheseschritt können Verbindungen der Formel (III) nach verschiedenen Methoden zu Carbonsäureamiden der Formeln (II) umgesetzt werden. Für R' = Alkyl kann diese Umsetzung ohne Aktivierung erfolgen, (vgl. B. M. Trost und I. Fleming in Comprehensive Organic Synthesis, Ed. Pergamon, 1991, Vol. 6). Alternativ sind aus der Literatur Aktivierungsmethoden durch die Bildung eines Aluminiumamids bekannt (siehe T. Ooi und K. Marouka in Science of Synthesis, Ed. Georg Thieme, 2003, Vol. 7, 225-246). Diese Aluminiumamide können beispielweise aus den Aminen oder deren Salze durch Umsetzung mit Trimethylaluminium oder deren luft-stabilem Addukt mit 1,4-Diazobicyclo[2.2.3]oktan (DABCO) hergestellt werden (vgl. S. Woodward in Tet. Lett. 2006, 47, 5767-5769).

Alternativ können die Aminopyrazole der Formel (III), mit R' = Alkyl, in zwei Stufen zu den Amiden der Formeln (II) umgesetzt werden: zuerst Verseifung zu den Carboxylaten, zum Beispiel durch Umsetzung mit einer anorganischen Base (bevorzugt Natron- und Kalilaugen), gegebenenfalls in einem inerten organischen Lösungsmittel, gegebenenfalls lassen sich durch Ansäuern mit einer verdünnten Säure (z.B. wässrige Salzsäure) die Carbonsäuren der Formel (III) mit R' = Wasserstoff herstellen und isolieren; anschließende Amidierungsreaktion mit den gewünschten Aminen führt zu den Verbindungen der Formel (II). Für den Amidierungsschritt sind zahlreiche Reaktionsbedingungen beschrieben worden, z.B. G. Benz in Comprehensive Organic Synthesis, 1st Ed., Pergamon Press, Oxford, 1991, Vol. 6, S. 381-417; P.D. Bailey et al. in Comprehensive Organic Functional Group Transformation, 1st Ed., Elsevier Science Ltd., Oxford, 1995, Vol. 5, S. 257-308 und R.C. Larock in Comprehensive Organic Transformations, 2nd Ed., Wiley-VCH, New York, Weinheim, 1999, S. 1929-1994. Einige dieser Reaktionen verlaufen über intermediäre Carbonsäurechloride, die isoliert oder in situ erzeugt eingesetzt werden können.

Die Amidierungsreaktionen erfolgen gegebenenfalls in Gegenwart eines Kondensationsmittels, gegebenenfalls in Gegenwart eines Säureakzeptors und gegebenenfalls in Gegenwart eines Lösungsmittels.

Als Kondensationsmittel kommen alle üblicherweise für derartige Amidierungsreaktionen verwendbaren Kondensationsmittel infrage. Beispielhaft genannt seien Säurehalogenidbildner wie Phosgen, Phosphortrichlorid, Oxalylchlorid oder Thionylchlorid; Carbodiimide, wie *N,N'-*Dicyclohexylcarbodiimid (DCC) und 1-(3-Dimethylaminopropyl)-3-ethyl-carbodiimid (EDCI), oder andere übliche Kondensationsmittel, wie Phosphorpentoxid, Polyphosphorsäure, *N,N'-*Carbonyldiimidazol, 2-Chlorpyridin 1-Methoiodid (Mukaiyamas Reagenz), 2-Ethoxy-N-ethoxycarbonyl-1,2-dihydrochinolin (EEDQ), Triphenylphosphin/Tetrachlorkohlenstoff, Bromtripyrrolidino-phosphonium-hexafluorphosphat (BROP), O-(1*H*-Benzotriazol-1-yloxy)tris(dimethyl-amino)phosphonium-hexafluorphosphat (BOP), *N,N,N'*,*N'*-Bis(tetramethylen)cloruronium-tetrafluorborat, O-(*1H*-Benzotriazol-1-yl)-*N,N,N',N'*-tetramethyluronium-hexafluorphosphat (HBTU), O-(*1H*-Benzotriazol-1-yl)-*N,N,N',N'*-bis(tetramethylen)uronium-hexafluorphosphat, O-(*1H-*Benzotriazol-1-yl)-*N,N,N',N'*-tetramethyluronium-tetrafluorborat (TBTU), O-(*1H*-Benzotriazol-1-yl)-*N,N,N',N'*-bis(tetramethylen)uronium-tetrafluorborat, O-(7-Azabenzotriazol-1-yl)-*N*,*N*,*N'*,*N*'-tetramethyluronium-hexafluorphosphat (HATU), 1-Hydroxybenzotriazol (HOBt) und 4-(4,6-Dimethoxy-1,3,5-triazin-2-yl)-4-methylmorpholiniumsalz (DMT.MM), meistens als Chlorid verfügbar. Diese Reagenzien können separat oder in Kombination eingesetzt werden.

Als Säureakzeptor kommen alle üblichen anorganischen oder organischen Basen infrage, beispielsweise Triethylamin, Diisopropylethylamin, *N*-Methylmorpholin oder *N*,*N*-Dimethylaminopyridin. Das erfindungsgemäße Verfahren A wird gegebenenfalls in Gegenwart eines geeigneten Reaktionshilfstoffes wie beispielsweise *N*,*N*-Dimethylformamid oder *N*,*N*-Dimethylaminopyridin durchgeführt. Als Lösungs- oder Verdünnungsmittel kommen alle interten organischen Lösungsmittel in Betracht, beispielsweise aliphatische oder aromatische Kohlenwasserstoffe (wie Petrolether, Toluol), halogenierte Kohlenwasserstoffe (wie Chlortoluol, Dichlormethan, Chloroform, 1,2-Dichlorethan), Ether (wie Diethylether, Dioxan, Tetrahydrofuran, 1,2-Dimethoxyethan), Ester (wie Essigsäureethylester- oder - methylester), Nitrokohlenwasserstoffe (wie Nitromethan, Nitroethan, Nitrobenzol), Nitrile (wie Acetonitril, Benzonitril), Amide (wie *N*,*N*-Dimethylformamid, *N*,*N*-Dimethylacetamide, *N-*Methylformanilid, *N*-Methylpyrrolidon, Hexamethylphosphorsäuretriamid) sowie Dimethylsulfoxid oder Wasser oder Gemische der genannten Lösungsmittel.

Es können auch gemischte Anhydride zur Darstellung von Verbindungen der Formel (III) verwendet werden (vgl. J. Am. Chem. Soc. 1967, 5012). Bei diesem Verfahren können Chlorameisensäureester zum Einsatz kommen, wie z.B. Chlorameisensäureisobutylester und Chlorameisensäureisopropylester. Ebenfalls können dafür Diethylacetylchlorid, Trimethylacetylchlorid und ähnliche Verbindungen verwendet werden.

In einem zweiten Syntheseschritt können die Carbonsäureamide der Formel (II) zu den Verbindungen der Formel (I) cyclisiert werden.

Für Q = C-R³, worin R³ für H oder Alkyl steht, kann die Cyclisierung von Carbonsäurenamiden der Formel (II) mit einem Orthoester, wie Orthoameisensäuretriethylester oder Orthoessigsäuretriethylester, gegebenenfalls in Gegenwart eines Lösungs- und Verdünnungsmittel (beispielsweise in Gegenwart von Alkoholen wie Ethanol, aber auch in Gegenwart von *N*,*N*-Dimethylformamid oder *N,N-*Dimethylacetamid), gegebenenfalls in Gegenwart einer organischen Säure (zum Beispiel *para-*Toluolsulfonsäure oder Essigsäure) oder anorganischen Säure (zum Beispiel Salzsäure oder Schwefelsäure) in katalytischen oder stöchiometrischen Mengen oder im Überschuss durchgeführt werden. Die genannten Säuren können auch anstelle des Lösungs- oder Verdünnungsmittels eingesetzt werden. Für R³ = H finden sich Beispiele für solche Reaktionen mit Orthoameisensäuretriethylester in Archiv der Pharmazie 2000, 333(8), 261-266 (zur Herstellung von Chinazolinonen), J. Het. Chem. 1990, 27(7), 1953-1956 (idem.), WO 2010/54398 (zur Herstellung von Pyrazinopyrimidinonen). Für R³ = Methyl siehe zum Beispiel WO 2010/100189 (zur Herstellung von Chinazolinonen).

Für Q = C-R³, worin R³ für Alkyl oder Halogenalkyl steht, kann die Herstellung der Pyrazolopyrimidinone der Formel (I) auch durch Umsetzung der Carbonsäureamide der Formel (II) mit entsprechenden Carbonsäurehalogeniden oder Carbonsäureanhydriden nach literaturbekannten Methoden erfolgen, wie zum Beispiel in WO 2009/143049 für R³ = Methyl und in WO 2008/039489 für R³ = Trifluormethyl beschrieben.

Für Q = N können die Pyrazolopyrimidinone der Formel (I) durch Azodiazotierung der Carbonsäureamide der Formel (II) nach literaturbekannten Methoden hergestellt werden. Beispielsweise werden Verbindungen der Formel (II) bei 0 bis 5 °C mit einer Nitritquelle, wie Natriumnitrit oder Isobutylnitrit versetzt, typischerweise in Wasser, Alkohol oder einem polaren, inerten Lösungsmittel und in Gegenwart einer organischen oder anorganischen Säure. Beispiele für Reaktionsbedingungen finden sich in WO 2004/242572 oder in J. Amer. Chem. Soc. Perkin Trans. 1, 1980, 633-638.

### Verfahren A-2

*N*-Substituierte Pyrazolopyrimidinone der Formel (I) mit Q = C-R³, worin R³ für H oder Alkyl steht, lassen sich in zwei Schritten aus den Aminopyrazolen der Formel (III) herstellen, indem sie zuerst in Intermediate der Formel (I-H) umgewandelt und diese dann am Stickstoff substituiert werden.

Die Umsetzung der Aminopyrazole der Formel (III) zu Pyrazolopyrimidinonen der Formel (I-H) ist für Q = C-H in der Literatur bekannt, s. US 2007/0281949, durch Reaktion mit Formamidinacetat in Methoxyethanol über Nacht unter Rückfluss.

Die *N*-Substitution der Pyrazolopyrimidinone der Formel (I-H) kann auf unterschiedliche Weise erfolgen. Literaturbekannt sind *N*-Arylierungen von Pyrimidinonen durch S_{N}Ar-Reaktion mit einem geeignetem Arylsubstrat, wie z.B. durch Nitro-, Nitril- oder Trifluoromethylgruppen aktivierten Arylfluoriden in Anwesenheit einer Base und eines inerten organischen Lösungsmittels, s. Beispiele in DE 4431218. Für unterschiedliche Aryl- und Hetarylverbindungen findet die Umsetzung bevorzugt unter Übergangsmetall-Katalyse oder -Vermittlung statt. Zahlreiche beispielhafte Reaktionsbedingungen sind in der Literatur beschrieben, zum Beispiel in WO2007/146824. Bevorzugt werden Kupfer oder Kupfersalze, zum Beispiel Kupfer(I)-iodid, Kupfer(I)-oxid, Kupfer(I)-triflat oder Kupfer(II)-triflat, als Katalysator verwendet, häufig in Gegenwart eines Liganden, zum Beispiel Diamin-Liganden wie *N*,*N*-Dimethylethylendiamin, *N*,*N*-Dimethylethylendiamin oder *trans*-*N*,*N*-Dimethyl-1,2-cyclohexandiamin. Eine Übersicht findet sich zum Beispiel in Chem. Sci. 2010, Vol. 1, 13-31. Alternativ können 1,3-Diketone, wie z. B. 2,4-Pentandion, 2,2,6,6-Tetramethyl-3,5-heptandion oder Dibenzoylmethan, Aminosäuren, wie z.B. L-Prolin oder Glycin oder andere Verbindungen wie 8-Hydroxychinolin (Tetrahedron Lett. 2009, Vol. 50, 7293-7296), Dibenzylidenaceton, Bipyridin oder Phenanthrolin verwendet werden. In der Regel wird die Umsetzung in Anwesenheit einer Base, häufig Carbonat- oder Phosphat-Basen, wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N*,*N*-Dimethylformamid durchgeführt. Es können auch Additive wie z. B. Kaliumiodid, Cäsiumfluorid oder andere Salze eingesetzt werden.

Alternativ lassen sich derartige Umsetzungen unter Palladiumkatalyse durchführen, etwa durch Einsatz von Katalysatoren wie Palladiumacetat, Tetrakis(triphenylphosphin)palladium, Bis-(triphenylphosphin)-palladium(II)-chlorid, Tris-(dibenzylidenaceton)-dipalladium(0) in Gegenwart von Liganden, zum Beispiel 2,2'-Bis-(diphenylphosphino)-1,1'-binaphthyl, 9,9-Dimethyl-4,5-bis-(diphenylphosphino)-xanthen, 1,1'-Bis-(diphenylphosphino)-ferrocen, und Basen wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N*,*N*-Dimethylformamid.

Verbindungen der Formel (I) können alternativ durch Umsetzung von geeigneten Boronsäuren mit den Pyrazolopyrimidinonen der Formel (I-H) hergestellt werden. In der Regel finden die Reaktionen unter Katalyse oder Vermittlung durch Kupfer(II)-salze wie zum Beispiel Kupfer(II)-acetat, Kupfer(II)triflat oder auch durch Kupfer(I)-salze wie zum Beispiel Kupfer(I)-chlorid, Kupfer(I)-acetat unter Luft- oder Sauerstoffatmosphäre, häufig unter wasserentziehenden Bedingungen (zum Beispiel mit Molekularsieb) statt. Als Basen werden zum Beispiel Triethylamin, *N*-Ethyldiisopropylamin, Pyridin, 2,6-Lutidin, *N*-Methylmorpholin oder 1,8-Diazabicycloundec-7-en in geeigneten Lösungmitteln wie zum Beispiel Dichlormethan, Dichlorethan, Methanol, *N*,*N*-Dimethylformamid, Tetrahydrofuran, Dioxan, Acetonitril, Essigester oder Toluol verwendet. In der Literatur werden zahlreiche Beispiele beschrieben, unter anderem in WO 2008/062905 oder in WO 2009/133970 für Pyrimidinone. Zusammenfassende Übersichten finden sich zum Beispiel in Synthesis 2011, No. 6, 829-856 oder in Tetrahedron 2012, Vol. 68, 7735-7754. Anstelle der Boronsäure können auch andere Borverbindungen wie etwa Kaliumtrifluorborate, Boronsäureester etc. sowie andere Organometallverbindungen wie etwa Stannane, Silane oder Bismuthane verwendet werden.

Die in den Verfahren A-1 und A-2 benötigten Aminopyrazole der Formeln (III) können beispielweise nach den Verfahren B und C hergestellt werden.

### Verfahren B

Aminopyrazole der Formeln (III) und (IIIa) können in einem Schritt, zum Beispiel mittels einer Ullmann Reaktion, nach im Prinzip bekannten Methoden (vgl. Chem. Rev. 2008, 108, 3054-3131) aus den entsprechenden Bromiden der Formel (IV) und den Aminopyrazolen der Formel (V) hergestellt werden.

Beispiele für die Arylierung von Aminopyrazolen sind in WO 2007/039146 beschrieben. Für derartige Reaktionen werden beispielsweise Katalysatoren auf Kupfer(I)-Basis (z.B. Kupfer(I)iodid) benutzt, in Anwesenheit einer Base (z.B. Kaliumcarbonat), und eines Ligandes (z.B. *trans*-1,2-Diaminocyclohexan oder *trans*-*N*,*N*'-Dimethyl-1,2-cyclohexandiamin) oder einer Kombination davon, in einem geeignetem Lösungsmittel (z.B. Dioxan, *N*,*N*-Dimethylformamid, *N*,*N*-Dimethylacetamid oder Pyridin) oder einer Kombination von Lösungsmitteln. Für die Reaktion werden meistens Temperaturen zwischen 80 und 180°C benötigt.

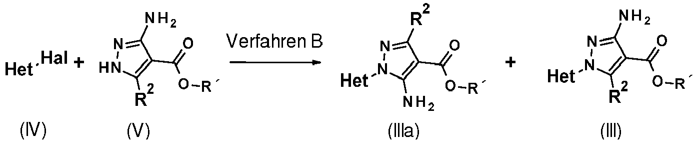

Bei der Durchführung des erfindungsgemäßen Verfahrens B kann jede für diese Reaktionen geeignete, handelsübliche Mikrowellenapparatur verwendet werden (z.B. Anton Paar Monowave 300, CEM Discover S, Biotage Initiator 60).

Die so hergestellten Aminopyrazole der Formeln (III) und (IIIa) können getrennt werden, zum Beispiel mittels chromatographischer Trennung über Kieselgel oder RP(C-18) oder durch Verrühren oder Umkristallisieren unter Verwendung geeigneter Lösungsmittel.

### Verfahren C

Alternativ zu Verfahren B können Aminopyrazole der Formel (III) nach literaturbekannten Methoden aus Hydrazinen der Formel (VI) oder deren Salzen (bevorzugt Hydrochloride) hergestellt werden (vgl. E. J. Med. Chem. 2011, 46, 3867-3876).

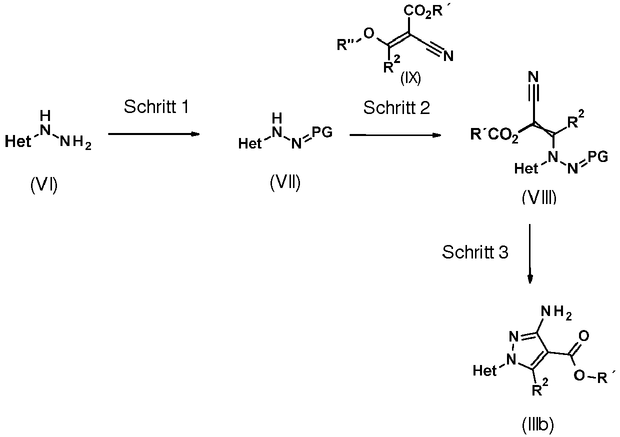

Im Schritt 1 wird das Hydrazin der Formel (VI) geschützt, z.B. durch Reaktion mit einem Aldehyd (typischerweise Benzaldehyd, vgl. Biorg. Med. Chem. Let 2004, 14, 4585-4589) unter Bildung eines Hydrazons der Formel (VII). Die geschützte Verbindung der Formel (VII) kann mit einem Cyanoderivat der Formel (IX) zu Verbindungen der Formel (VIII) umgesetzt werden (vgl. J. Het. Chem. 1990, 27, 1805-1807), typischerweise in einem inerten organischen Lösungsmittel (z.B. einem Alkohol) bei einer Temperatur zwischen 50°C und 100°C (Schritt 2). Anschließende Umsetzung von Verbindungen der Formel (VIII) mit einer Säure (z.B. Salzsäure) führt im Schritt 3 zu Aminopyrazolen der Formel (IIIb).

### Allgemeine Verfahren für die Oxidation von Thioethern zu Sulfoxiden und Sulfonen

Verindungen der Formel (I), in welchen W für SO steht (Sulfoxide) oder W für SO₂ steht (Sulfone) lassen sich durch Oxidation nach literaturbekannten Verfahren aus Verbindungen der Formel (I), in welchen W für S (Thioethern) herstellen, beispielsweise durch ein Oxidationsmittel in einem geeignetem Lösungs- bzw. Verdünnungsmittel. Als Oxidationsmittel eignen sich zum Beispiel verdünnte Salpetersäure, Wasserstoffperoxid, Oxone® und Peroxycarbonsäuren, wie etwa *meta-*Chlorperbenzoesäure. Als Lösungsmittel bzw. Verdünnungsmittel eignen sich inerte organische Lösungsmittel, typischerweise Acetonitril und halogenierte Lösungsmittel wie Dichlormethan, Chloroform oder Dichlorethan, sowie Wasser und Alkohole wie Methanol für die Reaktion mit Oxone®.

Möglich ist auch die Einführung geeigneter Aniline R¹-NH₂, Halogenide R¹-Hal oder Boronsäuren R¹-B(OH)₂, in welchen W für SO oder SO₂ steht, nach Verfahren A-1 oder A-2. Diese lassen sich aus den entsprechenden Vorstufen, in denen W für S steht, nach literaturbekannten Verfahren oxidieren, wie zum Beispiel in WO 2013/092350 beschrieben.

Zur Erzeugung enantiomeren angereicherten Sulfoxide eignen sich eine Vielfalt von Methoden, wie von A.R. Maguire in ARKIVOC, 2011(i), 1-110, beschrieben: metall-katalysierte asymmetrische Oxidationen von Thioethern, zum Beispiel mit Titanium oder Vanadium als meistbenutzten Katalysatorquellen, in Form von Ti(OⁱPr₄) oderVO(acac)₂, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nicht-metall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, beispielsweise durch präparative Trennung mittels einer chiralen HPLC.

### Erläuterung der Ausgangsstoffe und Zwischenprodukte

**Halogenide der Formel (IV)** (bevorzugt Chloride, Bromide und Iodide) sind kommerziell erhältlich oder können nach literaturbekannten Methoden synthetisiert werden, vgl. beispielsweise Y. Yamamoto, Heterocycles 1981, 16 (7), 1161-1164, für 3-Iodpyridin (Hal = Iod); S. M. E. Englert, J. Amer. Chem. Soc. 1929, 51(3), 863-866, für 3-Brompyridin (Hal = Brom); D. E. Pearson, J. Org. Chem. 1961, 26, 789-792, und für 3-Chlorpyridin (Hal = Chlor); WO 2006/074884 für 3-Brom-5-fluorpyridin und M. Schlosser, Eur. J. Org. Chem. 2002, 24, 4174-4180, für 3-Fluor-5-iodpyridin.

**Heterocyclische Hydrazine der Formel (VI)** sind kommerziell erhältlich oder können nach literaturbekannten Methoden aus den entsprechenden Halogeniden der Formel (IV) hergestellt werden, wie z.B. in WO 2010/015849 für diverse Heterocyclen beschrieben: durch Reaktion mit Hydrazinhydrat, gegebenenfalls in einem inerten organischen Lösungsmittel (z.B. Ethanol), bei Temperaturen zwischen 60 und 120 °C; oder durch Reaktion mit Di-*tert*-butyl-hydrazodicarboxylat und anschließender Spaltung der *tert*-Butylcarboxylatgruppen durch Versetzung mit einer Säure (typischerweise Salzsäure in einem organischen Lösungsmittel wie z.B. Dioxan; auch mit Trifluoressigsäure), was zur Bildung der entsprechenden Salze führt.

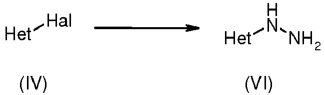

Kommerziell erhältlich sind z.B. folgende Hydrazine der Formel (VI): 3-Hydrazinylpyridin, 5-Hydrazinylpyrimidin.

**Cyanoverbindungen der Formel (IX)** können aus Cyanessigsäure-alkylester (bevorzugt -methylester und -ethylester) nach literaturbekannten Methoden hergestellt werden.

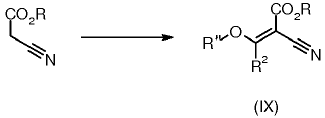

Kommerziell erhältlich sind z.B. Ethyl-2-cyan-3-ethoxyacrylat (R = R" = Ethyl; R² = Wasserstoff), Ethyl-2-cyan-3-ethoxybut-2-enoat - auch Ethyl-2-cyano-3-ethoxycroton genannt - (R = R" = Ethyl; R² = Methyl), Ethyl-(2E)-2-cyan-3-ethoxypent-2-enoat (R = R" = R² = Ethyl). Für andere Alkylgruppen kann die Synthese der Cyanoverbindungen der Formel (IX) nach der in J. Amer. Chem. Soc. 1956, 75, 5294-5299 beschriebenen Methode durchgeführt werden. Diese sieht die Umsetzung der entsprechenden Orthoester (z.B. Orthopropionsäuretriethylester für R² = Propyl) mit einem Cyanessigsäure-alkylester bei höheren Temperaturen vor.

**Aniline der Formel R¹-NH₂** sind kommerziell erhältlich oder können nach literaturbekannten Methoden hergestellt werden. Sie lassen sich in Verbindungen der Formel (X-1), (X-2) und (X-3) unterteilen.

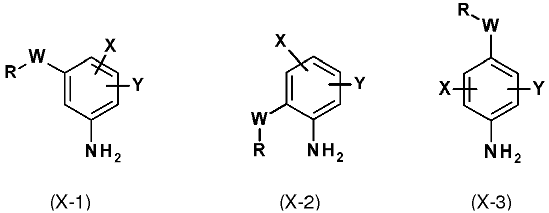

Aniline der Formel (X-1) sind literaturbekannt, z.B. aus JP 2007/284356, oder können anhand literaturbekannter Verfahren synthetisiert werden. In analoger Weise lassen sich die Aniline der Formel (X-2) und (X-3) synthetisieren.

Die Aniline der Formel (X-1), in denen W für S steht, können beispielsweise wie im folgenden Schema gezeigt hergestellt werden. 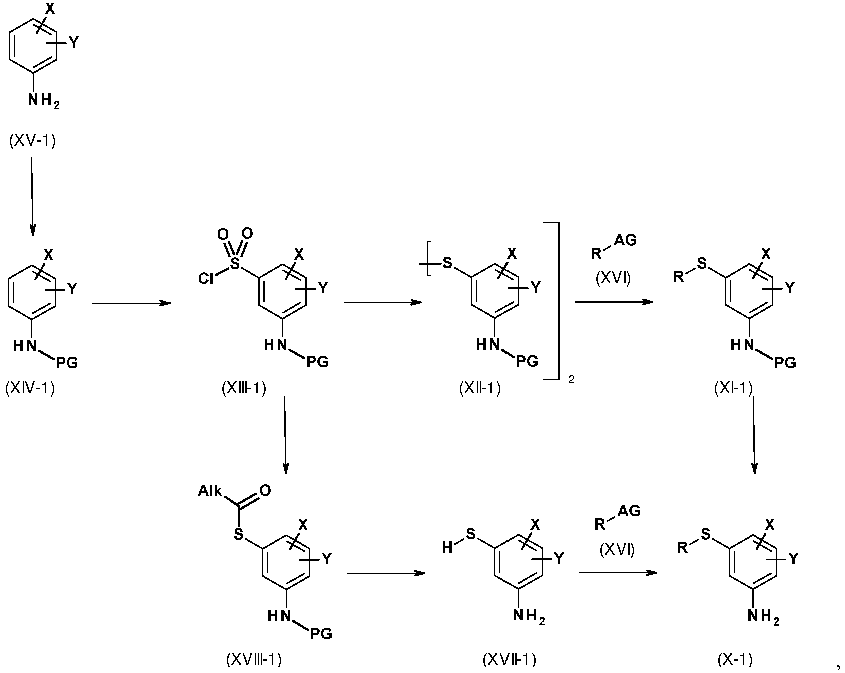 wobei X und Y die oben genannten Bedeutungen haben, AG für eine Abgangsgruppe und PG für eine Schutzgruppe steht.

Aniline der Formel (XV-1) sind entweder kommerziell erhältlich oder können durch bekannte Methoden hergestellt werden. Sie können mit einer geeigneten Schutzgruppe, wie z.B. einer Acetylgruppe, als Verbindungen der Formel (XIV-1) geschützt werden. Beispielsweise in Gegenwart von Säuren, Säureanhydriden oder Säurechloriden können die Aniline (XV-1) in die entsprechenden Anilide (XIV-1) überführt werden. Die Chlorsulfonierung der geschützen Aniline (XIV-1) mit Chlorsulfonsäure liefert die entsprechenden Sulfonylchloride (XIII-1). Die Reduktion der Sulfonylchloride (XIII-1) zu den Disulfiden (XII-1) ist mit literaturbekannten Methoden wie zum Beispiel Eisen in Salzsäure oder Iodid möglich. Die Umsetzung der Disulfide (XII-1) mit Halogenalkylelektrophilen der Formel (XVI), wobei AG für eine Abgangsgruppe wie zum Beispiel Chlor, Brom, Tosylat, Mesylat oder Triflat steht, liefert die Sulfide (XI-1). Die Schutzgruppe kann durch geeignete literaturbekannte Methoden abgespalten werden, man erhält Aniline der Formel (X-1).

Anstatt der Reduktion zum Disulfid (XII-1) kann das Sulfonylchlorid (XIII-1) mit einem geeigneten Reduktionsmittel, wie zum Beispiel Iod/Phosphor, zum Alkylthioat (XVIII-1) reduziert und anschließend durch eine geeignete Methode, wie zum Beispiel die Umsetzung mit Kaliumhydroxidlösung, zu Thiolen der Formel (XVII-1) umgesetzt werden. Die Umsetzung der Thiole (XVII-1) mit Halogenalkylelektrophilen der Formel (XVI), wobei AG für eine Abgangsgruppe wie zum Beispiel Chlor, Brom, Tosylat, Mesylat oder Triflat steht, liefert die Sulfide (X-1).

Ebenfalls bevorzugt können die Thioether der Formel (X-1) nach folgendem Schema hergestellt werden, 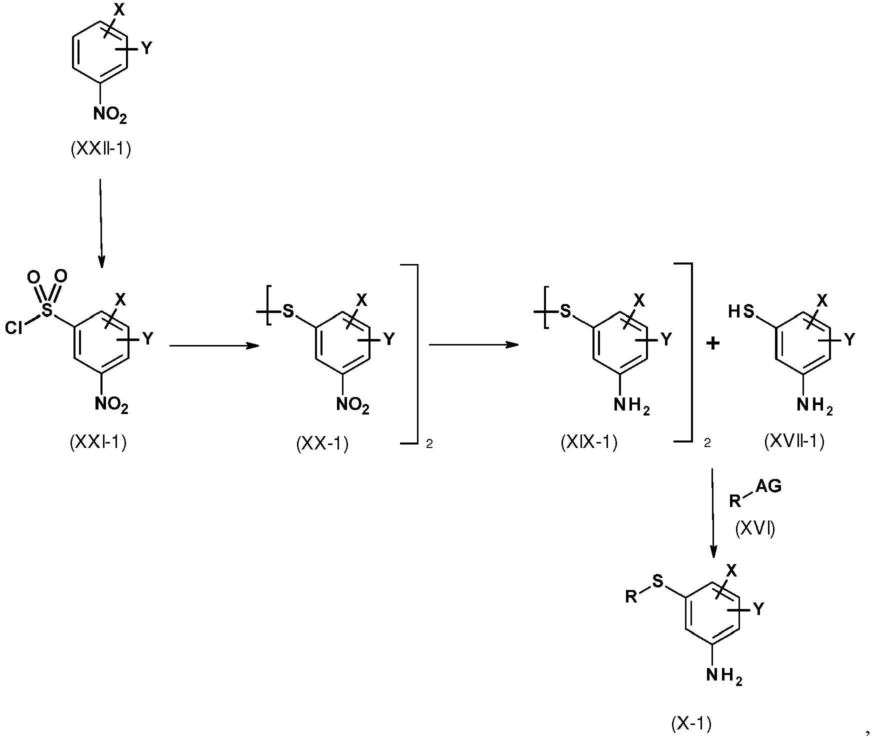 wobei X und Y die oben angegebenen Bedeutungen haben, AG für eine Abgangsgruppe und PG für eine Schutzgruppe steht.

Die Chlorsulfonierung der Nitroaromaten der Formel (XXII-1) mit Chlorsulfonsäure liefert die entsprechenden Sulfonylchloride (XXI-1). Die Reduktion der Sulfonylchloride (XXI-1) in die Bis(nitroaryl)disulfide (XX-1) ist mit literaturbekannten Methoden, wie zum Beispiel Umsetzung mit Iodid, möglich. Die Reduktion der Disulfide (XX-1) in die Disulfandiyldianiline (XIX-1), die teilweise als Gemisch mit den entsprechenden Aminoarylthiolen (XVII-1) entstehen, ist mit allgemein bekannten Reduktionsmitteln, wie zum Beispiel Wasserstoff, gegebenenfalls mit Hilfe heterogener Katalysatoren, wie zum Beispiel Raney-Nickel, Platin auf Aktivkohle oder Palladium auf Aktivkohle, möglich. Die Umsetzung der Disulfide (XIX-1) bzw. Thiophenole (XVII-1) mit Halogenalkylelektrophilen der Formel (XVI), wobei AG für eine Abgangsgruppe wie zum Beispiel Chlor, Brom, Iod, Tosylat, Mesylat oder Triflat steht, liefert die Aniline der Formel (X-1).

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereo¬isomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (= Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps" Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)-Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide,
weitere Wirkstoffe wie beispielsweise Afidopyropen, Azadirachtin, Benclothiaz, Benzoximate, Bifenazate, Bromopropylate, Chinomethionat, Cryolite,

Dicofol, Diflovidazin, Fluensulfone, Flometoquin, Flufenerim, Flufenoxystrobin, Flufiprole, Fluopyram, Flupyradifurone, Fufenozide, Heptafluthrin, Imidaclothiz, Iprodione, Meperfluthrin, Paichongding, Pyflubumide, Pyrifluquinazon, Pyriminostrobin, Tetramethylfluthrin und Iodmethan; desweiteren Präparate auf Basis von Bacillus firmus (I-1582, BioNeem, Votivo), sowie folgende Verbindungen:3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) und 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{5-[3-Chlor-5-(trifluormethyl)phenyl] -5-(trifluormethyl)-4,5-dihydro-1,2-oxazol-3-yl}-N-{2-oxo-2-[(2,2,2-trifluorethyl)amino]ethyl}-1-naphthamid (bekannt aus WO2009/002809), Methyl-2-[2-({[3-brom-1-(3-chlorpyridin-2-yl)-1H-pyrazol-5-yl]carbonyl}amino)-5-chlor-3-methylbenzoyl]-2-methylhydrazin-carboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethyl-hydrazincarboxylat (bekannt aus WO2005/085216), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}--1H-pyrazol-5-carboxamid (bekannt aus WO2010/069502), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 3-Chlor-N-(2-cyanpropan-2-yl)-N-[4-(1,1,1,2,3,3,3-heptafluorpropan-2-yl)-2-methylphenyl]phthalamid (bekannt aus WO2012/034472), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), (5S,8R)-1-[(6-Chlorpyridin-3-yl)methyl]-9-nitro-2,3,5,6,7,8-hexahydro-1H-5,8-epoxyimidazo[1,2-a]azepin (bekannt aus WO2010/069266), (2E)-1-[(6-Chlorpyridin-3-yl)methyl]-N'-nitro-2-pentylidenhydrazincarboximidamid (bekannt aus WO2010/060231), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imido-formamid und (1.64) O-[1-(4-Methoxyphenoxy)-3,3-dimethylbutan-2-yl]-1H-imidazol-1-carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}}phenyl)ethyliden]amino}oxy)methyl]phenyl}-2-(methoxyimino)-N-methylethanamid, (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid, (4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1), (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb" (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl {3-methyl-1-[(4-methylbenzoyl)amino]butan-2-yl}carbamat.
(12) Inhibitoren der Nukleinsäuresynthese, wiebeispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wiebeispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wiebeispielsweise (14.1) Binapacryl, (14.2) Dinocap, (14.3) Ferimzon, (14.4) Fluazinam und (14.5) Meptyldinocap.
(15) Weitere Verbindungen, wiebeispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c]dipyrrole-1,3,5,7(2H,6H)-tetron, (15.62) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazol-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl]-3-[3-methoxy-4-(prop-2-yn-1-yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N-{(E)-[(Cyclopropylmethoxy)imino][6-(difluormethoxy)-2,3-difluorphenyl]methyl}-2-phenyl-acetamid, (15.85) N-{(Z)-[(Cyclopropylmethoxy)imino][6-(difluormethoxy)-2,3-difluorphenyl]methyl}-2-phenylacetamid, (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazole-4-carboxamid, (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl {6-[({[(1-methyl-1H-tetrazol-5-yl)(phenyl)me-thylene]amino}oxy)methyl]pyridin-2-yl}carbamat, (15.91) Phenazine-1-carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Difluormethyl)-1-methyl-N-[4'-(trifluormethyl)biphenyl-2-yl]-1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3-dimethylbut-1-yn-1-yl)biphenyl-2-yl]-5-fluor-1,3-dimethyl-1H-pyrazol-4-carboxamid, (15.105) 3-(Difluormethyl)-N-(4'-ethynylbiphenyl-2-yl)-1-methyl-1H-pyrazol-4-carboxamid, (15.106) N-(4'-Ethynylbiphenyl-2-yl)-5-fluor-1,3-dimethyl-1H-pyrazol-4-carboxamid, (15.107) 2-Chlor-N-(4'-ethynylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N-[4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N-[4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl {6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N-[(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl]-2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-1,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl}-1H-1,2,4-triazol-5-yl thiocyanat, (15.134) 1-{[rel(2R,3R)-3-(2-Chlorphenyl)-2-(2,4-difluorphenyl)oxiran-2-yl]methyl}-1H-1,2,4-triazol-5-yl thiocyanat, (15.135) 5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol, (15.137) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridin-3-yl}-N-ethyl-N-methylimidoformamid, (15.154) N'-{5-Brom-6-[(1S)-1-(3,5-difluorphenyl)ethoxy]-2-methylpyridin-3-yl}-N-ethyl-N-methylimidoformamid, (15.155) N'-{5-Brom-6-[(cis-4-isopropylcyclohexyl)oxy]-2-methylpyridin-3-yl}-N-ethyl-N-methylimido-formamid, (15.156) N'-{5-Brom-6-[(trans-4-isopropylcyclohexyl)oxy]-2-methylpyridin-3-yl}-N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1-methyl-1H-pyrazol-4-carboxamid, (15.163) N-Cyclopropyl-3-(difluormethyl)-5-fluor-N-(5-fluor-2-isopropylbenzyl)-1-methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1-methyl-1H-pyrazol-4-carboxamid, (15.167) N-Cyclopropyl-3-(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3-(difluormethyl)-N-(2-ethyl-4,5-dimethylbenzyl)-5-fluor-1-methyl-1H-pyrazol-4-carboxamid, (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin. Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
Bacillus amyloliquefaciens, Stamm FZB42 (DSM 231179), oder Bacillus cereus, insbesondere B. cereus Stamm CNCM I-1562 oder Bacillus firmus, Stamm I-1582 (Accession number CNCM I-1582) oder Bacillus pumilus, insbesondere Stamm GB34 (Accession No. ATCC 700814) und Stamm QST2808 (Accession No. NRRL B-30087), oder Bacillus subtilis, insbesondere Stamm GB03 (Accession No. ATCC SD-1397), oder Bacillus subtilis Stamm QST713 (Accession No. NRRL B-21661) oder Bacillus subtilis Stamm OST 30002 (Accession No. NRRL B-50421) Bacillus thuringiensis, insbesondere B. thuringiensis subspecies israelensis (serotype H-14), Stamm AM65-52 (Accession No. ATCC 1276), oder B. thuringiensis subsp. aizawai, insbesondere Stamm ABTS-1857 (SD-1372), oder B. thuringiensis subsp. kurstaki Stamm HD-1, oder B. thuringiensis subsp. tenebrionis Stamm NB 176 (SD-5428), Pasteuria penetrans, Pasteuria spp. (Rotylenchulus reniformis nematode)-PR3 (Accession Number ATCC SD-5834), Streptomyces microflavus Stamm AQ6121 (= QRD 31.013, NRRL B-50550), Streptomyces galbus Stamm AQ 6047 (Acession Number NRRL 30232).

Beispiele für Pilze und Hefen, die als biologische Schädlingsbekämpfungsmittel eingesetzt werden bzw. verwendet werden können, sind:
Beauveria bassiana, insbesondere Stamm ATCC 74040, Coniothyrium minitans, insbesondere Stamm CON/M/91-8 (Accession No. DSM-9660), Lecanicillium spp., insbesondere Stamm HRO LEC 12, Lecanicillium lecanii, (ehemals bekannt als Verticillium lecanii), insbesondere Stamm KV01, Metarhizium anisopliae, insbesondere Stamm F52 (DSM3884/ ATCC 90448), Metschnikowia fructicola, insbesondere Stamm NRRL Y-30752, Paecilomyces fumosoroseus (neu: Isaria fumosorosea), insbesondere Stamm IFPC 200613, oder Stamm Apopka 97 (Accesion No. ATCC 20874), Paecilomyces lilacinus, insbesondere P. lilacinus Stamm 251 (AGAL 89/030550), Talaromyces flavus, insbesondere Stamm V117b, Trichoderma atroviride, insbesondere Stamm SC1 (Accession Number CBS 122089), Trichoderma harzianum, insbesondere T. harzianum rifai T39. (Accession Number CNCM I-952).

Beispiele für Viren, die als biologische Schädlingsbekämpfungsmittel eingesetzt werden bzw. verwendet werden können, sind:
Adoxophyes orana (Apfelschalenwickler) Granulosevirus (GV), Cydia pomonella (Apfelwickler) Granulosevirus (GV), Helicoverpa armigera (Baumwollkapselwurm) Nuklear Polyhedrosis Virus (NPV), Spodoptera exigua (Zuckerrübeneule) mNPV, Spodoptera frugiperda (Heerwurm) mNPV, Spodoptera littoralis (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
Agrobacterium spp., Azorhizobium caulinodans, Azospirillum spp., Azotobacter spp., Bradyrhizobium spp., Burkholderia spp., insbesondere Burkholderia cepacia (ehemals bekannt als Pseudomonas cepacia), Gigaspora spp., oder Gigaspora monosporum, Glomus spp., Laccaria spp., Lactobacillus buchneri, Paraglomus spp., Pisolithus tinctorus, Pseudomonas spp., Rhizobium spp., insbesondere Rhizobium trifolii, Rhizopogon spp., Scleroderma spp., Suillus spp., Streptomyces spp..

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,,,Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lager-raum nach den üblichen Behandlungsmethoden, z.B. durch Tauchen, Sprühen, Ver¬dampfen, Ver¬nebeln, Streuen, Auf¬streichen, In¬jizieren und bei Ver¬mehrungs¬material, ins¬besondere bei Saatgut, weiterhin durch ein- oder mehr¬schichtiges Um¬hüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid. Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, ohne sie zu beschränken. Die Produkte wurden mittels 1H-NMR Spektroskopie und/oder LC/MS (Liquid Chromatography Mass Spectrometry) charakterisiert.

Die Bestimmung der logP Werte erfolgte gemäß OECD Guideline 117 (EC Directive 92/69/EEC) durch HPLC (High Performance Liquid Chromatography) an reversed-phase (RP) Säulen (C18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker Avance 400, ausgestattet mit einem Durchflussprobenkopf (60 µl Volumen), bestimmt. In Einzelfällen wurden die NMR Spektren mit einem Bruker Avance II 600 gemessen.

Die NMR-Daten ausgewählter Beispiele werden in klassischer Form (δ-Werte, Multiplettaufspaltung, Anzahl der H-Atome) aufgeführt. Die Aufspaltung der Signale wurde wie folgt beschrieben: s (Singulett), d (Duplett), t (Triplett), q (Quartett), quint (Quintuplett), m (Multiplett), b (für breite Signale). Als Lösungsmittel wurden CD₃CN, CDCl₃ oder D6-DMSO verwendet, wobei als Referenz Tetramethylsilan (0.00 ppm) eingesetzt wurde.

### Herstellbeispiele

### Beispiel 1

### 5-{2-Fluor-4-methyl-5-[(2,2,2-trifluorethyl)sulfanyl]phenyl}-2-(pyridin-3-yl)-2,5-dihydro-4H-pyrazolo[3,4-d]pyrimidin-4-on (I-1-1)

### Schritt 1: 3-Amino-N-{2-fluor-4-methyl-5-[(2,2,2-trifluorethyl)sulfanyl]phenyl}-1-(pyridin-3-yl)-1H-pyrazol-4-carboxamid (II-1)

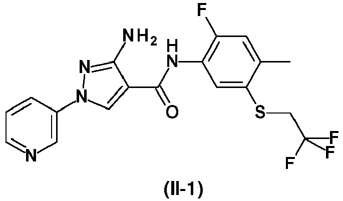

Zu 500 mg (2,09 mmol) 2-Fluor-4-methyl-5-[(2,2,2-trifluorethyl)sulfanyl]anilin in 1,3 ml 1,2-Dichlorethan wurden langsam 2,09 ml einer 2M Trimethylaluminium-Lösung in Toluol getropft und 1 Stunde (h) bei Raumtemperatur gerührt. 562 mg (2,09 mmol) Ethyl-3-amino-1-(pyridin-3-yl)-*1H-*pyrazol-4-carboxylat (III-1) wurden in 1,3 ml 1,2-Dichlorethan zugegeben und das Reaktionsgemisch über Nacht unter Rückfluss erhitzt. Nach dem Abkühlen wurde das Reaktionsgemisch mit Wasser verdünnt und vorsichtig auf eine 10%ige Kalium-Natrium-Tartrat-Lösung gegeben. Das Gemisch wurde drei Mal mit Methylenchlorid ausgeschüttelt, die organische Phase wurde abgetrennt, über Natriumsulfat getrocknet, filtriert und eingeengt. Der Rückstand wurde auf Kieselgel aufgezogen und über einer Kieselgel-Kartusche mittels präparativer MPLC gereinigt (Laufmittel Cyclohexan / Essigester). Die isolierte Fraktion lieferte 551 mg (100% Reinheit nach LC/MS, 62% d. Th.) der Titelverbindung.
logP[a]: 2,72; logP[b]: 2,63; 1H-NMR (D6-DMSO, 400MHz) δ ppm: 9,61(d,1H), 9,05(s,1H), 8,95(d,1H), 8,49-8,48(m,1H), 8,06-8,02(m,1H), 7,91(d,1H), 7,57-7,53(m,1H), 7,29(d,1H), 5,89(s,2H), 3,85(q,2H), 2,41(s,3H)

### Schritt 2: 5-{2-Fluor-4-methyl-5-[(2,2,2-trifluorethyl)sulfanyl]phenyl}-2-(pyridin-3-yl)-2,5-dihydro-4H-pyrazolo[3,4-d]pyrimidin-4-on (I-1-1)

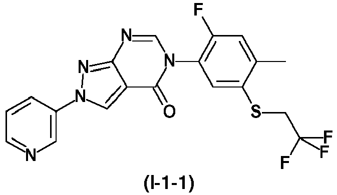

Zu 170 mg (0,40 mmol) 3-Amino-*N*-{2-fluor-4-methyl-5-[(2,2,2-trifluorethyl)sulfanyl]phenyl}-1-(pyridin-3-yl)-*1H*-pyrazol-4-carboxamid (II-1) in 2 ml *N*,*N*-Dimethylacetamid wurden 34,0 mg (0,20 mmol) *p*-Toluolsulfonsäure und 200 µl (178 mmol, 1,20 mmol) Orthoameisensäuretriethylester gegeben. Das Reaktionsgemisch wurde für 1 h bei 200 Watt in einer CEM Discover Mikrowelle auf 130 °C erhitzt. Nach Abkühlen wurde das Reaktionsgemisch mit RP-18-Material vermischt und das Lösemittel unter vermindertem Druck entfernt. Der Rückstand wurde über einer RP-18-Kartusche mittels präparativer MPLC gereinigt (Laufmittel Wasser / Acetonitril). Die isolierte Fraktion enthielt 80,0 mg (96% Reinheit nach LC/MS, 44% d. Th.) der Titelverbindung.
logP[a]: 2,65; logP[b]: 2,63; 1H-NMR (D6-DMSO, 400MHz) δ ppm: 9,59(d,1H), 9,29(s,1H), 8,68(dd,1H), 8,47-8,44(m,1H), 8,36(s,1H), 7,87(d,1H), 7,68-7,64(m,1H), 7,46(d,1H), 4,03(q,2H), 2,46(s,3H)

### Beispiel 2

### 3-[2-Fluor-4-methyl-5-(2,2,2-trifluoroethylsulfinyl)phenyl]-6-(3-pyridyl)pyrazolo[3,4-d]triazin-4-on (I-1-2) und 3-[2-Fluor-4-methyl-5-(2,2,2-trifluoroethylsulfanyl)phenyl]-6-(3-pyridyl)pyrazolo[3,4-d]triazin-4-on (I-1-3)

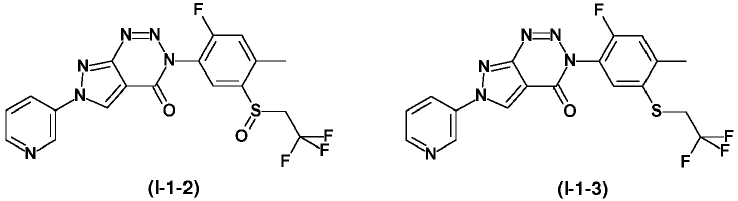

Zu 180 mg (0,42 mmol) 3-Amino-*N*-{2-fluor-4-methyl-5-[(2,2,2-trifluorethyl)sulfanyl]phenyl}-1-(pyridin-3-yl)-*1H*-pyrazol-4-carboxamid (II-1) in 5 ml Wasser und 5 ml konzertrierter Salzsäure wurde eine Lösung aus 248 mg (3,60 mmol) Natriumnitrit in 5 ml Wasser getropft. Der Ansatz wurde 6 Stunden bei 70 °C gerührt. Nach dem Abkühlen wurde das Reaktionsgemisch auf Methylenchlorid gegossen und der pH Wert wurde mit konzentrierter Natriumbicarbonat-Lösung auf 7 eingestellt. Das Gemisch wurde drei Mal mit Methylenchlorid extrahiert, die vereinten organischen Phasen mit Wasser gewaschen, über Natriumsulfat getrocknet, filtriert und eingeengt. Der Rückstand wurde auf RP-18-aufgezogen und über einer RP-18-Kartusche mittels präparativer MPLC gereinigt (Laufmittel Wasser / Acetonitril). Die isolierten Fraktionen enthielten 64,0 mg (100% Reinheit nach LC/MS, 33% d. Th.) 3-[2-Fluor-4-methyl-5-(2,2,2-trifluoroethylsulfinyl)phenyl]-6-(3-pyridyl)pyrazolo[3,4-d]triazin-4-on (I-1-2) und 72,0 mg (100% Reinheit nach LC/MS, 39% d. Th.) 3-[2-Fluor-4-methyl-5-(2,2,2-trifluoroethylsulfanyl)phenyl]-6-(3-pyridyl)pyrazolo[3,4-d]triazin-4-on (I-1-3).
(I-1-2) logP[a]: 2,22; logP[b]: 2,19; 1H-NMR (D6-DMSO, 400MHz) δ ppm: 9,84(s,1H), 9,37(s,1H), 9,36(d,1H), 8,78-8,76(m,1H), 8,20(d,1H), 7,75-7,71(m,1H), 7,64(d,1H), 4,39-4,01(m,2H), 3H unter dem DMSO-Peak.
(I-1-3) logP[a]: 3,18; logP[b]: 3,14; 1H-NMR (D6-DMSO, 400MHz) δ ppm: 9,84(s,1H), 9,36(s,1H), 8,78-8,76(m,1H), 8,56-8,53(d,1H), 7,95(d,1H), 7,75-7,71(m,1H), 7,52(d,1H), 3,99(q,2H), 3H unter dem DMSO-Peak.

### Beispiel 3

### 5-{2-Fluor-4-methyl-5-[(2,2,2-trifluorethyl)sulfinyl]phenyl}-2-(pyridin-3-yl)-2,5-dihydro-4H-pyrazolo[3,4-d]pyrimidin-4-on (I-1-4)

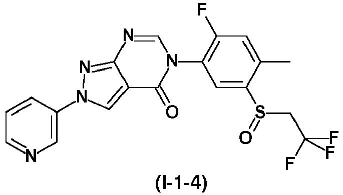

Zu einer Lösung von 61,0 mg (0,14 mmol) 5-{2-Fluor-4-methyl-5-[(2,2,2-trifluorethyl)sulfanyl]phenyl}-2-(pyridin-3-yl)-2,5-dihydro-*4H*-pyrazolo[3,4-d]pyrimidin-4-on (I-1-1) in 3 ml Methylenchlorid wurden bei 0 °C 35,4 mg (0,14 mmol) meta-Chlorperbenzoesäure gegeben. Das Reaktionsgemisch wurde 2 Stunden bei Raumtemperatur gerührt, dann mit Methylenchlorid verdünnt und mit konzentrierter Natriumbicarbonat-Lösung versetzt. Nach 10 Minuten wurden die Phasen getrennt, die organische Phase wurde mit RP-18-Material vermischt und das Lösemittel wurde unter vermindertem Druck entfernt. Der Rückstand wurde über einer RP-18-Kartusche mittels präparativer MPLC gereinigt (Laufmittel Wasser / Acetonitril). Die isolierte Fraktion enthielt 36,0 mg (98,5% Reinheit nach LC/MS, 56% d. Th.) der Titelverbindung.
logP[a]: 1,79; logP[b]: 1,78; 1H-NMR (D6-DMSO, 400MHz) δ ppm: 9,60(s,1H), 9,30(d,1H), 8,68(d,1H), 8,47-8,42(m,2H), 8,11(d,1H), 7,67-7,65(m,1H), 7,58(d,1H), 4,35-4,05(m,2H), 3H unter dem DMSO-Peak

### Beispiel 4

### 5-[2-Fluor-4-methyl-5-(2,2,2-trifluoroethylsulfinyl)phenyl]-2-(1-oxidopyridin-1-ium-3-yl)pyrazolo[3,4-d]pyrimidin-4-on (I-1-5)

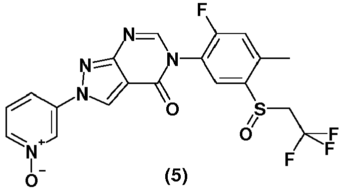

Zu einer Lösung von 100 mg (0,23 mmol) 5-{2-Fluor-4-methyl-5-[(2,2,2-trifluorethyl)sulfanyl]phenyl}-2-(pyridin-3-yl)-2,5-dihydro-*4H*-pyrazolo[3,4-d]pyrimidin-4-on (I-1-1) in 3 ml Methylenchlorid wurden bei 0 °C 119 mg (0,48 mmol) meta-Chlorperbenzoesäure gegeben. Das Reaktionsgemisch wurde über Nacht bei Raumtemperatur gerührt und anschließend mit 28,0 mg (0,12 mmol) meta-Chlorperbenzoesäure versetzt. Nach einer weiteren Nacht bei Raumtemperatur wurde das Reaktionsgemisch mit Methylenchlorid verdünnt und mit konzentrierter Natriumbicarbonat-Lösung versetzt. Nach 10 Minuten wurde ein Feststoff abgesaugt, der mit Wasser und Dichlormethan gewaschen und getrocknet wurde. Dieser enthielt 43,0 mg (78% Reinheit nach LC/MS, 31% d. Th.) der Titelverbindung.
logP[a]: 1,39; logP[b]: 1,33; 1H-NMR (D6-DMSO, 400MHz) δ ppm: 9,60(s,1H), 9,02(s,1H), 8,44(s,1H), 8,31(d,1H), 8,11(d,1H), 8,03(d,1H), 7,65-7,57(m,2H), 4,32-4,05(m,2H), 3H unter dem DMSO-Peak

### Synthese von Intermediaten

### Ethyl-5-amino-1-(pyridin-3-yl)-1H-pyrazol-4-carboxylat (IIIa-1) und Ethyl-3-amino-1-(pyridin-3-yl)-1H-pyrazol-4-carboxylat (III-1) nach Verfahren B

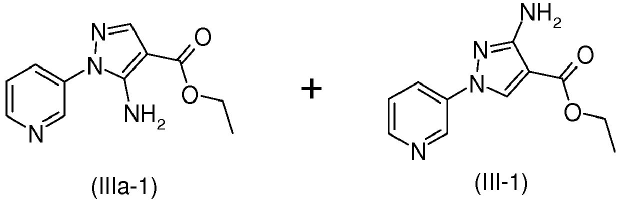

82,3 g (0,59 mol) Kaliumcarbonat wurden in einem Dreihalskolben vorgelegt. Dieser wurde unter Argon ausgeheizt und nacheinander wurden 2,70 g (0,014 mol) Kupfer(I)iodid, 44,0 g (0,28 mol) Ethyl-3-amino-4-pyrazolcarboxylat und 440 ml *N*,*N*-Dimethylacetamid zugegeben. Die Suspension wurde 10 Minuten gerührt und anschließend 7,18 g (0,056 mol) *trans*-1,2-Diaminocyclohexan und 53,77 g (0,34 mol) 3-Brompyridin zugegeben. Das Reaktionsgemisch wurde auf Rückflusstemperatur gebracht und über Nacht bei 145 °C gerührt. Nach dem Abkühlen wurde das Reaktionsgemisch abgesaugt, die Mutterlauge eingeengt und der Rückstand mittels MPLC über einer RP(C-18) Säule mit Acetonitril / Wasser / 0,1% Ameisensäure gereinigt. Eine erste Fraktion enthielt 39 g eines Gemisches von 70% Ethyl-5-amino-1-(pyridin-3-yl)-*1H*-pyrazol-4-carboxylat (IIIa-1) und 30% Ethyl-3-amino-1-(pyridin-3-yl)-*1H*-pyrazol-4-carboxylat (III-1), laut LC/MS. Eine zweite Fraktion enthielt 10 g bestehend aus 83% Ethyl-3-amino-1-(pyridin-3-yl)-*1H*-pyrazol-4-carboxylat (IIIb-1) nach LC/MS. Eine Trennung von (IIIa-1) und (III-1) aus der ersten Fraktion mittels präparativer HPLC lieferte weitere 24,5 g (96% Reinheit) Ethyl-5-amino-1-(pyridin-3-yl)-*1H*-pyrazol-4-carboxylat als Formiat (IIIa-1) und 11,5 g (99% Reinheit) Ethyl-3-amino-1-(pyridin-3-yl)-*1H*-pyrazol-4-carboxylat (III-1).
(IIIa-1).HCOOH logP[a]: 1,18; logP[b]: 1,27; 1H-NMR (D6-DMSO, 400MHz) δ ppm: 12,8(bs,1H), 8,78(d,1H), 8,62(dd,1H), 8,14(s,1H), 7,99-7,96(m,1H), 7,76(s,1H), 7,58(dd,1H), 6,51(bs,2H), 4,23(q,2H), 1,28(t,3H)
(III-1) logP[a]: 1,12; logP[b]: 1,32; 1H-NMR (D6-DMSO, 400MHz) δ ppm: 9,06(d,1H), 8,86(s,1H), 8,47(d,1H), 8,19(bd,1H), 7,49(dd,1H), 5,78(bs,2H), 4,26(q,2H), 1,30(t,3H)

### Ethyl-3-amino-1-(pyridin-3-yl)-1H-pyrazol-4-carboxylat (III-1) nach Verfahren C

### Schritt 1: Benzaldehydpyridin-3-ylhydrazon (VII-1)

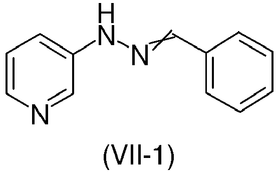

Zu 15,00 g (103,0 mol) 3-Hydrazinopyridinhydrochlorid (1:1) und 9,49 g (68,7 mmol) Kaliumcarbonat in 150 ml Toluol gab man langsam 7 ml (68,7 mmol) Benzaldehyd gelöst in 100 ml Toluol. Das Reaktionsgemisch wurde über Nacht unter Rückfluss (mit Wasserabscheider) gerührt. Nach dem Abkühlen wurden die unlöslichen Anteile abgesaugt. Der feste Rückstand wurde mehrmals in Essigester verrührt. Der Feststoff wurde abgesaugt und mehrmals heiß in Isopropanol verrührt. Die unlöslichen Anteile wurden abgesaugt und verworfen, das Filtrat wurde eingeengt. Die so erhaltenen 5,50 g (98% Reinheit, 40% d. Th.) der Titelverbindung wurden direkt weiter umgesetzt.
logP[a]: 0,86; logP[b]: 2,22

### Schritt 2: Ethyl-3-[2-benzyliden-1-(pyridin-3-yl)hydrazino]-2-cyanacrylat (VIII-1)

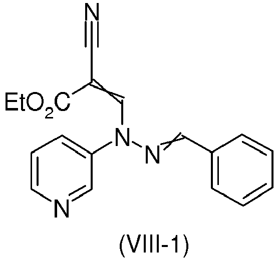

Zu 5,50 g (27,9 mmol) Benzaldehydpyridin-3-ylhydrazon in 14 ml Toluol wurden 4,72 g (27,9 mmol) Ethyl-2-cyan-3-ethoxyacrylat gegeben. Das Reaktionsgemisch wurde zunächst für 2 h und nach Zugabe einer Spatelspitze *para*-Toluolsulfonsäure für weitere 2 h unter Rückfluß erhitzt. Nach dem Abkühlen wurde der ausgefallene Feststoff abgesaugt und die organische Phase verworfen. Der Feststoff wurde nochmal in 15 ml Toluol vorgelegt und mit 1,69 g (10,0 mmol) Ethyl-2-cyan-3-ethoxyacrylat versetzt. Das Reaktionsgemisch wurde über Nacht unter Rückfluß erhitzt, zur Abkühlung stehen gelassen und anschließend mit Toluol verdünnt. Nach Zugabe von ca. 0.5 ml Acetonitril wurde der unlösliche Rückstand abgesaugt und unter Vakuum getrocknet. Isoliert wurden 3,11 g (90% Reinheit, 31% d. Th.) der Titelverbindung.
logP[a]: 2,54; logP[b]: 2,48

### Schritt 3: Ethyl-3-amino-1-(pyridin-3-yl)-1H-pyrazol-4-carboxylat (III-1)

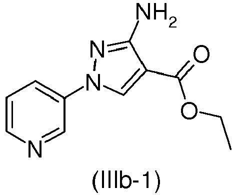

3,00 g (9,37 mmol) Ethyl-3-[2-benzyliden-1-(pyridin-3-yl)hydrazino]-2-cyanacrylat wurden in 11 ml Ethanol vorgelegt und mit 1,1 ml (13,11 mmol) einer 37%igen Salzsäurelösung versetzt. Das Reaktionsgemisch wurde 1 h unter Rückfluß erhitzt und nach dem Abkühlen eingeengt. Der Rückstand wurde zweimal in lauwarmem Toluol verrührt. Der Feststoff wurde abgesaugt und unter Vakuum getrocknet. Isoliert wurden 2,64 g (92% Reinheit, 97% d. Th.) der Titelverbindung.

Weitere Verbindungen der Formel (I) sind in der folgenden Tabelle aufgeführt.

**Tabelle 1**

| | | | | | | | |
|---|---|---|---|---|---|---|---|
| Verbindungen der Formel (I-1) | | | | | | | |
| 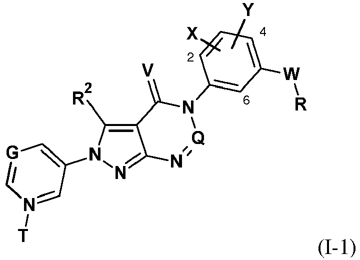 | | | | | | | |
| in welcher V für Sauerstoff steht, R² für Wasserstoff steht und die übrigen Variablen die in der Tabelle angegebenen Bedeutungen haben | | | | | | | |

| **Bsp.-Nr.** | **G** | **Q** | **R** | **X** | **Y** | **W** | **T** |
|---|---|---|---|---|---|---|---|
| I-1-1 | CH | CH | CF₃CH₂ | 2-F | 4-CH₃ | S | - |
| I-1-2 | CH | N | CF₃CH₂ | 2-F | 4-CH₃ | SO | - |
| I-1-3 | CH | N | CF₃CH₂ | 2-F | 4-CH₃ | S | - |
| I-1-4 | CH | CH | CF₃CH₂ | 2-F | 4-CH₃ | SO | - |
| I-1-5 | CH | CH | CF₃CH₂ | 2-F | 4-CH₃ | SO | O |
| I-1-6 | CH | CH | CH₃ | H | H | S | - |
| I-1-7 | CH | CH | CF₃CH₂ | H | 4-CH₃ | S | - |
| I-1-8 | CH | CH | CF₃CH₂ | H | 4-CH₃ | SO | - |
| I-1-9 | CH | CH | CF₃CH₂ | 2-F | H | S | - |
| I-1-10 | CH | CH | CF₃CH₂ | 2-F | H | SO | - |
| I-1-11 | CH | CH | cPrCH₂ | H | H | S | - |
| I-1-12 | CH | CH | CF₃CH₂ | H | H | S | - |
| I-1-13 | CH | CH | CF₃CH₂ | 2-F | 4-Cl | S | - |
| I-1-14 | CH | CH | CF₃CH₂ | 2-Cl | 4-Cl | S | - |
| I-1-15 | CH | CH | CF₃CH₂ | 2-Cl | 4-Cl | SO | O |
| I-1-16 | CH | CH | CF₃CH₂ | H | H | SO | - |
| I-1-17 | CH | CH | CF₃CH₂ | 2-CH₃ | 4- CH₃ | S | - |
| I-1-18 | CH | CH | CF₃CH₂ | 2-F | 4-Cl | SO | - |
| I-1-19 | CH | CH | CF₃CH₂ | 2-Cl | 4-Cl | SO | - |
| I-1-20 | CH | CH | CF₃CH₂ | 2-CH₃ | 4-CH₃ | SO | - |
| I-1-21 | CH | CH | cPrCH₂ | H | H | SO | - |
| I-1-22 | CH | C-CH₃ | CF₃CH₂ | 2-F | 4-CH₃ | S | - |

**Tabelle 2**

| Analytische Daten zu den angegebenen Verbindungen | | | |
|---|---|---|---|
| **Bsp.-Nr.** | **logP[a]** | **logP[b]** | **1H-NMR (D6-DMSO, 400 MHz) σ (ppm)** |
| I-1-6 | 1,82 | 1,78 | 9,58(s,1H), 9,30(d,1H), 8,67(d,1H), 8,46(d,1H), 8,34(s,1H), 7,67-7,64(m,1H), 7,51-7,38(m,3H), 7,27(d,1H), 3H unter dem DMSO-Peak |
| I-1-7 | 2,51 | 2,56 | 9,58(s,1H), 9,30(s,1H), 8,68-8,67(m1H), 8,46(d,1H), 8,31(s,1H), 7,70-7,64(m,2H), 7,43(d,1H), 7,33(d,1H), 4,08(q,2H), 2,42(s,3H) |
| I-1-8 | 1,65 | 1,67 | 9,59(s,1H), 9,31(d,1H), 8,67(d,1H), 8,45(dd,1H), 8,41(s,1H), 7,96(d,1H), 7,69-7,64(m,2H), 7,56(d,1H), 4,26-4,10(m,2H), 2,47(s,3H) |
| I-1-9 | 2,32 | 2,35 | 9,61(s,1H), 9,30(d,1H), 8,68(dd,1H), 8,48-8,44(m,1H), 8,39(s,1H), 7,88(dd,1H), 7,74-7,71(m,1H), 7,68-7,64(m,1H), 7,51(t,1H), 4,10(q,2H) |
| I-1-10 | 1,53 | 1,55 | 9,63(s,1H), 9,30(d,1H), 8,68(dd,1H), 8,48-8,43(m,2H), 8,15-8,14(m,1H), 8,05-8,01(m,1H), 7,80(t,1H), 7,68-7,65(m,1H), 4,35-4,10(m,2H) |

**Tabelle 3**

| | | | | | | | |
|---|---|---|---|---|---|---|---|
| Verbindungen der Formel (I-2) | | | | | | | |
| 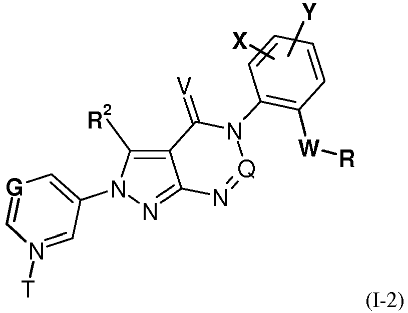 | | | | | | | |
| in welcher V für Sauerstoff steht, R² für Wasserstoff steht und die übrigen Variablen die in der Tabelle angegebenen Bedeutungen haben: | | | | | | | |

| **Bsp.-Nr.** | **G** | **Q** | **R** | **X** | **Y** | **W** | **T** |
|---|---|---|---|---|---|---|---|
| I-2-23 | CH | CH | CH₃ | H | H | S | - |

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| **Bsp.-Nr.** | logP [b] | logP [a] | |
|---|---|---|---|
| I-1-11 | 2,41 | 2,50 | ¹H-NMR(400,0 MHz, d₆-DMSO): δ= 9,574(15,4);9,300(5,3);9,294(5,4);8,6 77(3,7);8,674(4,0);8,665(3,9);8,662(4,0);8,475(2,3);8,472(2,7);8,468(2,6);8 ,465(2,3);8,454(2,5);8,451(2,7);8,448(2,9);8,444(2,4);8,330(16,0);7,670(3,2 );7,658(3,1);7,649(3,1);7,637(3,0);7,501(2,2);7,493(4,4);7,489(7,9);7,482(1 0,1);7,462(8,9);7,453(3,9);7,450(6,3);7,446(3,9);7,434(1,6);7,430(2,2);7,30 6(2,7);7,302(4,1);7,298(2,8);7,288(2,4);7,283(3,3);7,279(2,3);7,120(2,8);7, 100(2,5);3,332(56,6);3,331(57,8);3,008(12,4);2,990(12,6);2,676(0,5);2,672( 0,7);2,667(0,5);2,525(2,5);2,507(84,9);2,503(110,7);2,498(82,7);2,334(0,6); 2,329(0,8);2,325(0,6);2,288(10,6);1,103(0,6);1,096(0,7);1,091(0,5);1,084(1, 4);1,077(1,3);1,073(1,1);1,065(2,3);1,057(1,1);1,053(1,4);1,046(1,5);1,034( 0,8);1,027(0,6);0,571(1,9);0,560(5,8);0,556(6,2);0,546(3,0);0,540(6,0);0,53 6(5,7);0,526(2,2);0,302(2,3);0,292(6,7);0,288(6,7);0,280(6,1);0,276(7,0);0, 265(1,9);0,008(2,3);0,000(57,8);-0,008(2,5) |
| I-1-12 | 2,18 | 2,25 | ¹H-NMR(400,0 MHz, d₆-DMSO): δ= 9,826(1,1);9,587(15,8);9,306(6,6);9,3 00(6,8);9,006(1,9);8,958(0,9);8,952(0,9);8,677(5,0);8,668(4,8);8,666(5,0);8 ,499(0,8);8,479(3,2);8,476(3,2);8,473(2,9);8,461(2,8);8,458(3,3);8,455(3,4) ;8,452(2,9);8,344(16,0);8,065(0,4);8,043(0,5);7,861(1,1);7,696(8,2);7,675(3 ,7);7,663(3,7);7,654(3,6);7,642(3,6);7,630(3,4);7,610(5,7);7,594(0,9);7,573 (0,8);7,562(4,3);7,552(1,0);7,543(6,9);7,523(3,4);7,482(0,9);7,462(1,0);7,4 39(4,6);7,418(3,4);7,381(0,6);7,361(1,0);7,342(0,6);7,260(0,7);7,240(0,5);7 ,119(0,8);7,099(0,7);4,172(2,6);4,146(8,0);4,120(8,3);4,094(2,9);4,027(0,4) ;4,001(1,3);3,975(1,3);3,950(0,5);3,437(3,4);3,387(4,5);2,944(0,8);2,784(0, 7);2,672(0,8);2,565(0,7);2,551(1,5);2,537(1,4);2,507(98,1);2,503(126,9);2,4 99(102,5);2,330(0,9);2,287(2,9);2,076(0,9);1,958(0,8);0,000(13,4) |
| I-1-13 | 2,65 | 2,70 | ¹H-NMR(400,0 MHz, d₆-DMSO): δ= 9,798(0,4);9,619(16,0);9,299(6,6);9,2 92(6,7);8,688(4,5);8,686(4,8);8,676(4,7);8,674(4,8);8,474(2,5);8,471(2,9);8 ,468(2,8);8,465(2,6);8,454(2,8);8,450(3,0);8,448(3,1);8,444(2,6);8,378(15,3 );8,071(6,0);8,053(5,9);7,918(6,7);7,894(6,7);7,678(3,7);7,667(3,6);7,658(3 ,6);7,646(3,5);4,214(1,8);4,189(5,5);4,163(5,7);4,138(2,0);3,330(72,0);2,94 5(1,7);2,785(1,5);2,672(0,9);2,507(101,2);2,503(131,6);2,499(99,0);2,334(0 ,6);2,330(0,9);2,288(0,8);1,958(1,6);1,630(0,5);0,146(0,4);0,007(3,8);0,000 (88,1);-0,008(4,5);-0,060(0,5);-0,150(0,5) |
| I-1-14 | 2,83 | 2,91 | ¹H-NMR(400,0 MHz, d₆-DMSO): δ= 9,609(16,0);9,293(9,4);8,689(7,2);8,6 78(7,2);8,466(5,1);8,446(5,3);8,321(16,0);8,078(14,9);8,045(15,1);7,680(4, 6);7,669(4,9);7,659(4,8);7,648(4,3);7,482(1,1);7,464(1,2);7,122(1,2);7,102( |
| | | | 1, 1);4,308(0,4);4,282(1, 1);4,266(1,9);4,242(4,9);4,226(5,4);4,216(5,5);4,20 1(5,0);4,176(1,8);4,162(1,1);4,137(0,4);3,524(0,3);3,498(0,4);3,473(0,6);3, 346(468,3);3,229(0,7);2,945(1,7);2,786(1,7);2,674(1,3);2,504(198,5);2,400( 0,5);2,374(0,4);2,331(1,4);2,289(3,3);2,075(5,2);1,959(1,7);0,146(0,4);0,00 0(79,6);-0,149(0,5) |
| I-1-15 | 1,67 | 1,66 | ¹H-NMR(400,0 MHz, d₆-DMSO): δ= 9,621(12,4);9,602(10,7);9,346(0,3);9, 025(7,3);9,021(8,4);9,017(7,9);8,509(2,0);8,443(0,3);8,413(1,9);8,361(10,9) ;8,344(2,5);8,318(16,0);8,307(8,0);8,273(9,6);8,256(11,5);8,231(12,9);8,21 2(10,7);8,045(6,2);8,023(6,8);7,903(1,8);7,898(1,5);7,894(1,1);7,887(1,1);7 ,720(0,6);7,699(0,8);7,656(6,2);7,640(6,7);7,635(6,5);7,619(5,3);7,568(0,9) ;7,548(1,4);7,528(0,6);5,187(0,3);5,163(1,0);5,139(1,0);5,114(0,4);4,556(0, 4);4,529(1,3);4,518(0,6);4,502(1,5);4,492(1,7);4,475(0,7);4,465(1,6);4,437( 0,5);4,343(0,4);4,316(1,1);4,306(1,0);4,289(1,3);4,280(2,7);4,268(1,1);4,26 3(1,0);4,253(2,8);4,242(2,6);4,226(1,2);4,214(2,7);4,204(1,5);4,187(1,2);4, 176(2,3);4,165(0,7);4,149(2,0);4,138(1,5);4,122(0,8);4,112(1,4);4,085(0,5); 3,327(160,1);2,945(0,7);2,785(0,7);2,671(1,9);2,506(232,5);2,502(287,1);2, 329(1,9);1,958(0,7);1,235(0,8);0,000(32,0);-0,062(0,4) |
| I-1-16 | 1,41 | 1,46 | ¹H-NMR(400,0 MHz, d₆-DMSO): δ= 9,613(15,2);9,312(5,4);9,306(5,4);9,0 72(0,5);8,682(3,7);8,679(4,0);8,671(3,9);8,667(4,1);8,612(0,4);8,603(0,4);8 ,487(2,1);8,484(2,4);8,480(2,3);8,477(2,1);8,466(2,3);8,463(2,4);8,459(2,6) ;8,456(2,2);8,431(16,0);8,191(0,4);7,967(3,4);7,963(6,4);7,959(4,1);7,932(2 ,0);7,928(3,3);7,924(1,8);7,914(2,7);7,910(4,7);7,906(2,6);7,854(2,2);7,834 (5,5);7,815(4,6);7,808(5,7);7,804(3,3);7,793(1,2);7,789(1,7);7,784(0,9);7,6 76(2,9);7,664(2,8);7,655(2,8);7,643(3,0);7,628(0,4);7,622(0,6);7,618(0,4);7 ,603(0,4);7,491(0,4);4,307(1,0);4,298(0,8);4,280(1,3);4,271(2,6);4,253(1,0) ;4,244(2,7);4,229(2,5);4,217(1,2);4,202(2,8);4,193(1,3);4,176(1,1);4,166(1, 2);4,139(0,4);3,355(0,3);3,331(124,0);2,676(0,6);2,672(0,8);2,667(0,6);2,52 5(1,9);2,512(47,3);2,507(96,4);2,503(126,6);2,498(92,1);2,494(45,1);2,334( 0,6);2,329(0,8);2,325(0,6);0,146(1,0);0,024(0,4);0,008(7,4);0,000(205,8);-0,009(7,8);-0,150(1,0) |
| I-1-17 | 2,72 | 2,75 | ¹H-NMR(400,0 MHz, d₆-DMSO): δ= 9,568(5,9);9,291(4,2);8,680(3,2);8,67 0(3,2);8,464(2,4);8,446(2,5);8,220(5,7);7,674(2,2);7,662(2,5);7,655(2,5);7, 643(2,2);7,609(5,6);7,333(5,1);4,044(1,7);4,019(4,1);3,993(4,1);3,967(1,6); 3,334(29,9);2,670(0,6);2,503(62,1);2,398(15,5);2,330(0,8);2,288(0,4);2,061 (16,0);0,000(10,6) |
| I-1-18 | 1,94 | 2,02 | ¹H-NMR(400,0 MHz, d₆-DMSO): δ= 9,626(16,0);9,302(6,0);9,296(6,2);8,6 89(4,3);8,686(4,5);8,677(4,5);8,674(4,5);8,478(2,3);8,474(2,7);8,471(2,6);8 ,468(2,3);8,457(2,5);8,453(2,8);8,450(2,9);8,447(2,4);8,420(14,4);8,226(5,6 );8,207(5,6);8,107(4,8);8,084(4,8);7,681(3,2);7,669(3,1);7,660(3,1);7,648(3 ,1);5,758(0,5);4,350(0,6);4,342(0,6);4,244(0,8);4,217(0,9);3,331(66,1);2,67 7(0,5);2,672(0,7);2,668(0,5);2,526(1,6);2,512(39,6);2,508(79,2);2,503(103, 5);2,499(76,3);2,334(0,5);2,330(0,7);2,325(0,5);1,233(0,4);0,008(1,0);0,000 (30,7);-0,008(1,3) |
| I-1-19 | 2,09 | 2,17 | ¹H-NMR(400,0 MHz, d₆-DMSO): δ= 9,629(16,0);9,609(13,3);9,300(11,0);9 ,293(11,2);8,690(7,5);8,687(7,8);8,678(7,8);8,675(7,8);8,472(4,6);8,470(4,4 );8,466(3,9);8,455(4,2);8,451(4,7);8,449(4,8);8,446(4,0);8,350(13,0);8,318( 0,6);8,307(16,0);8,276(11,5);8,259(13,8);8,240(0,3);8,228(15,6);8,207(12,9 );7,681(5,9);7,669(5,7);7,660(5,7);7,648(5,6);5,758(7,1);4,559(0,4);4,532(1 ,3);4,522(0,6);4,504(1,6);4,494(1,8);4,477(0,7);4,467(1,8);4,440(0,6);4,321 (1,0);4,311(0,9);4,294(1,2);4,284(2,9);4,267(0,7);4,257(3,2);4,251(3,0);4,2 24(3,0);4,214(1,6);4,197(1,2);4,188(2,6);4,177(0,6);4,161(2,2);4,151(1,5);4 ,134(0,8);4,124(1,5);4,098(0,5);3,331(128,7);2,677(0,9);2,672(1,2);2,668(0, 9);2,526(3,5);2,508(135,1);2,503(174,8);2,499(128,7);2,335(0,8);2,330(1,1) ;2,326(0,8);1,234(0,9);0,008(2,1);0,000(52,5) |
| I-1-20 | 1,79 | 1,84 | ¹H-NMR(400,0 MHz, d₆-DMSO): δ= 9,595(7,7);9,579(4,9);9,303(4,9);9,29 7(5,0);8,683(3,5);8,671(3,6);8,478(1,9);8,474(2,3);8,471(2,2);8,468(1,9);8, 457(2,1);8,453(2,3);8,451(2,3);8,447(1,9);8,296(8,1);8,278(5,1);8,144(0,6); 7,862(5,8);7,849(3,7);7,677(2,6);7,665(2,5);7,656(2,6);7,644(2,5);7,470(2,8 );7,447(4,3);5,757(0,9);4,306(0,5);4,278(0,6);4,269(0,7);4,241(0,7);4,177(0 ,9);4,150(2,9);4,123(3,0);4,095(1,1);4,012(0,6);3,985(0,7);3,975(0,6);3,948 (0,6);3,328(25,6);2,676(0,5);2,672(0,6);2,507(71,2);2,503(89,7);2,499(67,4) ;2,445(10,4);2,438(14,4);2,399(0,4);2,339(0,6);2,334(0,6);2,330(0,7);2,325( 0,6);2,294(0,8);2,276(0,3);2,262(0,7);2,167(16,0);2,061(0,4);0,146(0,4);0,0 08(4,4);0,000(85,5);-0,008(4,5);-0,149(0,4) |
| I-1-21 | 1,32 | 1,27 | ¹H-NMR(400,0 MHz, d₆-DMSO): δ= 9,600(6,6);9,594(16,0);9,308(7,3);9,3 02(7,6);9,081(0,3);9,067(0,3);8,680(5,5);8,677(5,0);8,668(5,7);8,666(5,1);8 ,482(3,1);8,479(3,5);8,476(3,3);8,461(3,2);8,458(3,6);8,455(3,5);8,424(5,8) ;8,405(15,9);8,317(0,5);8,088(1,5);8,084(2,6);8,080(1,8);8,034(1,3);8,014(1 ,5);7,940(0,9);7,935(0,7);7,923(1,2);7,918(1,5);7,873(1,8);7,854(2,3);7,833 (7,2);7,829(5,1);7,823(2,1);7,819(2,6);7,803(3,8);7,800(6,1);7,796(3,5);7,7 86(3,8);7,767(5,9);7,748(2,8);7,714(3,0);7,710(4,4);7,705(2,8);7,695(1,9);7 ,691(2,7);7,687(1,7);7,674(3,8);7,662(3,8);7,653(3,8);7,641(3,8);7,577(0,3) ;3,360(3,6);3,342(4,6);3,329(133,8);3,269(0,4);3,251(0,3);2,948(0,4);2,930( 0,6);2,914(5,6);2,910(5,7);2,897(5,3);2,891(5,5);2,877(0,6);2,858(0,6);2,67 6(1,0);2,672(1,4);2,667(1,0);2,525(3,6);2,507(152,2);2,503(199,4);2,498(14 9,5);2,447(0,4);2,438(0,3);2,334(1,0);2,329(1,4);2,325(1,0); 1,514(0,5); 1,29 9(1,6);1,259(2,3);1,234(4,5);1,216(0,6);1,209(0,7);1,022(0,4);1,016(0,7);1, 004(1,3);0,997(1,2);0,985(2,0);0,973(1,3);0,966(1,4);0,954(0,9);0,947(0,6); 0,935(0,5);0,917(0,6);0,910(0,5);0,899(0,8);0,887(0,6);0,879(0,7);0,868(0,6 );0,861(0,6);0,853(0,8);0,842(0,5);0,836(0,5);0,609(0,3);0,586(1,6);0,575(5 ,7);0,566(3,2);0,555(5,7);0,546(1,9);0,533(0,5);0,523(0,5);0,507(0,7);0,496 (1,8);0,491(2,0);0,476(2,0);0,471(2,0);0,460(0,8);0,371(0,7);0,365(1,0);0,3 60(1,2);0,353(1,3);0,340(2,8);0,328(2,6);0,313(0,7);0,300(0,5);0,286(2,6);0 ,273(2,8);0,265(1,1);0,260(1,4);0,253(1,3);0,249(1,0);0,242(0,6);0,162(0,7) ;0,151(2,2);0,148(2,2);0,136(2,5);0,124(0,8);0,008(1,1);0,000(31,6);-0,008(1,4) |
| I-1-22 | 2,69 | 2,74 | ¹H-NMR(400,0 MHz, d₆-DMSO): δ= 9,532(7,3);9,278(3,0);9,272(3,1);8,67 1(2,2);8,668(2,2);8,659(2,3);8,657(2,2);8,445(1,2);8,442(1,4);8,439(1,3);8, 424(1,3);8,421(1,4);8,418(1,5);8,155(1,7);7,876(2,6);7,857(2,7);7,668(1,6); 7,656(1,6);7,647(1,7);7,635(1,6);7,494(2,4);7,468(2,3);4,100(0,5);4,086(0,5 );4,074(0,7);4,060(1,3);4,035(1,5);4,015(1,4);3,989(1,4);3,976(0,7);3,963(0 ,6);3,950(0,6);3,326(70,9);2,671(2,1);2,506(252,6);2,502(322,0);2,498(247, 4);2,457(14,9);2,416(0,7);2,328(2,2);2,242(0,4);2,205(0,3);2,178(16,0);2,15 0(0,4);2,075(5,3);0,146(1,3);0,008(12,9);0,000(279,4);-0,034(0,4);-0,150(1,3) |
| I-2-23 | 1,67 | 1,65 | ¹H-NMR(400,0 MHz, d₆-DMSO): δ= 9,571(6,2);9,293(2,2);9,287(2,2);8,68 1(1,6);8,678(1,6);8,670(1,7);8,666(1,6);8,469(0,9);8,465(1,0);8,462(1,0);8, 458(0,9);8,448(1,0);8,444(1,0);8,441(1,1);8,438(0,9);8,170(6,5);7,673(1,2); 7,661(1,2);7,652(1,2);7,640(1,1);7,585(0,5);7,582(0,6);7,565(1,4);7,562(1,3 );7,547(1,3);7,544(1,4);7,527(1,9);7,524(2,1);7,507(1,0);7,504(0,8);7,496(1 ,4);7,493(1,4);7,477(2,0);7,474(1,7);7,392(1,1);7,388(1,0);7,373(1,3);7,370 (1,3);7,355(0,7);7,351(0,6);3,332(15,7);2,944(1,1);2,785(0,9);2,511(13,3);2 ,507(25,8);2,503(33,0);2,498(23,5);2,494(11,2);2,442(16,0);2,075(0,5);1,95 8(0,9);0,008(2,0);0,000(45,7);-0,009(1,7) |

### Biologische Beispiele

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigten z.B. die folgenden Verbindungen der Herstellungsbeispiele eine Wirkung von 80 % bei einer Aufwandmenge von 100 ppm: I-1-2, I-1-3.

### Myzus persicae - Sprühtest (MYZUPE)

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 100 % bei einer Aufwandmenge von 500 g/ha: I-1-2, I-1-3, I-1-4, I-1-5, I-1-10, I-1-11.

Bei diesem Test zeigten z. B. die folgenden Verbindungen der Herstellungsbeispiele Wirkung von 90 % bei einer Aufwandmenge von 500 g/ha: I-1-6, I-1-7, I-1-8, I-1-12, I-1-14, I-1-15, I-1-16, I-1-17, I-1-18, I-1-19, I-1-21, I-1-22, I-2-23.

Bei diesem Test zeigten z. B. die folgenden Verbindungen der Herstellungsbeispiele Wirkung von 90% bei einer Aufwandmenge von 100 g/ha: I-1-9.

Bei diesem Test zeigen z. B. die folgenden Verbindungen der Herstellungsbeispiele Wirkung von 90% bei einer Aufwandmenge von 20 g/ha: I-1-20.

### Tetranychus urticae - Sprühtest, OP-resistent (TETRUR)

| | |
|---|---|
| Lösungsmittel: | 78,0 GewichtsteileAceton |
| | 1,5 Gewichtsteile Dimethylformamid |
| Emulgator : | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von 100% bei einer Aufwandmenge von 500g/ha: I-1-4.

Bei diesem Test zeigten z. B. die folgenden Verbindungen der Herstellungsbeispiele eine Wirkung von 90 % bei einer Aufwandmenge von 500 g/ha: I-1-20.

### Myzus persicae - Sprühtest (MYZUPE)

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (*Capsicum annuum*), die stark von der Grünen Pfirsichblattlaus (*Myzus persicae*) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigten z. B. die folgenden Verbindungen der Herstellungsbeispiele Wirkung von 100 % bei einer Aufwandmenge von 20 ppm: I-1-1

Bei diesem Test zeigten z. B. die folgenden Verbindungen der Herstellungsbeispiele Wirkung von 100 % bei einer Aufwandmenge von 4 ppm: I-1-13

## Patentansprüche

1. Verbindungen der Formel (I) 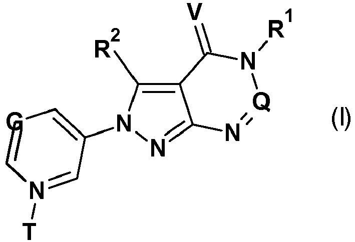 worin
G für N oder C-A¹ steht,
A¹ für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
T für ein Elektronenpaar oder für Sauerstoff steht,
R¹ für den Rest der Formel 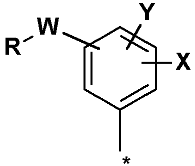 steht,
worin die Bindung zum Stickstoffatom in der C(=V)-N-Q-Gruppe in der Formel (I) mit dem Stern (*) markiert ist,
R für NR⁷R⁸ oder für einen jeweils gegebenenfalls substituierten Rest aus der Reihe Alkyl, Alkenyl, Alkinyl, Alkoxyalkyl, Alkyl-S(O)ₘ-alkyl, R⁷-CO-alkyl, NR⁷R⁸-CO-alkyl, Cycloalkyl, Cycloalkenyl, Cycloalkylalkyl, Cycloalkenylalkyl, Heterocyclyl, Heterocyclylalkyl, Phenyl, Phenylalkyl, Hetaryl und Hetarylalkyl steht,
W für einen Rest aus der Reihe O, S, SO und SO₂ steht,
X für einen Rest aus der Reihe Wasserstoff, Halogen, Cyano, Alkyl, Halogenalkyl, Alkoxy, Halogenalkoxy und Cycloalkyl steht,
Y für einen Rest aus der Reihe Wasserstoff, Halogen, Cyano, Alkyl, Halogenalkyl, Alkoxy, Halogenalkoxy, Cycloalkyl und NR⁵R⁶ steht,
R² für Wasserstoff oder Alkyl steht,
Q für Stickstoff oder C-R³ steht, worin
R³ für einen Rest aus der Reihe Wasserstoff, Halogen, Cyano, Nitro, Hydroxy, Alkyl, Halogenalkyl, Cycloalkyl, Alkoxy, Halogenalkoxy, Cycloalkylalkyl, Alkoxyalkyl, Halogenalkoxyalkyl, SH, Alkylsulfanyl, Alkylsulfinyl, Alkylsulfonyl, Halogenalkylsulfanyl, Halogenalkylsulfinyl, Halogenalkylsulfonyl, NH₂, Alkylamino und Dialkylamino steht,
V für einen Rest aus der Reihe Sauerstoff, Schwefel und NR⁴ steht und
R⁴ für einen Rest aus der Reihe Wasserstoff, Cyano, Alkyl, Halogenalkyl, Cycloalkyl, Nitro, Carbonylalkyl, Carbonylhalogenalkyl und Carbonylalkoxy steht,
R⁵ für einen Rest aus der Reihe Wasserstoff, Alkyl und Halogenalkyl steht,
R⁶ für einen Rest aus der Reihe Wasserstoff, Alkyl und Halogenalkyl steht,
oder
R⁵ und R⁶ gemeinsam mit dem Stickstoff, an dem sie gebunden sind, für einen gegebenenfalls substituierten und gegebenenfalls weitere Heteroatome enthaltenden gesättigten oder ungesättigten 3- bis 6-gliedrigen Ring stehen,
R⁷ für Wasserstoff, Hydroxy oder einen jeweils gegebenenfalls substituierten Rest aus der Reihe Alkyl, Alkoxy, Alkoxyalkyl, Alkyl-S(O)ₘ-alkyl, Alkylcarbonyl, Alkoxycarbonyl, Cycloalkyl, Cycloalkenyl, Cycloalkylalkyl, Cycloalkenylalkyl, Heterocyclyl, Heterocyclylalkyl, Phenyl, Phenylalkyl, Hetaryl und Hetarylalkyl steht,
R⁸ für Wasserstoff, ein Metallion, ein gegebenenfalls substituiertes Ammoniumion oder einen jeweils gegebenenfalls substituierten Rest aus der Reihe Alkyl, Alkoxy, Alkoxyalkyl, Alkyl-S(O)ₘ-alkyl steht und
m für eine Zahl aus der Reihe 0, 1 und 2 steht.

2. Verbindungen der Formel (I) gemäß Anspruch 1, worin
G für N oder C-A¹ steht,
A¹ für einen Rest aus der Reihe Wasserstoff, Halogen, Cyano, C₁-C₆-Alkyl, C₁-C₆-Halogenalkyl und C₃-C₆-Cycloalkyl steht,
T für ein Elektronenpaar oder für Sauerstoff steht,
R¹ für den Rest der Formel 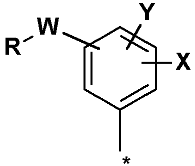 steht,
worin die Bindung zum Stickstoffatom in der C(=V)-N-Q-Gruppe in der Formel (I) mit dem Stern (*) markiert ist,
R für NR⁷R⁸ oder für jeweils gegebenfalls durch Halogen oder Cyano substituiertes C₁-C₆-Alkyl, C₃-C₆-Alkenyl, C₃-C₆-Alkinyl, C₁-C₆-Alkoxy-C₁-C₄-alkyl, C₁-C₆-Alkyl-S(O)ₘ-C₁-C₄-alkyl, für R⁷-CO-C₁-C₄-alkyl, für NR⁷R⁸-CO-C₁-C₄-alkyl, für jeweils gegebenenfalls einfach oder zweifach durch Sauerstoff (führt zu C=O), Halogen, Cyano, C₁-C₄-Alkyl, C₁-C₄-Alkoxy und C₁-C₄-Halogenalkyl substituiertes C₃-C₈-Cycloalkyl, für jeweils gegebenenfalls einfach oder zweifach durch Sauerstoff (führt zu C=O), C₁-C₄-Alkyl, C₁-C₄-Alkoxy und C₁-C₄-Halogenalkyl substituiertes C₃-C₈-Cycloalkenyl, für gegebenenfalls einfach oder zweifach durch Sauerstoff (führt zu C=O), Halogen, Cyano, C₁-C₄-Alkyl, C₁-C₄-Alkoxy und C₁-C₄-Halogenalkyl substituiertes C₃-C₆-Cycloalkyl-C₁-C₄-alkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes C₃-C₆-Cycloalkenyl-C₁-C₄-alkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes Heterocyclyl, für gegebenenfalls einfach oder zweifach durch Sauerstoff (führt zu C=O), C₁-C₄-Alkyl, C₁-C₄-Alkoxy und C₁-C₄-Halogenalkyl substituiertes Heterocyclyl-C₁-C₄-alkyl oder für jeweils gegebenenfalls einfach bis dreifach durch Halogen, Cyano, C₁-C₄-Alkyl, C₁-C₄-Halogenalkyl, C₁-C₄-Alkoxy oder C₁-C₄-Halogenalkoxy substituiertes Phenyl, Phenyl-C₁-C₄-alkyl, Hetaryl und Hetaryl-C₁-C₄-alkyl steht,
m für eine Zahl aus der Reihe 0, 1 und 2 steht,
W für einen Rest aus der Reihe O, S, SO und SO₂ steht,
X für einen Rest aus der Reihe Wasserstoff, Halogen, Cyano, C₁-C₆-Alkyl, C₁-C₆-Halogenalkyl, C₁-C₆-Alkoxy, C₁-C₆-Halogenalkoxy und C₃-C₆-Cycloalkyl steht,
Y für einen Rest aus der Reihe Wasserstoff, Halogen, Cyano, C₁-C₆-Alkyl, C₁-C₆-Halogenalkyl, C₁-C₆-Alkoxy, C₁-C₆-Halogenalkoxy, C₃-C₆-Cycloalkyl und NR⁵R⁶ steht,
R² für Wasserstoff oder C₁-C₆-Alkyl steht,
Q für Stickstoff oder C-R³ steht,
R³ für einen Rest aus der Reihe Wasserstoff, Hydroxy, C₁-C₆-Alkyl, C₁-C₆-Halogenalkyl, C₃-C₆-Cycloalkyl, C₁-C₆-Alkoxy, C₁-C₆-Halogenalkoxy, C₃-C₆-Cycloalkyl-C₁-C₄-alkyl, C₁-C₆-Alkoxy-C₁-C₄-alkyl, C₁-C₆-Halogenalkoxy-C₁-C₄-alkyl, SH, C₁-C₆-Alkylsulfanyl, C₁-C₆-Alkylsulfinyl, C₁-C₆-Alkylsulfonyl, NH₂, C₁-C₆-Alkylamino und Di-(C₁-C₆-alkyl)-amino steht,
V für einen Rest aus der Reihe Sauerstoff, Schwefel und NR⁴ steht,
R⁴ für einen Rest aus der Reihe Wasserstoff, Cyano, C₁-C₆-Alkyl, C₂-C₆-Halogenalkyl und C₃-C₆-Cycloalkyl steht,
R⁵ für einen Rest aus der Reihe Wasserstoff, C₁-C₆-Alkyl und C₂-C₆-Halogenalkyl steht,
R⁶ für einen Rest aus der Reihe Wasserstoff, C₁-C₆-Alkyl und C₂-C₆-Halogenalkyl steht,
R⁵ und R⁶ auch gemeinsam mit dem Stickstoffatom, an das sie gebunden sind, für einen gegebenenfalls durch Halogen, Cyano, C₁-C₄-Alkyl, C₁-C₄-Halogenalkyl, C₁-C₄-Alkoxy, C₁-C₄-Halogenalkoxy substituierten gesättigten bis dreifach ungesättigten 3- bis 6-gliedrigen Ring stehen können,
R⁷ für einen Rest aus der Reihe Wasserstoff, Hydroxy, jeweils gegebenenfalls einfach oder mehrfach durch Halogen oder einfach oder zweifach durch Cyano substituiertes C₁-C₆-Alkyl, C₁-C₆-Alkoxy, C₁-C₆-Alkoxy-C₁-C₄-alkyl, C₁-C₆-Alkyl-S(O)ₘ-C₁-C₄-alkyl, C₁-C₆-Alkylcarbonyl, C₁-C₆-Alkoxy-carbonyl, C₃-C₆-Cycloalkyl, C₃-C₆-Cycloalkenyl, C₃-C₆-Cycloalkyl-C₁-C₃-alkyl, C₃-C₆-Cycloalkenyl-C₁-C₃-alkyl, Heterocyclyl, Heterocyclyl-C₁-C₃-alkyl und jeweils gegebenenfalls einfach bis vierfach durch C₁-C₄-Alkyl, C₁-C₄-Halogenalkyl, C₁-C₄-Alkoxy, C₁-C₄-Halogenalkoxy, C₃-C₆-Cycloalkyl, Halogen oder Cyano substituiertes Phenyl, Phenyl-C₁-C₃-alkyl, Hetaryl und Hetaryl-C₁-C₃-alkyl steht und
R⁸ für Wasserstoff, ein Metallion oder für ein gegebenenfalls einfach bis vierfach durch C₁-C₄-Alkyl substituiertes Ammoniumion oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₄-alkyl und C₁-C₄-Alkyl-S(O)ₘ-alkyl steht.

3. Verbindungen der Formel (I) gemäß Anspruch 1, worin
G für N oder C-A¹ steht.
A¹ für einen Rest aus der Reihe Wasserstoff, Fluor, Chlor, Brom, C₁-C₄-Alkyl und C₁-C₄-Halogenalkyl steht,
T für ein Elektronenpaar oder für Sauerstoff steht,
R¹ für den Rest der Formel 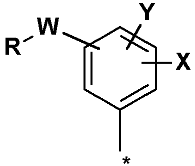 steht,
worin die Bindung zum Stickstoffatom in der C(=V)-N-Q-Gruppe in der Formel (I) mit dem Stern (*) markiert ist,
R für NR⁷R⁸ oder für jeweils gegebenfalls einfach bis siebenfach durch Halogen, einfach oder zweifach durch Sauerstoff (führt zu C=O)oder einfach oder zweifach durch Cyano substituiertes C₁-C₆-Alkyl, C₃-C₆-Alkenyl, C₃-C₆-Alkinyl, C₁-C₄-Alkoxy-C₁-C₃-alkyl, C₁-C₄-Alkyl-S(O)ₘ-C₁-C₃-alkyl, für R⁷-CO-C₁-C₂-alkyl, für NR⁷R⁸-CO-C₁-C₂-alkyl, für gegebenenfalls einfach oder zweifach durch Sauerstoff (führt zu C=O), Halogen, Cyano, C₁-C₄-Alkyl, C₁-C₄-Alkoxy und C₁-C₄-Halogenalkyl substituiertes C₃-C₈-Cycloalkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes C₃-C₈-Cycloalkenyl, für gegebenenfalls einfach oder zweifach durch Sauerstoff (führt zu C=O), Halogen, Cyano, C₁-C₄-Alkyl, C₁-C₄-Alkoxy und C₁-C₄-Halogenalkyl substituiertes C₃-C₆-Cycloalkyl-C₁-C₄-alkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes, gesättigtes oder ungesättigtes C₃-C₆-Cycloalkenyl-C₁-C₄-alkyl, für gegebenenfalls einfach oder zweifach durch Sauerstoff (führt zu C=O), C₁-C₄-Alkyl, C₁-C₄-Alkoxy und C₁-C₄-Halogenalkyl substituiertes Heterocyclyl, für gegebenenfalls einfach oder zweifach durch Sauerstoff (führt zu C=O), C₁-C₄-Alkyl, C₁-C₄-Alkoxy und C₁-C₄-Halogenalkyl substituiertes Heterocyclyl-C₁-C₄-alkyl oder für jeweils gegebenenfalls einfach bis dreifach durch Halogen, Cyano, C₁-C₄-Alkyl, C₁-C₄-Halogenalkyl, C₁-C₄-Alkoxy oder C₁-C₄-Halogenalkoxy substituiertes Phenyl, Phenyl-C₁-C₃-alkyl, Hetaryl und Hetaryl-C₁-C₃-alkyl steht,
W für einen Rest aus der Reihe S, SO und SO₂ steht,
X für einen Rest aus der Reihe Wasserstoff, Fluor, Chlor, Brom, Jod, Cyano, C₁-C₄-Alkyl, C₁-C₄-Halogenalkyl, C₁-C₄-Alkoxy und C₁-C₄-Halogenalkoxy steht,
Y für einen Rest aus der Reihe Wasserstoff, Fluor, Chlor, Brom, Jod, Cyano, C₁-C₄-Alkyl, C₁-C₄-Halogenalkyl, C₁-C₄-Alkoxy und C₁-C₄-Halogenalkoxy steht,
R² für Wasserstoff oder C₁-C₄-Alkyl steht,
Q für Stickstoff oder C-R³ steht,
R³ für einen Rest aus der Reihe Wasserstoff, Hydroxy, C₁-C₄-Alkyl, C₁-C₄-Halogenalkyl, C₃-C₆-Cycloalkyl, C₁-C₄-Alkoxy, C₁-C₄-Halogenalkoxy, C₃-C₆-Cycloalkyl-C₁-C₃-alkyl, C₁-C₄-Alkoxy-C₁-C₃-alkyl, C₁-C₄-Halogenalkoxy-C₁-C₃-alkyl, SH, C₁-C₄-Alkylsulfanyl, C₁-C₄-Alkylsulfinyl, C₁-C₄-Alkylsulfonyl, NH₂, C₁-C₄-Alkylamino und Di-(C₁-C₄-alkyl)-amino steht,
V für Sauerstoff steht,
R⁷ für Wasserstoff, Hydroxy oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₄-alkyl, C₁-C₄-Alkyl-S(O)ₘ-C₁-C₃-alkyl, C₁-C₄-Alkyl-carbonyl, C₁-C₄-Alkoxy-carbonyl, C₃-C₆-Cycloalkyl, C₃-C₆-Cycloalkyl-C₁-C₃-alkyl, Heterocyclyl, Heterocyclyl-C₁-C₃-alkyl und jeweils gegebenenfalls einfach bis dreifach durch C₁-C₄-Alkyl, C₁-C₃-Halogenalkyl, C₁-C₃-Alkoxy, C₁-C₃-Halogenalkoxy, Cyclopropyl, Fluor Chlor, Brom oder Cyano substituiertes Phenyl, Benzyl, Pyridyl, Pyrimidyl, Thiazolyl, Oxazolyl, Pyrazolyl, Thienyl, Furanyl, Pyridinyl-methyl und Thiazolylmethyl steht und
R⁸ für Wasserstoff, für ein Metallion oder für ein gegebenenfalls einfach bis vierfach durch C₁-C₄-Alkyl substituiertes Ammoniumion oder für einen jeweils gegebenenfalls einfach oder mehrfach durch Halogen oder einfach oder zweifach durch Cyano substituierten Rest aus der Reihe C₁-C₄-Alkyl, C₁-C₄-Alkoxy, C₁-C₄-Alkoxy-C₁-C₂-alkyl und C₁-C₄-Alkyl-S(O)ₘ-C₁-C₂-alkyl steht.
m für eine Zahl aus der Reihe 0, 1 und 2 steht.

4. Verbindungen der Formel (I) gemäß Anspruch 1, worin
G für N oder C-A¹ steht,
A¹ für einen Rest aus der Reihe Wasserstoff, Fluor, Chlor, Brom, Methyl, Ethyl, Isopropyl, *tert-*Butyl, Trifluormethyl, Difluormethyl, 2,2,2-Trifluorethyl und 2,2-Difluorethyl steht,
T für ein Elektronenpaar oder für Sauerstoff steht,
R¹ für den Rest der Formel 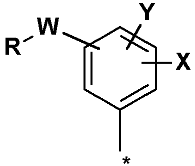 steht,
worin die Bindung zum Stickstoffatom in der C(=V)-N-Q-Gruppe in der Formel (I) mit dem Stern (*) markiert ist,
R für NR⁷R⁸ oder für einen jeweils gegebenenfalls einfach, zweifach, dreifach, vierfach oder fünffach durch Fluor, Chlor oder zweifach durch Cyano substituierten Rest aus der Reihe C₁-C₄-Alkyl, C₃-C₄-Alkenyl, C₃-C₄-Alkinyl, C₁-C₂-Alkoxy-C₁-C₂-alkyl und C₁-C₂-Alkyl-S(O)ₘ-C₁-C₂-alkyl, für R⁷-CO-C₁-C₂-alkyl, für NR⁷R⁸-CO-C₁-C₂-alkyl, für gegebenenfalls einfach oder zweifach durch Halogen, Cyano, C₁-C₂-Alkyl, C₁-C₂-Alkoxy oder C₁-C₂-Halogenalkyl oder durch ein Sauerstoffatom (führt zu C=O) substituiertes C₃-C₆-Cycloalkyl, für gegebenenfalls einfach oder zweifach durch C₁-C₂-Alkyl, C₁-C₂-Alkoxy oder C₁-C₂-Halogenalkyl oder durch ein Sauerstoffatom (führt zu C=O) substituiertes C₃-C₆-Cycloalkenyl, für gegebenenfalls einfach bis zweifach durch Halogen, Cyano, C₁-C₂-Alkyl, C₁-C₂-Alkoxy oder C₁-C₂-Halogenalkyl substituiertes C₃-C₆-Cycloalkyl-C₁-C₂-alkyl, für gegebenenfalls einfach oder zweifach durch C₁-C₂-Alkyl, C₁-C₂-Alkoxy oder C₁-C₂-Halogenalkyl substituiertes C₃-C₆-Cycloalkenyl-C₁-C₂-alkyl, für gegebenenfalls einfach oder zweifach durch C₁-C₂-Alkyl, C₁-C₂-Alkoxy oder C₁-C₂-Halogenalkyl substituiertes Heterocyclyl, für gegebenenfalls einfach oder zweifach durch C₁-C₂-Alkyl, C₁-C₂-Alkoxy oder C₁-C₂-Halogenalkyl substituiertes Heterocyclyl-C₁-C₂-alkyl oder für jeweils gegebenenfalls einfach oder zweifach durch Fluor, Chlor, Brom, Cyano, C₁-C₄-Alkyl, C₁-C₄-Halogenalkyl, C₁-C₄-Alkoxy oder C₁-C₄-Halogenalkoxy substituiertes Phenyl, Benzyl, Pyridyl, Pyrimidyl, Thiazolyl, Oxazolyl, Pyrazolyl, Thienyl, Furanyl, Pyridinylmethyl oder Thiazolylmethyl steht,
W für einen Rest aus der Reihe S, SO und SO₂ steht,
X für einen Rest aus der Reihe Wasserstoff, Fluor, Chlor, Brom, Cyano, Methyl, Ethyl, Trifluomethyl, Methoxy, Ethoxy, Difluormethoxy und Trifluormethoxy steht,
Y für einen Rest aus der Reihe Wasserstoff, Fluor, Chlor, Brom, Cyano, Methyl, Ethyl, Trifluomethyl, Methoxy, Ethoxy, Difluormethoxy und Trifluormethoxy steht,
R² für Wasserstoff oder Methyl steht,
Q für Stickstoff oder C-R³ steht,
R³ für einen Rest aus der Reihe Wasserstoff, Methyl, Trifluormethyl und Cyclopropyl steht,
V für Sauerstoff steht,
R⁷ für einen Rest aus der Reihe Wasserstoff, Hydroxy, gegebenenfalls einfach, zweifach, dreifach, vierfach oder fünffach durch Fluor, Chlor oder einfach oder zweifach durch Cyano substituiertes C₁-C₄-Alkyl, C₁-C₄-Alkoxy, C₁-C₄-Alkoxy-C₁-C₄-alkyl, C₁-C₄-Alkyl-S(O)ₘ-C₁-C₂-alkyl, C₁-C₄-Alkylcarbonyl, C₁-C₄-Alkoxycarbonyl und C₃-C₆-Cycloalkyl, C₃-C₆-Cycloalkyl-C₁-C₃-alkyl, Heterocyclyl und Heterocyclyl-C₁-C₃-alkyl oder für jeweils gegebenenfalls einfach bis dreifach durch C₁-C₄-Alkyl, C₁-C₃-Halogenalkyl, C₁-C₃-Alkoxy, C₁-C₃-Halogenalkoxy, Cyclopropyl, Fluor, Chlor, Brom oder Cyano substituiertes Phenyl, Benzyl, Pyridyl, Pyrimidyl, Thiazolyl, Oxazolyl, Pyrazolyl, Thienyl, Furanyl, Pyridinylmethyl und Thiazolylmethyl steht,
R⁸ für Wasserstoff, ein Alkali- oder Erdalkaliion, für ein gegebenenfalls einfach bis vierfach durch C₁-C₄-Alkyl substituiertes Ammoniumion oder für einen jeweils gegebenenfalls einfach oder mehrfach durch Fluor, Chlor oder einfach oder zweifach durch Cyano substituierten Rest aus der Reihe C₁-C₄-Alkyl, C₁-C₄-Alkoxy, C₁-C₄-Alkoxy-C₁-C₂-alkyl und C₁-C₄-Alkyl-S(O)ₘ-C₁-C₂-alkyl steht und
m für eine Zahl aus der Reihe 0, 1 und 2 steht.

5. Verbindungen der Formel (I) gemäß Anspruch 1, worin
G für N oder C-A¹ steht,
A¹ für einen Rest aus der Reihe Wasserstoff und Fluor steht,
T für ein Elektronenpaar oder für Sauerstoff steht,
R¹ für den Rest aus der Reihe 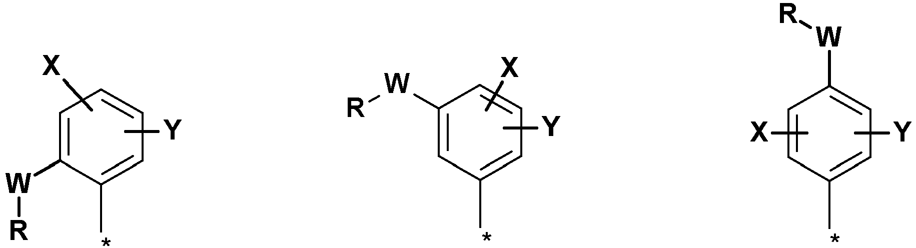 steht, worin die Bindung zum Stickstoffatom in der C(=V)-N-Q-Gruppe in der Formel (I) mit dem Stern (*) markiert ist,
R für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Fluor, Chlor, Cyano, Methyl, Ethyl, Methoxy, Ethoxy oder Trifluormethyl substituiertes C₃-C₆-Cycloalkyl, für gegebenenfalls einfach durch Fluor, Chlor, Cyano, Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl steht,
W für einen Rest aus der Reihe S, SO und SO₂ steht,
X für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy steht,
Y für einen Rest aus der Reihe Wasserstoff, Fluor, Chlor, Brom, Cyano, Methyl, Ethyl, Trifluormethyl, Methoxy, Ethoxy, Difluormethoxy und Trifluormethoxy steht,
R² steht für Wasserstoff oder Methyl,
Q für Stickstoff oder C-R³ steht,
R³ für einen Rest aus der Reihe Wasserstoff, Methyl, Trifluormethyl und Cyclopropyl steht,
V für Sauerstoff steht.

6. Verbindungen der Formel (I) gemäß Anspruch 1, worin
G für N oder C-A¹ steht,
A¹ für einen Rest aus der Reihe Wasserstoff und Fluor steht,
T für ein Elektronenpaar steht,
R¹ für den Rest der Formel 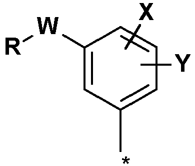 steht,
worin die Bindung zum Stickstoffatom in der C(=V)-N-Q-Gruppe in der Formel (I) mit dem Stern (*) markiert ist,
R für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Fluor, Chlor, Cyano, Methyl, Ethyl, Methoxy, Ethoxy oder Trifluormethyl substituiertes C₃-C₆-Cycloalkyl, für gegebenenfalls einfach durch Fluor, Chlor, Cyano, Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethyl, iso-Propyl, tert.-Butyl, Trifluormethyl, Difluormethyl, Methoxy, Ethoxy, Difluormethoxy oder Trifluormethoxy substituiertes Phenyl, Benzyl Pyridyl, Pyrimidyl, Thiazolyl und Pyridinylmethyl steht,
W für einen Rest aus der Reihe S, SO und SO₂ steht,
X für einen Rest aus der Reihe Wasserstoff, Fluor, Chlor, Brom, Cyano, Methyl, Ethyl und Trifluormethyl steht,
Y für einen Rest aus der Reihe Wasserstoff, Fluor, Chlor, Brom, Cyano, Methyl, Ethyl und Trifluormethyl steht,
R² für Wasserstoff oder Methyl steht,
Q für Stickstoff oder C-R³ steht,
R³ für einen Rest aus der Reihe Wasserstoff und Methyl steht,
V für Sauerstoff steht.

7. Verbindungen der Formel (I) gemäß Anspruch 1, worin
G für N oder C-A¹ steht,
A¹ für einen Rest aus der Reihe Wasserstoff oder Fluor steht,
T für ein Elektronenpaar steht,
R¹ für den Rest der Formel 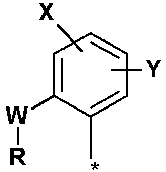 steht,
worin die Bindung zum Stickstoffatom in der C(=V)-N-Q-Gruppe in der Formel (I) mit dem Stern (*) markiert ist,
R für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Fluor, Chlor, Cyano, Methyl, Ethyl, Methoxy, Ethoxy oder Trifluormethyl substituiertes C₃-C₆-Cycloalkyl, für gegebenenfalls einfach durch Fluor, Chlor, Cyano, Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl steht,
W für einen Rest aus der Reihe S, SO und SO₂ steht,
X für einen Rest aus der Reihe Wasserstoff, Fluor, Chlor, Brom, Cyano, Methyl, Ethyl und Trifluormethyl steht,
Y für einen Rest aus der Reihe Wasserstoff, Fluor, Chlor, Brom, Cyano, Methyl, Ethyl und Trifluormethyl steht,
R² für Wasserstoff oder Methyl steht,
Q für Stickstoff oder C-R³ steht,
R³ für einen Rest aus der Reihe Wasserstoff und Methyl steht,
V für Sauerstoff steht.

8. Verbindungen der Formel (I) gemäß Anspruch 1, worin
G für N oder C-A¹ steht,
A¹ für einen Rest aus der Reihe Wasserstoff oder Fluor steht,
T für ein Elektronenpaar steht,
R¹ für den Rest der Formel 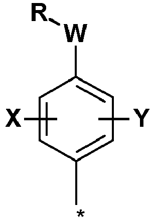 steht,
worin die Bindung zum Stickstoffatom in der C(=V)-N-Q-Gruppe in der Formel (I) mit dem Stern (*) markiert ist,
R für jeweils gegebenenfalls einfach, zweifach oder dreifach durch Fluor oder einfach durch Cyano substituiertes Methyl, Ethyl, Propyl, iso-Propyl, n-Butyl, isoButyl, sec-Butyl, tert-Butyl, Allyl, 2-Butenyl, Propargyl, 2-Butinyl, für gegebenenfalls einfach durch Fluor, Chlor, Cyano, Methyl, Ethyl, Methoxy, Ethoxy oder Trifluormethyl substituiertes C₃-C₆-Cycloalkyl, für gegebenenfalls einfach durch Fluor, Chlor, Cyano, Methyl, Ethyl, Methoxy oder Trifluormethyl substituiertes C₃-C₆-Cycloalkylmethyl oder für jeweils gegebenenfalls einfach oder zweifach durch Fluor, Chlor, Brom, Cyano, Methyl, Ethlyl, iso-Propyl, tert.-Butyl, Trifluormethyl, Difluormethyl, Methoxy, Ethoxy, Difluormethoxy oder Trifluormethoxy substituiertes Phenyl, Benzyl, Pyridyl, Pyrimidyl, Thiazolyl und Pyridinylmethyl steht,
W für einen Rest aus der Reihe S, SO und SO₂ steht.
X für einen Rest aus der Reihe Wasserstoff, Fluor, Chlor, Brom, Cyano, Methyl, Ethyl und Trifluormethyl steht,
Y für einen Rest aus der Reihe Wasserstoff, Fluor, Chlor, Brom, Cyano, Methyl, Ethyl und Trifluormethyl steht,
R² für Wasserstoff oder Methyl steht,
Q für Stickstoff oder C-R³ steht,
R³ für einen Rest aus der Reihe Wasserstoff und Methyl steht,
V für Sauerstoff steht.

9. Verbindungen der Formel (I) gemäß Anspruch 1, worin
G für C-A¹ steht,
A¹ für Wasserstoff steht,
T für ein Elektronenpaar oder Sauerstoff steht,
R¹ für den Rest der Formel 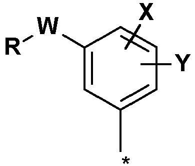 steht,
worin die Bindung zum Stickstoffatom in der C(=V)-N-Q-Gruppe in der Formel (I) mit dem Stern (*) markiert ist,
R für einen Rest aus der Reihe Methyl, Trifluorethyl und Cyclopropylmethyl steht,
W für einen Rest aus der Reihe S und SO steht,
X für einen Rest aus der Reihe Wasserstoff, Fluor, Chlor und Methyl steht,
Y für einen Rest aus der Reihe Wasserstoff, Chlor und Methyl steht,
R² für Wasserstoff steht,
Q für Stickstoff oder C-R³ steht,
R³ für Wasserstoff oder Methyl steht und
V für Sauerstoff steht.

10. Mittel, **gekennzeichnet durch** einen Gehalt von mindestens einer Verbindung der Formel (I) gemäß Ansprüchen 1 bis 9 und üblichen Streckmitteln, ausgewählt aus Wasser und polaren und unpolaren organischen chemischen Flüssigkeiten, und/oder oberflächenaktiven Substanzen.

11. Verwendung von Verbindungen der Formel (I) gemäß Ansprüchen 1 bis 9 oder von Mitteln gemäß Anspruch 10 zur Bekämpfung von Schädlingen.

12. Verbindungen der Formel (II) 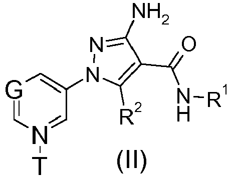 in welcher G, T, R¹ und R² die in Anspruch 1 genannten Bedeutungen haben.

13. Verbindung der Formel (VIII-1)

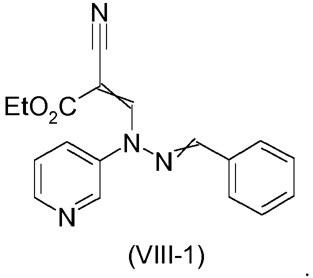

## Claims

1. Compounds of the formula (I) 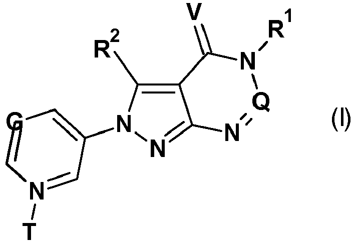 in which
G represents N or C-A¹,
A¹ represents hydrogen, halogen, cyano, nitro, alkyl, haloalkyl, alkoxy, haloalkoxy or in each case optionally substituted cycloalkyl or cycloalkenyl,
T represents an electron pair or oxygen,
R¹ represents the radical of the formula 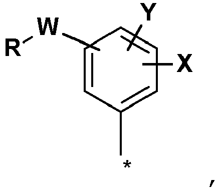 in which the bond with the nitrogen atom in the C(=V)-N-Q group in formula (I) is marked by the asterisk (*),
R represents NR⁷R⁸, or represents an in each case optionally substituted radical from the series alkyl, alkenyl, alkynyl, alkoxyalkyl, alkyl-S(O)ₘ-alkyl, R⁷-CO-alkyl, NR⁷R⁸-CO-alkyl, cycloalkyl, cycloalkenyl, cycloalkylalkyl, cycloalkenylalkyl, heterocyclyl, heterocyclylalkyl, phenyl, phenylalkyl, hetaryl and hetarylalkyl,
W represents a radical from the series O, S, SO and SO₂,
X represents a radical from the series hydrogen, halogen, cyano, alkyl, haloalkyl, alkoxy, haloalkoxy and cycloalkyl,
Y represents a radical from the series hydrogen, halogen, cyano, alkyl, haloalkyl, alkoxy, haloalkoxy, cycloalkyl and NR⁵R⁶,
R² represents hydrogen or alkyl,
Q represents nitrogen or C-R³ in which
R³ represents a radical from the series hydrogen, halogen, cyano, nitro, hydroxyl, alkyl, haloalkyl, cycloalkyl, alkoxy, haloalkoxy, cycloalkylalkyl, alkoxyalkyl, haloalkoxyalkyl, SH, alkylsulphanyl, alkylsulphinyl, alkylsulphonyl, haloalkylsulphanyl, haloalkylsulphinyl, haloalkylsulphonyl, NH₂, alkylamino and dialkylamino,
V represents a radical from the series oxygen, sulphur and NR⁴ and
R⁴ represents a radical from the series hydrogen, cyano, alkyl, haloalkyl, cycloalkyl, nitro, carbonylalkyl, carbonylhaloalkyl and carbonylalkoxy,
R⁵ represents a radical from the series hydrogen, alkyl and haloalkyl,
R⁶ represents a radical from the series hydrogen, alkyl and haloalkyl,
or R⁵ and R⁶ together with the nitrogen to which they are bonded represent an optionally substituted saturated or unsaturated 3- to 6-membered ring which optionally contains further heteroatoms,
R⁷ represents hydrogen, hydroxyl, or an in each case optionally substituted radical from the series alkyl, alkoxy, alkoxyalkyl, alkyl-S(O)ₘ-alkyl, alkylcarbonyl, alkoxycarbonyl, cycloalkyl, cycloalkenyl, cycloalkylalkyl, cycloalkenylalkyl, heterocyclyl, heterocyclylalkyl, phenyl, phenylalkyl, hetaryl and hetarylalkyl,
R⁸ represents hydrogen, a metal ion, an optionally substituted ammonium ion or an in each case optionally substituted radical from the series alkyl, alkoxy, alkoxyalkyl, alkyl-S(O)ₘ-alkyl and
m represents a number from 0, 1 and 2.

2. Compounds of the formula (I) according to Claim 1, in which
G represents N or C-A¹,
A¹ represents a radical from the series hydrogen, halogen, cyano, C₁-C₆-alkyl, C₁-C₆-haloalkyl and C₃-C₆-cycloalkyl,
T represents an electron pair or oxygen,
R¹ represents the radical of the formula 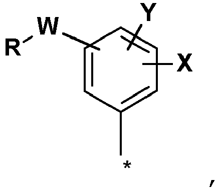 in which the bond with the nitrogen atom in the C(=V)-N-Q group in formula (I) is marked by the asterisk (*),
R represents NR⁷R⁸ or represents C₁-C₆-alkyl, C₃-C₆-alkenyl, C₃-C₆-alkynyl, C₁-C₆-alkoxy-C₁-C₄-alkyl, C₁-C₆-alkyl-S (O)ₘ-C₁-C₄-alkyl, each of which is optionally substituted by halogen or cyano, or represents R⁷-CO-C₁-C₄-alkyl, or represents NR⁷R⁸-CO-C₁-C₄-alkyl, or represents C₃-C₈-cycloalkyl, each of which is optionally monosubstituted or disubstituted by oxygen (leads to C=O), halogen, cyano, C₁-C₄-alkyl, C₁-C₄-alkoxy and C₁-C₄-haloalkyl, or represents C₃-C₈-cycloalkenyl, each of which is optionally monosubstituted or disubstituted by oxygen (leads to C=O), C₁-C₄-alkyl, C₁-C₄-alkoxy and C₁-C₄-haloalkyl, or represents C₃-C₆-cycloalkyl-C₁-C₄-alkyl which is optionally monosubstituted or disubstituted by oxygen (leads to C=O), halogen, cyano, C₁-C₄-alkyl, C₁-C₄-alkoxy and C₁-C₄-haloalkyl, or represents C₃-C₆-cycloalkenyl-C₁-C₄-alkyl which is optionally monosubstituted or disubstituted by oxygen (leads to C=O), C₁-C₄-alkyl, C₁-C₄-alkoxy and C₁-C₄-haloalkyl, or represents heterocyclyl which is optionally monosubstituted or disubstituted by oxygen (leads to C=O), C₁-C₄-alkyl, C₁-C₄-alkoxy and C₁-C₄-haloalkyl, or represents heterocyclyl-C₁-C₄-alkyl which is optionally monosubstituted or disubstituted by oxygen (leads to C=O), C₁-C₄-alkyl, C₁-C₄-alkoxy and C₁-C₄-haloalkyl, or represents phenyl, phenyl-C₁-C₄-alkyl, hetaryl and hetaryl-C₁-C₄-alkyl, each of which is optionally monosubstituted to trisubstituted by halogen, cyano, C₁-C₄-alkyl, C₁-C₄-haloalkyl, C₁-C₄-alkoxy or C₁-C₄-haloalkoxy,
m represents a number from 0, 1 and 2,
W represents a radical from the series O, S, SO and SO₂,
X represents a radical from the series hydrogen, halogen, cyano, C₁-C₆-alkyl, C₁-C₆-haloalkyl, C₁-C₆-alkoxy, C₁-C₆-haloalkoxy and C₃-C₆-cycloalkyl,
Y represents a radical from the series hydrogen, halogen, cyano, C₁-C₆-alkyl, C₁-C₆-haloalkyl, C₁-C₆-alkoxy, C₁-C₆-haloalkoxy, C₃-C₆-cycloalkyl and NR⁵R⁶,
R² represents hydrogen or C₁-C₆-alkyl,
Q represents nitrogen or C-R³,
R³ represents a radical from the series hydrogen, hydroxyl, C₁-C₆-alkyl, C₁-C₆-haloalkyl, C₃-C₆-cycloalkyl, C₁-C₆-alkoxy, C₁-C₆-haloalkoxy, C₃-C₆-cycloalkoxy-C₁-C₄-alkyl, C₁-C₆-alkoxy-C₁-C₄-alkyl, C₁-C₆-haloalkoxy-C₁-C₄-alkyl, SH, C₁-C₆-alkylsulphanyl, C₁-C₆-alkylsulphinyl, C₁-C₆-alkylsulphonyl, NH₂, C₁-C₆-alkylamino and di-(C₁-C₆-alkyl)-amino,
V represents a radical from the series oxygen, sulphur and NR⁴,
R⁴ represents a radical from the series hydrogen, cyano, C₁-C₆-alkyl, C₂-C₆-haloalkyl and C₃-C₆-cycloalkyl,
R⁵ represents a radical from the series hydrogen, C₁-C₆-alkyl and C₂-C₆-haloalkyl,
R⁶ represents a radical from the series hydrogen, C₁-C₆-alkyl and C₂-C₆-haloalkyl,
R⁵ and R⁶ can also together with the nitrogen atom to which they are bonded represent a saturated to triunsaturated 3- to 6-membered ring which is optionally substituted by halogen, cyano, C₁-C₄-alkyl, C₁-C₄-haloalkyl, C₁-C₄-alkoxy, C₁-C₄-haloalkoxy,
R⁷ represents a radical from the series hydrogen, hydroxyl, or C₁-C₆-alkyl, C₁-C₆-alkoxy, C₁-C₆-alkoxy-C₁-C₄-alkyl, C₁-C₆-alkyl-S(O)ₘ-C₁-C₄-alkyl, C₁-C₆-alkylcarbonyl, C₁-C₆-alkoxycarbonyl, C₃-C₆-cycloalkyl, C₃-C₆-cycloalkenyl, C₃-C₆-cycloalkyl-C₁-C₃-alkyl, C₃-C₆-cycloalkenyl-C₁-C₃-alkyl, heterocyclyl, heterocyclyl-C₁-C₃-alkyl, each of which is optionally monosubstituted or polysubstituted by halogen or monosubstituted or disubstituted by cyano, and represents phenyl, phenyl-C₁-C₃-alkyl, hetaryl and hetaryl-C₁-C₃-alkyl, each of which is optionally monosubstituted to tetrasubstituted by C₁-C₄-alkyl, C₁-C₄-haloalkyl, C₁-C₄-alkoxy, C₁-C₄-haloalkoxy, C₃-C₆-cycloalkyl, halogen or cyano, and
R⁸ represents hydrogen, a metal ion, or represents an ammonium ion which is optionally monosubstituted to tetrasubstituted by C₁-C₄-alkyl, or represents a radical from the series C₁-C₄-alkyl, C₁-C₄-alkoxy, C₁-C₄-alkoxy-C₁-C₄-alkyl and C₁-C₄-alkyl-S(O)ₘ-alkyl, each of which is optionally monosubstituted or polysubstituted by halogen or monsubstituted or disubstituted by cyano.

3. Compounds of the formula (I) according to Claim 1, in which
G represents N or C-A¹.
A¹ represents a radical from the series halogen, fluorine, chlorine, bromine, C₁-C₄-alkyl and C₁-C₄-haloalkyl,
T represents an electron pair or oxygen,
R¹ represents the radical of the formula 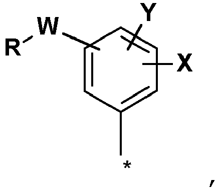 in which the bond with the nitrogen atom in the C(=V)-N-Q group in formula (I) is marked by the asterisk (*),
R represents NR⁷R⁸, or represents C₁-C₆-alkyl, C₃-C₆-alkenyl, C₃-C₆-alkynyl, C₁-C₄-alkoxy-C₁-C₃-alkyl, C₁-C₄-alkyl-S(O)ₘ-C₁-C₃-alkyl, each of which is optionally monosubstituted to heterosubstituted by halogen or monosubstituted or disubstituted by oxygen (leads to C=O) or monosubstituted or disubstituted by cyano, or represents R⁷-CO-C₁-C₂-alkyl, or represents NR⁷R⁸-CO-C₁-C₂-alkyl, or represents C₃-C₈-cycloalkyl which is optionally monosubstituted or disubstituted by oxygen (leads to C=O), halogen, cyano, C₁-C₄-alkyl, C₁-C₄-alkoxy and C₁-C₄-haloalkyl, or represents C₃-C₈-cycloalkenyl which is optionally monosubstituted or disubstituted by oxygen (leads to C=O), C₁-C₄-alkyl, C₁-C₄-alkoxy and C₁-C₄-haloalkyl, or represents C₃-C₆-cycloalkyl-C₁-C₄-alkyl which is optionally monosubstituted or disubstituted by oxygen (leads to C=O), halogen, cyano, C₁-C₄-alkyl, C₁-C₄-alkoxy and C₁-C₄-haloalkyl, or represents saturated or unsaturated C₃-C₆-cycloalkenyl-C₁-C₄-alkyl which is optionally monosubstituted or disubstituted by oxygen (leads to C=O), C₁-C₄-alkyl, C₁-C₄-alkoxy and C₁-C₄-haloalkyl, or represents heterocyclyl which is optionally monosubstituted or disubstituted by oxygen (leads to C=O), C₁-C₄-alkyl, C₁-C₄-alkoxy and C₁-C₄-haloalkyl, or represents heterocyclyl-C₁-C₄-alkyl which is optionally monosubstituted or disubstituted by oxygen (leads to C=O), C₁-C₄-alkyl, C₁-C₄-alkoxy and C₁-C₄-haloalkyl, or represents phenyl, phenyl-C₁-C₃-alkyl, hetaryl and hetaryl-C₁-C₃-alkyl, each of which is optionally monosubstituted to trisubstituted by halogen, cyano, C₁-C₄-alkyl, C₁-C₄-haloalkyl, C₁-C₄-alkoxy or C₁-C₄-haloalkoxy,
W represents a radical from the series S, SO and SO₂,
X represents a radical from the series hydrogen, fluorine, chlorine, bromine, iodine, cyano, C₁-C₄-alkyl, C₁-C₄-haloalkyl, C₁-C₄-alkoxy and C₁-C₄-haloalkoxy,
Y represents a radical from the series hydrogen, fluorine, chlorine, bromine, iodine, cyano, C₁-C₄-alkyl, C₁-C₄-haloalkyl, C₁-C₄-alkoxy and C₁-C₄-haloalkoxy,
R² represents hydrogen or C₁-C₄-alkyl,
Q represents nitrogen or C-R³,
R³ represents a radical from the series hydrogen, hydroxyl, C₁-C₄-alkyl, C₁-C₄-haloalkyl, C₃-C₆-cycloalkyl, C₁-C₄-alkoxy, C₁-C₄-haloalkoxy, C₃-C₆-cycloalkoxy-C₁-C₃-alkyl, C₁-C₄-alkoxy-C₁-C₃-alkyl, C₁-C₄-haloalkoxy-C₁-C₃-alkyl, SH, C₁-C₄-alkylsulphanyl, C₁-C₄-alkylsulphinyl, C₁-C₄-alkylsulphonyl, NH₂, C₁-C₄-alkylamino and di-(C₁-C₄-alkyl)-amino,
V represents oxygen,
R⁷ represents hydrogen, hydroxyl, or represents a radical from the series C₁-C₄-alkyl, C₁-C₄-alkoxy, C₁-C₄-alkoxy-C₁-C₄-alkyl, C₁-C₄-alkyl-S(O)ₘ-C₁-C₃-alkyl, C₁-C₄-alkylcarbonyl, C₁-C₄-alkoxycarbonyl, C₃-C₆-cycloalkyl, C₃-C₆-cycloalkyl-C₁-C₃-alkyl, heterocyclyl, heterocyclyl-C₁-C₃-alkyl, each of which is optionally monosubstituted or polysubstituted by halogen or monosubstituted or disubstituted by cyano, and represents phenyl, benzyl, pyridyl, pyrimidyl, thiazolyl, oxazolyl, pyrazolyl, thienyl, furanyl, pyridinylmethyl and thiazolylmethyl, each of which is optionally monosubstituted to trisubstituted by C₁-C₄-alkyl, C₁-C₃-haloalkyl, C₁-C₃-alkoxy, C₁-C₃-haloalkoxy, cyclopropyl, fluorine, chlorine, bromine or cyano, and
R⁸ represents hydrogen, a metal ion, or represents an ammonium ion optionally monosubstituted to tetrasubstituted by C₁-C₄-alkyl or a radical from the series C₁-C₄-alkyl, C₁-C₄-alkoxy, C₁-C₄-alkoxy-C₁-C₂-alkyl and C₁-C₄-alkyl-S(O)ₘ-C₁-C₂-alkyl, each of which is optionally monosubstituted or polysubstituted by halogen or monosubstituted or disubstituted by cyano.
m represents a number from the series 0, 1 and 2.

4. Compounds of the formula (I) according to Claim 1, in which
G represents N or C-A¹,
A¹ represents a radical from the series hydrogen, fluorine, chlorine, bromine, methyl, ethyl, isopropyl, *tert*-butyl, trifluoromethyl, difluoromethyl, 2,2,2-trifluoroethyl and 2,2-difluoroethyl,
T represents an electron pair or oxygen,
R¹ represents the radical of the formula 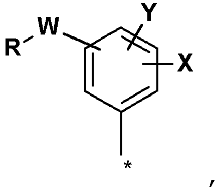 in which the bond with the nitrogen atom in the C(=V)-N-Q group in formula (I) is marked by the asterisk (*),
R represents NR⁷R⁸, or represents a radical from the series C₁-C₄-alkyl, C₃-C₄-alkenyl, C₃-C₄-alkynyl, C₁-C₂-alkoxy-C₁-C₂-alkyl and C₁-C₂-alkyl-S(O)ₘ-C₁-C₂-alkyl, each of which is optionally monosubstituted, disubstituted, trisubstituted, tetrasubstituted or pentasubstituted by fluorine, chlorine or disubstituted by cyano, or represents R⁷-CO-C₁-C₂-alkyl, or represents NR⁷R⁸-CO-C₁-C₂-alkyl, or represents C₃-C₆-cycloalkyl which is optionally monosubstituted or disubstituted by halogen, cyano, C₁-C₂-alkyl, C₁-C₂-alkoxy or C₁-C₂-haloalkyl or by an oxygen atom (leads to C=O), or represents C₃-C₆-cycloalkenyl which is optionally monosubstituted or disubstituted by C₁-C₂-alkyl, C₁-C₂-alkoxy or C₁-C₂-haloalkyl or by an oxygen atom (leads to C=O), or represents C₃-C₆-cycloalkyl-C₁-C₂-alkyl which is optionally monosubstituted to disubstituted by halogen, cyano, C₁-C₂-alkyl, C₁-C₂-alkoxy or C₁-C₂-haloalkyl, or represents C₃-C₆-cycloalkenyl-C₁-C₂-alkyl which is optionally monosubstituted or disubstituted by C₁-C₂-alkyl, C₁-C₂-alkoxy or C₁-C₂-haloalkyl, or represents heterocyclyl which is optionally monosubstituted or disubstituted by C₁-C₂-alkyl, C₁-C₂-alkoxy or C₁-C₂-haloalkyl, or represents heterocyclyl-C₁-C₂-alkyl which is optionally monosubstituted or disubstituted by C₁-C₂-alkyl, C₁-C₂-alkoxy or C₁-C₂-haloalkyl, or represents phenyl, benzyl, pyridyl, pyrimidyl, thiazolyl, oxazolyl, pyrazolyl, thienyl, furanyl, pyridinylmethyl or thiazolylmethyl, each of which is optionally monosubstituted or disubstituted by fluorine, chlorine, bromine, cyano, C₁-C₄-alkyl, C₁-C₄-haloalkyl, C₁-C₄-alkoxy or C₁-C₄-haloalkoxy,
W represents a radical from the series S, SO and SO₂,
X represents a radical from the series hydrogen, fluorine, chlorine, bromine, cyano, methyl, ethyl, trifluoromethyl, methoxy, ethoxy, difluoromethoxy and trifluoromethoxy,
Y represents a radical from the series hydrogen, fluorine, chlorine, bromine, cyano, methyl, ethyl, trifluoromethyl, methoxy, ethoxy, difluoromethoxy and trifluoromethoxy,
R² represents hydrogen or methyl,
Q represents nitrogen or C-R³,
R³ represents a radical from the series hydrogen, methyl, trifluoromethyl and cyclopropyl,
V represents oxygen,
R⁷ represents a radical from the series hydrogen, hydroxyl, or C₁-C₄-alkyl, C₁-C₄-alkoxy, C₁-C₄-alkoxy-C₁-C₄-alkyl, C₁-C₄-alkyl-S(O)ₘ-C₁-C₂-alkyl, C₁-C₄-alkylcarbonyl, C₁-C₄-alkoxycarbonyl, C₃-C₆-cycloalkyl, C₃-C₆-cycloalkyl-C₁-C₃-alkyl, heterocyclyl and heterocyclyl-C₁-C₃-alkyl which is optionally monosubstituted, disubstituted, trisubstituted, tetrasubstituted or pentasubstituted by fluorine, chlorine or monosubstituted or disubstituted by cyano, or represents phenyl, benzyl, pyridyl, pyrimidyl, thiazolyl, oxazolyl, pyrazolyl, thienyl, furanyl, pyridinylmethyl and thiazolylmethyl, each of which is optionally monosubstituted to trisubstituted by C₁-C₄-alkyl, C₁-C₃-haloalkyl, C₁-C₃-alkoxy, C₁-C₃-haloalkoxy, cyclopropyl, fluorine, chlorine, bromine or cyano,
R⁸ represents hydrogen, an alkali or alkaline-earth metal ion, an ammonium ion which is optionally monosubstituted to tetrasubstituted by C₁-C₄-alkyl, or represents a radical from the series C₁-C₄-alkyl, C₁-C₄-alkoxy, C₁-C₄-alkoxy-C₁-C₂-alkyl and C₁-C₄-alkyl-S(O)ₘ-C₁-C₂-alkyl, each of which is optionally monosubstituted or polysubstituted by fluorine, chlorine or is monosubstituted or disubstituted by cyano, and
m represents a number from series 0, 1 and 2.

5. Compounds of the formula (I) according to Claim 1, in which
G represents N or C-A¹,
A¹ represents a radical from the series hydrogen and fluorine,
T represents an electron pair or oxygen,
R¹ represents the radical from the series 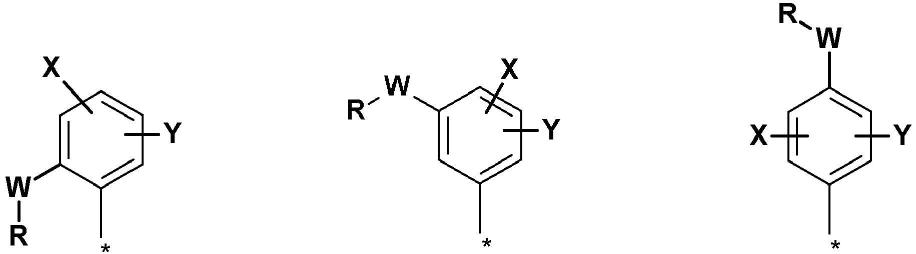 in which the bond with the nitrogen atom in the C(=V)-N-Q group in formula (I) is marked by the asterisk (*),
R represents methyl, ethyl, propyl, isopropyl, n-butyl, isobutyl, sec-butyl, tert-butyl, allyl, 2-butenyl, propargyl, 2-butynyl, each of which is optionally monosubstituted, disubstituted or trisubstituted by fluorine or monosubstituted by cyano, or represents C₃-C₆-cycloalkyl which is optionally monosubstituted by fluorine, chlorine, cyano, methyl, ethyl, methoxy, ethoxy or trifluoromethyl, or represents C₃-C₆-cycloalkylmethyl which is optionally monosubstituted by fluorine, chlorine, cyano, methyl, ethyl, methoxy or trifluoromethyl, or represents phenyl, benzyl, pyridyl, pyrimidyl, thiazolyl and pyridinylmethyl, each of which is optionally monosubstituted or disubstituted by fluorine, chlorine, bromine, cyano, methyl, ethyl, isopropyl, tert-butyl, trifluoromethyl, difluoromethyl, methoxy, ethoxy, difluoromethoxy or trifluoromethoxy,
W represents a radical from the series S, SO and SO₂,
X represents a radical from the series hydrogen, fluorine, chlorine, bromine, cyano, methyl, ethyl, trifluoromethyl, methoxy, ethoxy, difluoromethoxy and trifluoromethoxy,
Y represents a radical from the series hydrogen, fluorine, chlorine, bromine, cyano, methyl, ethyl, trifluoromethyl, methoxy, ethoxy, difluoromethoxy and trifluoromethoxy,
R² represents hydrogen or methyl,
Q represents nitrogen or C-R³,
R³ represents a radical from the series hydrogen, methyl, trifluoromethyl and cyclopropyl,
V represents oxygen.

6. Compounds of the formula (I) according to Claim 1, in which
G represents N or C-A¹,
A¹ represents a radical from the series hydrogen and fluorine,
T represents an electron pair,
R¹ represents the radical of the formula 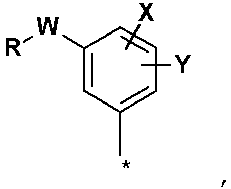 in which the bond with the nitrogen atom in the C(=V)-N-Q group in formula (I) is marked by the asterisk (*),
R represents methyl, ethyl, propyl, isopropyl, n-butyl, isobutyl, sec-butyl, tert-butyl, allyl, 2-butenyl, propargyl, 2-butynyl, each of which is optionally monosubstituted, disubstituted or trisubstituted by fluorine or monosubstituted by cyano, or represents C₃-C₆-cycloalkyl which is optionally monosubstituted by fluorine, chlorine, cyano, methyl, ethyl, methoxy, ethoxy or trifluoromethyl, or represents C₃-C₆-cycloalkylmethyl which is optionally monosubstituted by fluorine, chlorine, cyano, methyl, ethyl, methoxy or trifluoromethyl, or represents phenyl, benzyl, pyridyl, pyrimidyl, thiazolyl and pyridinylmethyl, each of which is optionally monosubstituted or disubstituted by fluorine, chlorine, bromine, cyano, methyl, ethyl, isopropyl, tert-butyl, trifluoromethyl, difluoromethyl, methoxy, ethoxy, difluoromethoxy or trifluoromethoxy,
W represents a radical from the series S, SO and SO₂,
X represents a radical from the series hydrogen, fluorine, chlorine, bromine, cyano, methyl, ethyl and trifluoromethyl,
Y represents a radical from the series hydrogen, fluorine, chlorine, bromine, cyano, methyl, ethyl and trifluoromethyl,
R² represents hydrogen or methyl,
Q represents nitrogen or C-R³,
R³ represents a radical from the series hydrogen and methyl,
V represents oxygen.

7. Compounds of the formula (I) according to Claim 1, in which
G represents N or C-A¹,
A¹ represents a radical from the series hydrogen and fluorine,
T represents an electron pair,
R¹ represents the radical of the formula 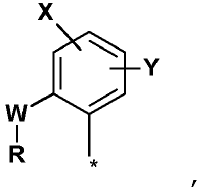 in which the bond with the nitrogen atom in the C(=V)-N-Q group in formula (I) is marked by the asterisk (*),
R represents methyl, ethyl, propyl, isopropyl, n-butyl, isobutyl, sec-butyl, tert-butyl, allyl, 2-butenyl, propargyl, 2-butynyl, each of which is optionally monosubstituted, disubstituted or trisubstituted by fluorine or monosubstituted by cyano, or represents C₃-C₆-cycloalkyl which is optionally monosubstituted by fluorine, chlorine, cyano, methyl, ethyl, methoxy, ethoxy or trifluoromethyl, or represents C₃-C₆-cycloalkylmethyl which is optionally monosubstituted by fluorine, chlorine, cyano, methyl, ethyl, methoxy or trifluoromethyl, or represents phenyl, benzyl, pyridyl, pyrimidyl, thiazolyl and pyridinylmethyl, each of which is optionally monosubstituted or disubstituted by fluorine, chlorine, bromine, cyano, methyl, ethyl, isopropyl, tert-butyl, trifluoromethyl, difluoromethyl, methoxy, ethoxy, difluoromethoxy or trifluoromethoxy,
W represents a radical from the series S, SO and SO₂,
X represents a radical from the series hydrogen, fluorine, chlorine, bromine, cyano, methyl, ethyl and trifluoromethyl,
Y represents a radical from the series hydrogen, fluorine, chlorine, bromine, cyano, methyl, ethyl and trifluoromethyl,
R² represents hydrogen or methyl,
Q represents nitrogen or C-R³,
R³ represents a radical from the series hydrogen and methyl,
V represents oxygen.

8. Compounds of the formula (I) according to Claim 1, in which
G represents N or C-A¹,
A¹ represents a radical from the series hydrogen or fluorine,
T represents an electron pair,
R¹ represents the radical of the formula 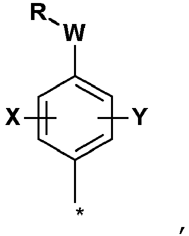 in which the bond with the nitrogen atom in the C(=V)-N-Q group in formula (I) is marked by the asterisk (*),
R represents methyl, ethyl, propyl, isopropyl, n-butyl, isobutyl, sec-butyl, tert-butyl, allyl, 2-butenyl, propargyl, 2-butynyl, each of which is optionally monosubstituted, disubstituted or trisubstituted by fluorine or monosubstituted by cyano, or represents C₃-C₆-cycloalkyl which is optionally monosubstituted by fluorine, chlorine, cyano, methyl, ethyl, methoxy, ethoxy or trifluoromethyl, or represents C₃-C₆-cycloalkylmethyl which is optionally monosubstituted by fluorine, chlorine, cyano, methyl, ethyl, methoxy or trifluoromethyl, or represents phenyl, benzyl, pyridyl, pyrimidyl, thiazolyl and pyridinylmethyl, each of which is optionally monosubstituted or disubstituted by fluorine, chlorine, bromine, cyano, methyl, ethyl, isopropyl, tert-butyl, trifluoromethyl, difluoromethyl, methoxy, ethoxy, difluoromethoxy or trifluoromethoxy,
W represents a radical from the series S, SO and SO₂.
X represents a radical from the series hydrogen, fluorine, chlorine, bromine, cyano, methyl, ethyl and trifluoromethyl,
Y represents a radical from the series hydrogen, fluorine, chlorine, bromine, cyano, methyl, ethyl and trifluoromethyl,
R² represents hydrogen or methyl,
Q represents nitrogen or C-R³,
R³ represents a radical from the series hydrogen and methyl,
V represents oxygen.

9. Compounds of the formula (I) according to Claim 1, in which
G represents C-A¹,
A¹ represents hydrogen,
T represents an electron pair or oxygen,
R¹ represents the radical of the formula 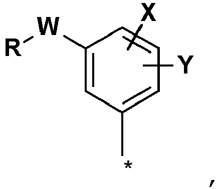 in which the bond with the nitrogen atom in the C(=V)-N-Q group in formula (I) is marked by the asterisk (*),
R represents a radical from the series methyl, trifluoroethyl and cyclopropylmethyl,
W represents a radical from the series S and SO,
X represents a radical from the series hydrogen, fluorine, chlorine and methyl,
Y represents a radical from the series hydrogen, chlorine and methyl,
R² represents hydrogen,
Q represents nitrogen or C-R³,
R³ represents hydrogen or methyl and
V represents oxygen.

10. Composition, **characterized by** a content of at least one compound of the formula (I) according to Claims 1 to 9 and customary extenders, selected from water and polar and nonpolar organic chemical liquids, and/or surface-active substances.

11. Use of compounds of the formula (I) according to Claims 1 to 9 or of compositions according to Claim 10 for controlling pests.

12. Compounds of the formula (II) 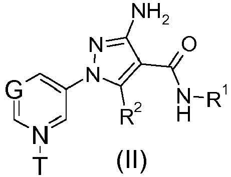 in which G, T, R¹ and R² have the meanings mentioned in Claim 1.

13. Compound of the formula (VIII-1)

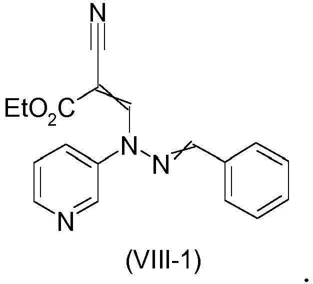

## Revendications

1. Composés de formule (I) 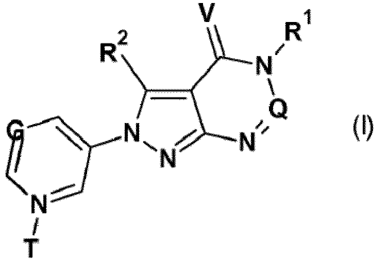 dans lesquels
G représente N ou C-A¹,
A¹ représente hydrogène, halogène, cyano, nitro, alkyle, halogénoalkyle, alcoxy, halogénoalcoxy ; ou cycloalkyle ou cycloalcényle, chacun éventuellement substitués,
T représente une paire d'électrons ou l'oxygène,
R¹ représente le radical de formule 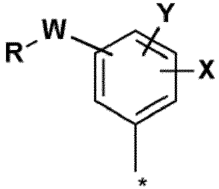
dans laquelle la liaison à l'atome d'azote dans le groupe C(=V)-N-Q dans la formule (I) est marquée avec l'étoile (*),
R représente NR⁷R⁸ ou un radical à chaque fois éventuellement substitué de la série constituée par alkyle, alcényle, alcynyle, alcoxyalkyle, alkyle-S(O)ₘ-alkyle, R⁷-CO-alkyle, NR⁷R⁸-CO-alkyle, cycloalkyle, cycloalcényle, cycloalkylalkyle, cycloalcénylalkyle, hétérocyclyle, hétérocyclylalkyle, phényle, phénylalkyle, hétaryle et hétarylalkyle,
W représente un radical de la série constituée par O, S, SO et SO₂,
X représente un radical de la série constituée par hydrogène, halogène, cyano, alkyle, halogénoalkyle, alcoxy, halogénoalcoxy et cycloalkyle,
Y représente un radical de la série constituée par hydrogène, halogène, cyano, alkyle, halogénoalkyle, alcoxy, halogénoalcoxy, cycloalkyle et NR⁵R⁶,
R² représente hydrogène ou alkyle,
Q représente azote ou C-R³,
R³ représentant un radical de la série constituée par hydrogène, halogène, cyano, nitro, hydroxy, alkyle, halogénoalkyle, cycloalkyle, alcoxy, halogénoalcoxy, cycloalkylalkyle, alcoxyalkyle, halogénoalcoxyalkyle, SH, alkylsulfanyle, alkylsulfinyle, alkylsulfonyle, halogénoalkylsulfanyle, halogénoalkylsulfinyle, halogénoalkylsulfonyle, NH₂, alkylamino et dialkylamino, V représente un radical de la série constituée par oxygène, soufre et NR⁴, et
R⁴ représente un radical de la série constituée par hydrogène, cyano, alkyle, halogénoalkyle, cycloalkyle, nitro, carbonylalkyle, carbonylhalogénoalkyle et carbonylalcoxy,
R⁵ représente un radical de la série constituée par hydrogène, alkyle et halogénoalkyle,
R⁶ représente un radical de la série constituée par hydrogène, alkyle et halogénoalkyle,
ou
R⁵ et R⁶ représentent ensemble avec l'azote auquel ils sont reliés un cycle saturé ou insaturé de 3 à 6 chaînons éventuellement substitué et contenant éventuellement d'autres hétéroatomes,
R⁷ représente hydrogène, hydroxy ou un radical à chaque fois éventuellement substitué de la série constituée par alkyle, alcoxy, alcoxyalkyle, alkyl-S(O)ₘ-alkyle, alkylcarbonyle, alcoxycarbonyle, cycloalkyle, cycloalcényle, cycloalkylalkyle, cycloalcénylalkyle, hétérocyclyle, hétérocyclylalkyle, phényle, phénylalkyle, hétaryle et hétarylalkyle,
R⁸ représente hydrogène, un ion métallique, un ion ammonium éventuellement substitué ou un radical à chaque fois éventuellement substitué de la série constituée par alkyle, alcoxy, alcoxyalkyle, alkyl-S(O)ₘ-alkyle, et
m représente un nombre de la série constituée par 0, 1 et 2.

2. Composés de formule (I) selon la revendication 1, dans lesquels
G représente N ou C-A¹,
A¹ représente un radical de la série constituée par hydrogène, halogène, cyano, alkyle en C₁-C₆, halogénoalkyle en C₁-C₆ et cycloalkyle en C₃-C₆,
T représente une paire d'électrons ou l'oxygène,
R¹ représente le radical de formule 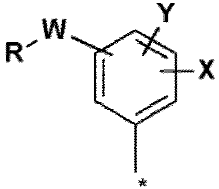
dans laquelle la liaison à l'atome d'azote dans le groupe C(=V)-N-Q dans la formule (I) est marquée avec l'étoile (*),
R représente NR⁷R⁸ ou alkyle en C₁-C₆, alcényle en C₃-C₆, alcynyle en C₃-C₆, alcoxy en C₁-C₆-alkyle en C₁-C₄, alkyle en C₁-C₆-S(O)ₘ-alkyle en C₁-C₄, chacun éventuellement substitués par halogène ou cyano ; R⁷-CO-alkyle en C₁-C₄; NR⁷R⁸-CO-alkyle en C₁-C₄ ; cycloalkyle en C₃-C₈ à chaque fois éventuellement substitué une fois ou deux fois par oxygène (conduit à C=O), halogène, cyano, alkyle en C₁-C₄, alcoxy en C₁-C₄ et halogénoalkyle en C₁-C₄ ; cycloalcényle en C₃-C₈ à chaque fois éventuellement substitué une fois ou deux fois par oxygène (conduit à C=O), alkyle en C₁-C₄, alcoxy en C₁-C₄ et halogénoalkyle en C₁-C₄ ; cycloalkyle en C₃-C₆-alkyle en C₁-C₄ éventuellement substitué une fois ou deux fois par oxygène (conduit à C=O), halogène, cyano, alkyle en C₁-C₄, alcoxy en C₁-C₄ et halogénoalkyle en C₁-C₄ ; cycloalcényle en C₃-C₆-alkyle en C₁-C₄ éventuellement substitué une fois ou deux fois par oxygène (conduit à C=O), alkyle en C₁-C₄, alcoxy en C₁-C₄ et halogénoalkyle en C₁-C₄ ; hétérocyclyle éventuellement substitué une fois ou deux fois par oxygène (conduit à C=O), alkyle en C₁-C₄, alcoxy en C₁-C₄ et halogénoalkyle en C₁-C₄ ; hétérocyclyl-alkyle en C₁-C₄ éventuellement substitué une fois ou deux fois par oxygène (conduit à C=O), alkyle en C₁-C₄, alcoxy en C₁-C₄ et halogénoalkyle en C₁-C₄ ; ou phényle, phényl-alkyle en C₁-C₄, hétaryle et hétaryl-alkyle en C₁-C₄, chacun éventuellement substitués une fois à trois fois par halogène, cyano, alkyle en C₁-C₄, halogénoalkyle en C₁-C₄, alcoxy en C₁-C₄ ou halogénoalcoxy en C₁-C₄ ;
m représente un nombre de la série constituée par 0, 1 ou 2,
W représente un radical de la série constituée par O, S, SO et SO₂,
X représente un radical de la série constituée par hydrogène, halogène, cyano, alkyle en C₁-C₆, halogénoalkyle en C₁-C₆, alcoxy en C₁-C₆, halogénoalcoxy en C₁-C₆ et cycloalkyle en C₃-C₆,
Y représente un radical de la série constituée par hydrogène, halogène, cyano, alkyle en C₁-C₆, halogénoalkyle en C₁-C₆, alcoxy en C₁-C₆, halogénoalcoxy en C₁-C₆, cycloalkyle en C₃-C₆ et NR⁵R⁶,
R² représente hydrogène ou alkyle en C₁-C₆,
Q représente azote ou C-R³,
R³ représente un radical de la série constituée par hydrogène, hydroxy, alkyle en C₁-C₆, halogénoalkyle en C₁-C₆, cycloalkyle en C₃-C₆, alcoxy en C₁-C₆, halogénoalcoxy en C₁-C₆, cycloalkyle en C₃-C₆-alkyle en C₁-C₄, alcoxy en C₁-C₆-alkyle en C₁-C₄, halogénoalcoxy en C₁-C₆-alkyle en C₁-C₄, SH, alkylsulfanyle en C₁-C₆, alkylsulfinyle en C₁-C₆, alkylsulfonyle en C₁-C₆, NH₂, alkylamino en C₁-C₆ et di-(alkyle en C₁-C₆)-amino,
V représente un radical de la série constituée par oxygène, soufre et NR⁴,
R⁴ représente un radical de la série constituée par hydrogène, cyano, alkyle en C₁-C₆, halogénoalkyle en C₂-C₆ et cycloalkyle en C₃-C₆,
R⁵ représente un radical de la série constituée par hydrogène, alkyle en C₁-C₆ et halogénoalkyle en C₂-C₆,
R⁶ représente un radical de la série constituée par hydrogène, alkyle en C₁-C₆ et halogénoalkyle en C₂-C₆,
R⁵ et R⁶ peuvent également représenter ensemble avec l'azote auquel ils sont reliés un cycle saturé à trois fois insaturé de 3 à 6 chaînons éventuellement substitué par halogène, cyano, alkyle en C₁-C₄, halogénoalkyle en C₁-C₄, alcoxy en C₁-C₄, halogénoalcoxy en C₁-C₄,
R⁷ représente un radical de la série constituée par hydrogène, hydroxy ; alkyle en C₁-C₆, alcoxy en C₁-C₆, alcoxy en C₁-C₆-alkyle en C₁-C₄, alkyle en C₁-C₆-S(O)ₘ-alkyle en C₁-C₄, alkylcarbonyle en C₁-C₆, alcoxycarbonyle en C₁-C₆, cycloalkyle en C₃-C₆, cycloalcényle en C₃-C₆, cycloalkyle en C₃-C₆-alkyle en C₁-C₃, cycloalcényle en C₃-C₆-alkyle en C₁-C₃, hétérocyclyle, hétérocyclyl-alkyle en C₁-C₃, chacun éventuellement substitués une fois ou plusieurs fois par halogène ou une fois ou deux fois par cyano ; et phényle, phényl-alkyle en C₁-C₃, hétaryle et hétaryl-alkyle en C₁-C₃, chacun éventuellement substitués une fois à quatre fois par alkyle en C₁-C₄, halogénoalkyle en C₁-C₄, alcoxy en C₁-C₄, halogénoalcoxy en C₁-C₄, cycloalkyle en C₃-C₆, halogène ou cyano, et
R⁸ représente hydrogène, un ion métallique ou un ion ammonium éventuellement substitué une fois à quatre fois par alkyle en C₁-C₄ ou un radical à chaque fois éventuellement substitué une fois ou plusieurs fois par halogène ou une fois ou deux fois par cyano, de la série constituée par alkyle en C₁-C₄, alcoxy en C₁-C₄, alcoxy en C₁-C₄-alkyle en C₁-C₄ et alkyle en C₁-C₄-S(O)ₘ-alkyle.

3. Composés de formule (I) selon la revendication 1, dans lesquels
G représente N ou C-A¹,
A¹ représente un radical de la série constituée par hydrogène, fluor, chlore, brome, alkyle en C₁-C₄ et halogénoalkyle en C₁-C₄,
T représente une paire d'électrons ou l'oxygène,
R¹ représente le radical de formule 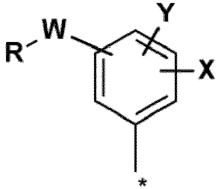
dans laquelle la liaison à l'atome d'azote dans le groupe C(=V)-N-Q dans la formule (I) est marquée avec l'étoile (*),
R représente NR⁷R⁸ ou alkyle en C₁-C₆, alcényle en C₃-C₆, alcynyle en C₃-C₆, alcoxy en C₁-C₄-alkyle en C₁-C₃, alkyle en C₁-C₄-S(O)ₘ-alkyle en C₁-C₃, chacun éventuellement substitués une fois à sept fois par halogène, une fois ou deux fois par oxygène (conduit à C=O) ou une fois ou deux fois par cyano ; R⁷-CO-alkyle en C₁-C₂ ; NR⁷R⁸-CO-alkyle en C₁-C₂ ; cycloalkyle en C₃-C₈ éventuellement substitué une fois ou deux fois par oxygène (conduit à C=O), halogène, cyano, alkyle en C₁-C₄, alcoxy en C₁-C₄ et halogénoalkyle en C₁-C₄ ; cycloalcényle en C₃-C₈ éventuellement substitué une fois ou deux fois par oxygène (conduit à C=O), alkyle en C₁-C₄, alcoxy en C₁-C₄ et halogénoalkyle en C₁-C₄ ; cycloalkyle en C₃-C₆-alkyle en C₁-C₄ éventuellement substitué une fois ou deux fois par oxygène (conduit à C=O), halogène, cyano, alkyle en C₁-C₄, alcoxy en C₁-C₄ et halogénoalkyle en C₁-C₄ ; cycloalcényle en C₃-C₆-alkyle en C₁-C₄ saturé ou insaturé, éventuellement substitué une fois ou deux fois par oxygène (conduit à C=O), alkyle en C₁-C₄, alcoxy en C₁-C₄ et halogénoalkyle en C₁-C₄; hétérocyclyle éventuellement substitué une fois ou deux fois par oxygène (conduit à C=O), alkyle en C₁-C₄, alcoxy en C₁-C₄ et halogénoalkyle en C₁-C₄ ; hétérocyclyl-alkyle en C₁-C₄ éventuellement substitué une fois ou deux fois par oxygène (conduit à C=O), alkyle en C₁-C₄, alcoxy en C₁-C₄ et halogénoalkyle en C₁-C₄ ; ou phényle, phényl-alkyle en C₁-C₃, hétaryle et hétaryl-alkyle en C₁-C₃, chacun éventuellement substitués une fois à trois fois par halogène, cyano, alkyle en C₁-C₄, halogénoalkyle en C₁-C₄, alcoxy en C₁-C₄ ou halogénoalcoxy en C₁-C₄ ;
W représente un radical de la série constituée par S, SO et SO₂,
X représente un radical de la série constituée par hydrogène, fluor, chlore, brome, iode, cyano, alkyle en C₁-C₄, halogénoalkyle en C₁-C₄, alcoxy en C₁-C₄ et halogénoalcoxy en C₁-C₄,
Y représente un radical de la série constituée par hydrogène, fluor, chlore, brome, iode, cyano, alkyle en C₁-C₄, halogénoalkyle en C₁-C₄, alcoxy en C₁-C₄ et halogénoalcoxy en C₁-C₄,
R² représente hydrogène ou alkyle en C₁-C₄,
Q représente azote ou C-R³,
R³ représente un radical de la série constituée par hydrogène, hydroxy, alkyle en C₁-C₄, halogénoalkyle en C₁-C₄, cycloalkyle en C₃-C₆, alcoxy en C₁-C₄, halogénoalcoxy en C₁-C₄, cycloalkyle en C₃-C₆-alkyle en C₁-C₃, alcoxy en C₁-C₄-alkyle en C₁-C₃, halogénoalcoxy en C₁-C₄-alkyle en C₁-C₃, SH, alkylsulfanyle en C₁-C₄, alkylsulfinyle en C₁-C₄, alkylsulfonyle en C₁-C₄, NH₂, alkylamino en C₁-C₄ et di-(alkyle en C₁-C₄)-amino,
V représente oxygène,
R⁷ représente hydrogène, hydroxy ou un radical à chaque fois éventuellement substitué une fois ou plusieurs fois par halogène ou une fois ou deux fois par cyano, de la série constituée par alkyle en C₁-C₄, alcoxy en C₁-C₄, alcoxy en C₁-C₄-alkyle en C₁-C₄, alkyle en C₁-C₄-S (O) ₘ-alkyle en C₁-C₃, alkylcarbonyle en C₁-C₄, alcoxycarbonyle en C₁-C₄, cycloalkyle en C₃-C₆, cycloalkyle en C₃-C₆-alkyle en C₁-C₃, hétérocyclyle, hétérocyclyl-alkyle en C₁-C₃; et phényle, benzyle, pyridyle, pyrimidyle, thiazolyle, oxazolyle, pyrazolyle, thiényle, furanyle, pyridinyl-méthyle et thiazolylméthyle, chacun éventuellement substitués une fois à trois fois par alkyle en C₁-C₄, halogénoalkyle en C₁-C₃, alcoxy en C₁-C₃, halogénoalcoxy en C₁-C₃, cyclopropyle, fluor, chlore, brome ou cyano, et
R⁸ représente hydrogène, un ion métallique ou un ion ammonium éventuellement substitué une fois à quatre fois par alkyle en C₁-C₄ ou un radical à chaque fois éventuellement substitué une fois ou plusieurs fois par halogène ou une fois ou deux fois par cyano, de la série constituée par alkyle en C₁-C₄, alcoxy en C₁-C₄, alcoxy en C₁-C₄-alkyle en C₁-C₂ et alkyle en C₁-C₄-S(O)ₘ-alkyle en C₁-C₂,
m représente un nombre de la série constituée par 0, 1 et 2.

4. Composés de formule (I) selon la revendication 1, dans lesquels
G représente N ou C-A¹,
A¹ représente un radical de la série constituée par hydrogène, fluor, chlore, brome, méthyle, éthyle, isopropyle, *tert*-butyle, trifluorométhyle, difluorométhyle, 2,2,2-trifluoroéthyle et 2,2-difluoroéthyle,
T représente une paire d'électrons ou l'oxygène,
R¹ représente le radical de formule 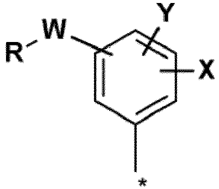
dans laquelle la liaison à l'atome d'azote dans le groupe C(=V)-N-Q dans la formule (I) est marquée avec l'étoile (*),
R représente NR⁷R⁸ ou un radical à chaque fois éventuellement substitué une fois, deux fois, trois fois, quatre fois ou cinq fois par fluor, chlore ou deux fois par cyano, de la série constituée par alkyle en C₁-C₄, alcényle en C₃-C₄, alcynyle en C₃-C₄, alcoxy en C₁-C₂-alkyle en C₁-C₂ et alkyle en C₁-C₂-S(O)ₘ-alkyle en C₁-C₂ ; R⁷-CO-alkyle en C₁-C₂ ; NR⁷R⁸-CO-alkyle en C₁-C₂ ; cycloalkyle en C₃-C₆ éventuellement substitué une fois ou deux fois par halogène, cyano, alkyle en C₁-C₂, alcoxy en C₁-C₂ ou halogénoalkyle en C₁-C₂ ou par un atome d'oxygène (conduit à C=O) ; cycloalcényle en C₃-C₆ éventuellement substitué une fois ou deux fois par alkyle en C₁-C₂, alcoxy en C₁-C₂ ou halogénoalkyle en C₁-C₂ ou par un atome d'oxygène (conduit à C=O) ; cycloalkyle en C₃-C₆-alkyle en C₁-C₂ éventuellement substitué une fois à deux fois par halogène, cyano, alkyle en C₁-C₂, alcoxy en C₁-C₂ ou halogénoalkyle en C₁-C₂ ; cycloalcényle en C₃-C₆-alkyle en C₁-C₂ éventuellement substitué une fois ou deux fois par alkyle en C₁-C₂, alcoxy en C₁-C₂ ou halogénoalkyle en C₁-C₂ ; hétérocyclyle éventuellement substitué une fois ou deux fois par alkyle en C₁-C₂, alcoxy en C₁-C₂ ou halogénoalkyle en C₁-C₂ ; hétérocyclyl-alkyle en C₁-C₂ éventuellement substitué une fois ou deux fois par alkyle en C₁-C₂, alcoxy en C₁-C₂ ou halogénoalkyle en C₁-C₂ ; ou phényle, benzyle, pyridyle, pyrimidyle, thiazolyle, oxazolyle, pyrazolyle, thiényle, furanyle, pyridinylméthyle ou thiazolylméthyle, chacun éventuellement substitués une fois ou deux fois par fluor, chlore, brome, cyano, alkyle en C₁-C₄, halogénoalkyle en C₁-C₄, alcoxy en C₁-C₄ ou halogénoalcoxy en C₁-C₄ ;
W représente un radical de la série constituée par S, SO et SO₂,
X représente un radical de la série constituée par hydrogène, fluor, chlore, brome, cyano, méthyle, éthyle, trifluorométhyle, méthoxy, éthoxy, difluorométhoxy et trifluorométhoxy,
Y représente un radical de la série constituée par hydrogène, fluor, chlore, brome, cyano, méthyle, éthyle, trifluorométhyle, méthoxy, éthoxy, difluorométhoxy et trifluorométhoxy,
R² représente hydrogène ou méthyle,
Q représente azote ou C-R³,
R³ représente un radical de la série constituée par hydrogène, méthyle, trifluorométhyle et cyclopropyle,
V représente oxygène,
R⁷ représente un radical de la série constituée par hydrogène, hydroxy, alkyle en C₁-C₄, alcoxy en C₁-C₄, alcoxy en C₁-C₄-alkyle en C₁-C₄, alkyle en C₁-C₄-S(O)ₘ-alkyle en C₁-C₂, alkylcarbonyle en C₁-C₄, alcoxycarbonyle en C₁-C₄ et cycloalkyle en C₃-C₆, cycloalkyle en C₃-C₆-alkyle en C₁-C₃, hétérocyclyle et hétérocyclyl-alkyle en C₁-C₃, éventuellement substitués une fois, deux fois, trois fois, quatre fois ou cinq fois par fluor, chlore ou une fois ou deux fois par cyano ; ou phényle, benzyle, pyridyle, pyrimidyle, thiazolyle, oxazolyle, pyrazolyle, thiényle, furanyle, pyridinyl-méthyle et thiazolylméthyle, chacun éventuellement substitués une fois à trois fois par alkyle en C₁-C₄, halogénoalkyle en C₁-C₃, alcoxy en C₁-C₃, halogénoalcoxy en C₁-C₃, cyclopropyle, fluor, chlore, brome ou cyano,
R⁸ représente hydrogène, un ion alcalin ou alcalino-terreux, un ion ammonium éventuellement substitué une fois à quatre fois par alkyle en C₁-C₄ ou un radical à chaque fois éventuellement substitué une fois ou plusieurs fois par fluor, chlore ou une fois ou deux fois par cyano, de la série constituée par alkyle en C₁-C₄, alcoxy en C₁-C₄, alcoxy en C₁-C₄-alkyle en C₁-C₂ et alkyle en C₁-C₄-S(O)ₘ-alkyle en C₁-C₂, et
m représente un nombre de la série constituée par 0, 1 et 2.

5. Composés de formule (I) selon la revendication 1, dans lesquels
G représente N ou C-A¹,
A¹ représente un radical de la série constituée par hydrogène et fluor,
T représente une paire d'électrons ou l'oxygène,
R¹ représente un radical de la série constituée par : 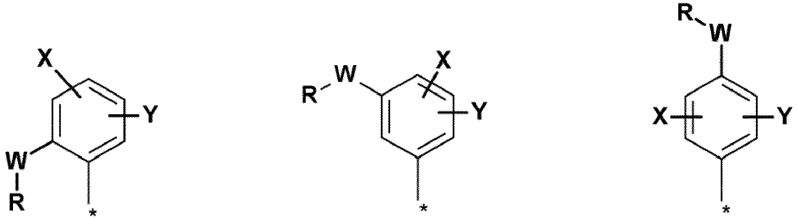
dans lesquels la liaison à l'atome d'azote dans le groupe C(=V)-N-Q dans la formule (I) est marquée avec l'étoile (*),
R représente méthyle, éthyle, propyle, iso-propyle, n-butyle, isobutyle, sec-butyle, tert-butyle, allyle, 2-butényle, propargyle, 2-butynyle, chacun éventuellement substitués une fois, deux fois ou trois fois par fluor ou une fois par cyano ; cycloalkyle en C₃-C₆ éventuellement substitué une fois par fluor, chlore, cyano, méthyle, éthyle, méthoxy, éthoxy ou trifluorométhyle ; cycloalkylméthyle en C₃-C₆ éventuellement substitué une fois par fluor, chlore, cyano, méthyle, éthyle, méthoxy ou trifluorométhyle ; ou phényle, benzyle, pyridyle, pyrimidyle, thiazolyle et pyridinylméthyle, chacun éventuellement substitués une fois ou deux fois par fluor, chlore, brome, cyano, méthyle, éthyle, iso-propyle, tert.-butyle, trifluorométhyle, difluorométhyle, méthoxy, éthoxy, difluorométhoxy ou trifluorométhoxy,
W représente un radical de la série constituée par S, SO et SO₂,
X représente un radical de la série constituée par hydrogène, fluor, chlore, brome, cyano, méthyle, éthyle, trifluorométhyle, méthoxy, éthoxy, difluorométhoxy et trifluorométhoxy,
Y représente un radical de la série constituée par hydrogène, fluor, chlore, brome, cyano, méthyle, éthyle, trifluorométhyle, méthoxy, éthoxy, difluorométhoxy et trifluorométhoxy,
R² représente hydrogène ou méthyle,
Q représente azote ou C-R³,
R³ représente un radical de la série constituée par hydrogène, méthyle, trifluorométhyle et cyclopropyle,
V représente oxygène.

6. Composés de formule (I) selon la revendication 1, dans lesquels
G représente N ou C-A¹,
A¹ représente un radical de la série constituée par hydrogène et fluor,
T représente une paire d'électrons,
R¹ représente le radical de formule 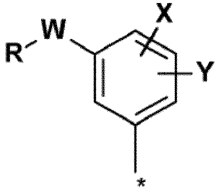
dans laquelle la liaison à l'atome d'azote dans le groupe C(=V)-N-Q dans la formule (I) est marquée avec l'étoile (*),
R représente méthyle, éthyle, propyle, iso-propyle, n-butyle, isobutyle, sec-butyle, tert-butyle, allyle, 2-butényle, propargyle, 2-butynyle, chacun éventuellement substitués une fois, deux fois ou trois fois par fluor ou une fois par cyano ; cycloalkyle en C₃-C₆ éventuellement substitué une fois par fluor, chlore, cyano, méthyle, éthyle, méthoxy, éthoxy ou trifluorométhyle ; cycloalkylméthyle en C₃-C₆ éventuellement substitué une fois par fluor, chlore, cyano, méthyle, éthyle, méthoxy ou trifluorométhyle ; ou phényle, benzyle, pyridyle, pyrimidyle, thiazolyle et pyridinylméthyle, chacun éventuellement substitués une fois ou deux fois par fluor, chlore, brome, cyano, méthyle, éthyle, iso-propyle, tert.-butyle, trifluorométhyle, difluorométhyle, méthoxy, éthoxy, difluorométhoxy ou trifluorométhoxy,
W représente un radical de la série constituée par S, SO et SO₂,
X représente un radical de la série constituée par hydrogène, fluor, chlore, brome, cyano, méthyle, éthyle et trifluorométhyle,
Y représente un radical de la série constituée par hydrogène, fluor, chlore, brome, cyano, méthyle, éthyle et trifluorométhyle,
R² représente hydrogène ou méthyle,
Q représente azote ou C-R³,
R³ représentant un radical de la série constituée par hydrogène et méthyle,
V représente oxygène.

7. Composés de formule (I) selon la revendication 1, dans lesquels
G représente N ou C-A¹,
A¹ représente un radical de la série constituée par hydrogène ou fluor,
T représente une paire d'électrons,
R¹ représente le radical de formule 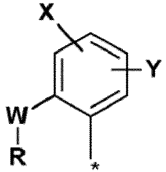
dans laquelle la liaison à l'atome d'azote dans le groupe C(=V)-N-Q dans la formule (I) est marquée avec l'étoile (*),
R représente méthyle, éthyle, propyle, iso-propyle, n-butyle, isobutyle, sec-butyle, tert-butyle, allyle, 2-butényle, propargyle, 2-butynyle, chacun éventuellement substitués une fois, deux fois ou trois fois par fluor ou une fois par cyano ; cycloalkyle en C₃-C₆ éventuellement substitué une fois par fluor, chlore, cyano, méthyle, éthyle, méthoxy, éthoxy ou trifluorométhyle ; cycloalkylméthyle en C₃-C₆ éventuellement substitué une fois par fluor, chlore, cyano, méthyle, éthyle, méthoxy ou trifluorométhyle ; ou phényle, benzyle, pyridyle, pyrimidyle, thiazolyle et pyridinylméthyle, chacun éventuellement substitués une fois ou deux fois par fluor, chlore, brome, cyano, méthyle, éthyle, iso-propyle, tert.-butyle, trifluorométhyle, difluorométhyle, méthoxy, éthoxy, difluorométhoxy ou trifluorométhoxy,
W représente un radical de la série constituée par S, SO et SO₂,
X représente un radical de la série constituée par hydrogène, fluor, chlore, brome, cyano, méthyle, éthyle et trifluorométhyle,
Y représente un radical de la série constituée par hydrogène, fluor, chlore, brome, cyano, méthyle, éthyle et trifluorométhyle,
R² représente hydrogène ou méthyle,
Q représente azote ou C-R³,
R³ représente un radical de la série constituée par hydrogène et méthyle,
V représente oxygène.

8. Composés de formule (I) selon la revendication 1, dans lesquels
G représente N ou C-A¹,
A¹ représente un radical de la série constituée par hydrogène ou fluor,
T représente une paire d'électrons,
R¹ représente le radical de formule 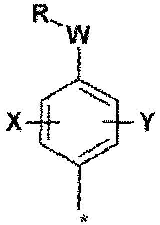
dans laquelle la liaison à l'atome d'azote dans le groupe C(=V)-N-Q dans la formule (I) est marquée avec l'étoile (*),
R représente méthyle, éthyle, propyle, iso-propyle, n-butyle, isobutyle, sec-butyle, tert-butyle, allyle, 2-butényle, propargyle, 2-butynyle, chacun éventuellement substitués une fois, deux fois ou trois fois par fluor ou une fois par cyano ; cycloalkyle en C₃-C₆ éventuellement substitué une fois par fluor, chlore, cyano, méthyle, éthyle, méthoxy, éthoxy ou trifluorométhyle ; cycloalkylméthyle en C₃-C₆ éventuellement substitué une fois par fluor, chlore, cyano, méthyle, éthyle, méthoxy ou trifluorométhyle ; ou phényle, benzyle, pyridyle, pyrimidyle, thiazolyle et pyridinylméthyle, chacun éventuellement substitués une fois ou deux fois par fluor, chlore, brome, cyano, méthyle, éthyle, iso-propyle, tert.-butyle, trifluorométhyle, difluorométhyle, méthoxy, éthoxy, difluorométhoxy ou trifluorométhoxy,
W représente un radical de la série constituée par S, SO et SO₂,
X représente un radical de la série constituée par hydrogène, fluor, chlore, brome, cyano, méthyle, éthyle et trifluorométhyle,
Y représente un radical de la série constituée par hydrogène, fluor, chlore, brome, cyano, méthyle, éthyle et trifluorométhyle,
R² représente hydrogène ou méthyle,
Q représente azote ou C-R³,
R³ représente un radical de la série constituée par hydrogène et méthyle,
V représente oxygène.

9. Composés de formule (I) selon la revendication 1, dans lesquels
G représente C-A¹,
A¹ représente hydrogène,
T représente une paire d'électrons ou l'oxygène,
R¹ représente le radical de formule 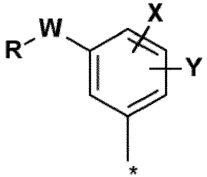
dans laquelle la liaison à l'atome d'azote dans le groupe C(=V)-N-Q dans la formule (I) est marquée avec l'étoile (*),
R représente un radical de la série constituée par méthyle, trifluoroéthyle et cyclopropylméthyle,
W représente un radical de la série constituée par S et SO,
X représente un radical de la série constituée par hydrogène, fluor, chlore et méthyle,
Y représente un radical de la série constituée par hydrogène, chlore et méthyle,
R² représente hydrogène,
Q représente azote ou C-R³,
R³ représente hydrogène ou méthyle, et
V représente oxygène.

10. Agent, **caractérisé par** une teneur en au moins un composé de formule (I) selon les revendications 1 à 9 et des extendeurs usuels, choisis parmi l'eau et les liquides chimiques organiques polaires et apolaires, et/ou des substances tensioactives.

11. Utilisation de composés de formule (I) selon les revendications 1 à 9 ou d'agents selon la revendication 10 pour lutter contre des nuisibles.

12. Composés de formule (II) 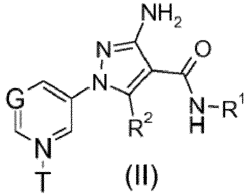 dans laquelle G, T, R¹ et R² ont les significations indiquées dans la revendication 1.

13. Composé de formule (VIII-1)
